# EUROPEAN PATENT APPLICATION

(11) **EP 4 651 147 A2**
(43) Date of publication of application: **19.11.2025**
(21) Application number: 25206673.3
(22) Date of filing: 27.08.2024
(51) Int. Cl.: G16H 20/70

(54) **DELIVERY OF MULTI-LAYER DIGITAL THERAPY TO USERS FOR ADDRESSING FUNCTIONAL IMPAIRMENT**

(30) Priority: 30.08.2023 US 202363535557 P; 12.12.2023 US 202363609268 P; 07.03.2024 US 202463562589 P; 10.04.2024 US 202463632292 P; 09.08.2024 US 202418799387
(62) Divisional of application: 24196784.3
(71) Applicant: Click Therapeutics, Inc., New York, NY 10013 (US)
(72) Inventor: SNIPES, Cassandra, New York, 10013 (US); UNG, Eehwa, New York, 10013 (US); LONGENECKER, Julia, New York, 10013 (US); SODHI, Puneet, New York, 10013 (US); CHEHAYEB, Ross, New York, 10013 (US)
(74) Representative: Marks & Clerk LLP

(57) **Abstract**

Provided herein are systems and methods for presenting interactive sessions to address functional impairment in users related to a disease or disorder. A computing device may provide a first session for a cognitive training layer associated with a skill. The computing system may provide a second session for a virtual functional training layer for the user to apply the skill in virtual settings. The computing system may provide a third session for a functional training layer for the user to perform an activity to apply the skill. The efficacy of the medication that the user is taking to address the disease or disorder may be increased.

## Description

### CROSS REFERENCE TO RELATED APPLICATIONS

The present application claims priority to U.S. Non-Provisional Application No. 18/799,387, titled "Delivery of Multi-Layer Digital Therapy to Users for Addressing Functional Impairment," filed August 9, 2024, and U.S. Provisional Application No. 63/632,292, titled "Delivery of Multi-Layer Digital Therapy to Users for Addressing Functional Impairment," filed April 10, 2024, and U.S. Provisional Application No. 63/562,589, titled "Delivery of Multi-Layer Digital Therapy to Users for Addressing Functional Impairment," filed March 7, 2024, and U.S. Provisional Application No. 63/609,268, titled "Delivery of Multi-Layer Digital Therapy to Users for Addressing Functional Impairment," filed on December 12, 2023, and U.S. Provisional Application No. 63/535,557, titled "Delivery of Multi-Layer Digital Therapy to Users for Addressing Functional Impairment," filed August 30, 2023, each of which are incorporated herein by reference in their entireties.

### BACKGROUND

Certain conditions may cause a patient to lack or suffer from functional impairments, such as social processing impairment, non-social processing impairment, or both. Patients with conditions which affect their ability to understand or navigate social circumstances may encounter difficulty functioning in environments such as at work, home, business establishments, or other areas where patients interact with other persons. The social processing impairment experienced by these patients can cause apprehension surrounding social settings, thereby further isolating the patients from engaging in social circumstances. The conditions can cause a patient to have difficulty discerning social cues, such as those shown through body language or tone of voice. This lack of social skills can cause a patient to have extreme difficulty navigating social settings due to not understanding or comprehending the social cues exhibited by those around the patient.

In addition, patients may suffer from non-social processing impairments such as memory (e.g., verbal memory to store and recall verbally conveyed information), executive functions (e.g., as problem-solving and planning), and other cognitive functions. These cognitive impairments may lead to a reduction in a capability to perceive, understand, or respond to the patient's surroundings and the people around the patient. In the case of verbal memory, deficiencies in this cognitive function may pose significant challenges for individuals in various aspects of their daily lives, such as difficulty in remembering conversations with others, impairment in expressiveness, and memory recall issues with names, dates, and other information. Impairments to verbal memory can lead to frustration and a sense of helplessness. This can also affect personal relationships with family and friends, such as forgetting important events, conversations, or details that lead to misunderstanding.

Treating functional impairments in patients with conditions that affect their ability to navigate social circumstances or properly respond to their surroundings can prove difficult due to the inherent nature of the condition. For instance, while a patient can learn about skills in the environment of a clinician setting who the patient may know well, the patient may be fearful of or unable to apply those skills in a real-life environment. The leap between a clinical setting and a real-life environment (e.g., a bustling coffee shop or a time-sensitive social interaction like boarding the correct city bus) may be too great for many patients with a functional impairment. As another example, patients with difficulties processing information may be unable to follow instructions or guidance from the clinician, especially after the therapy session.

Remediation through traditional in-person clinical measures with a clinician to provide certain psychosocial therapies and other intervention techniques may not provide an adequate method to improve the functional impairment. Consistent adherence to the therapies may be difficult to obtain for the patients due to difficulties with social interactions and other inabilities. Furthermore, such in-person clinical measures can be restricted by scheduling and physical constraints. For example, there may be no specialized or trained clinicians near a patient (e.g., particularly patients located in rural or non-metro areas). In addition, even for specialized or trained clinicians, such clinicians may not be able to meet with patients frequently or immediately within a short amount of time. This problem may be exacerbated by the fact that the patients may have a time-sensitive condition to be treated or addressed.

### SUMMARY

To resolve these and other technical challenges, a digital therapy in the form of a three-layer interventional system (also referred herein as enhanced life skills by cognitive intervention (ELSCI)) can fill in the gaps and limitations of the current standard-of-care treatment, by moving users from a low-stimulus psychoeducation layer to an intermediate practice and response layer to a high-stimulus real-world application layer. By using this multimodal multi-layer system, users may be trained to familiarize themselves to apply skills related to functional impairment in an intermediate virtualized environment before applying the skills and knowledge in a real-world environment. By creating a bridge between training and real-world application, users can successfully transfer knowledge into learned and applied skills in real-world functioning. The transfer of knowledge and application of skills can boost selfconfidence, help users in their daily lives at school or at work, and strengthen users' relationships with friends and family. In addition, the digital delivery allows patients to experience an increased frequency of interactions compared to traditional therapy limited to infrequent in-person clinical settings, specifically in the form of frequent (e.g., daily multiple times per day) real-time feedback and proactive reminders to help the user execute on the training tasks.

Each layer may be aimed at enhancing abilities in users with functional impairments through a series of exercises and can build on the prior session, with increasingly complex and life-like virtual scenarios to simulate and contextualize real-life situations. The sessions can progress through digital cognitive remediation tasks to train the user to recognize cues (or other markers) from images displayed through the device to an ecological generalization of cognitive remediation in which the user can interact in real-world settings. The ecological generalization of cognitive remediation can correspond to applying the therapies of other sessions into a virtual or real-life environment. For example, to address social processing impairment that leads to functional impairments, the sessions may present the user with images and prompts via the end user device to train the user to recognize certain social cues and to respond with certain interactions in social settings. To address non-social processing impairment that leads to functional impairments, the session may present images and prompts via the end user device to help the user build up cognitive skills, such as memory, communication, attention, and problem-solving, among others. Through a combination of cognitive remediation and ecological generalization of such remediation through sessions associated with different layers, the user may be able to improve functional impairment.

The training can include three layers, moving from low stimulus and difficulty in practice settings to high stimulus and difficulty and real-world functioning. For addressing social processing impairment, the first layer can include a set of cognitive exercises aimed at increasing a subject's cognitive abilities via repetition. For example, an application on the user's device can present a set of images, videos, or audio recordings of social cues (e.g., in the form of body language) with a prompt inquiring the user to select the correct social cue depicted in the images. To address non-social processing impairment (e.g., verbal memory), in the first layer, the application can present a set of images, videos or audio recordings followed by a prompt inquiring the user to select the correct object or character depicted in the images.

The second layer can include a set of virtual scenarios with the objective of improving the subject's ability to apply skills in virtual social settings. In the second layer for addressing social processing impairment that leads to functional impairments, the application can present another set of images of social settings, with a prompt directing the user to select responses (e.g., dialogue or action) to characters depicted in the setting to further cement social skills. To address non-social processing impairment that leads to functional impairments, in the second layer, the application can present a set of images, videos, or audio of virtual settings with characters or objects, followed a prompt directing the user to select responses (e.g., actions) to help the user build up cognitive skills, such as verbal memory or problem solving. In both cases, the user can be provided feedback in response to user selections.

Continuing on, the third layer can include a set of prompts with the goal of having the user carry out skills (e.g., social and non-social) in real world settings. For example, the application can prompt the user to perform an activity in an environment of the user, such as interacting with others to improve social processing. Through this multilayer approach, the user can be trained to develop social skills and to apply the social skills towards real-world functioning. As a result, the user can be conditioned to develop social skills to overcome the functional impairment associated with the user's condition (e.g., schizophrenia or affective disorders, such as depression and bi-polar disorder).

The treatment component may integrate cognitive remediation with additional layers in the functional remediation model that help patients learn, practice, and apply skills in more complex applied settings to transfer cognitive improvements to functional demands. Content may be contextualized and individualized, with functional areas for remediation to drive engagement. The user may be presented with rationale that explicitly links cognitive remediation exercises to the two layers of ecological generalization. For example, when completing exercises in the first layer, the exercise may allow the user to practice cognitive exercises associated with social or non-social skills. In the second layer, the intervention may incorporate relatable, realistic aspects of daily living into in-app exercises. In the third layer, users may be asked to practice activities in the real world, such as observation of social cues during an interaction with a trusted person. Activity progress may be reviewed after each practice session, with notifications to celebrate successes and improvements to the implementation of the skills in the user's daily life. Across these components, learning theory approaches may be used to increase self-efficacy and engagement. The additional ecological generalization component may optimize the likelihood that the user transfers cognitive remediation, resulting in meaningful impact on the user's ability to function in the real world.

To provide these digital therapy sessions, the service can select and provide stimuli and associated prompts for the user for presentation via the end user device. For the first layer, the service can select a set of media including images, video, audio, or a combination thereof with a character of a certain social cue, along with a prompt providing a set of candidate choices of social cues. Upon a successful rate of response for each presented media of the first session, the service can provide a second session. For the second layer, the service can identify a set of media of virtual social settings with one or more characters, with a prompt defining a set of candidate responses (e.g., dialogue or actions) for the user to select in interacting with the characters. The service can provide feedback based on the user selection. When the rate of correct responses satisfies a threshold, the service can determine to provide sessions of the third layer to the user. Through this multi-session, multi-layer approach to remediation therapy for social processing impairments that leads to functional impairments, the subject may improve the ability to identify social cues and perform social interactions in a real-world environment.

In addition, the service may also time the delivery or presentation of the layer to the user to avoid the user from having to actively access the digital therapeutics application on their device while experiencing their condition. The service may time the delivery or presentation of the layer to the user subsequent to the completion of a prior action related to the session. Furthermore, as the service acquires additional data about the user, the service may be able to select images, prompts, or text more targeted toward the specific user and their condition and may store this data in a profile of the user. The service may calculate a performance metric for the user in the session. The service may select a subsequent session based on at least the prior selections or responses, the performance metric, the completion of a prior action, or the profile of the user, among others.

By using a multi-layer approach to improving functional impairments, the user of the digital therapeutic application can learn, practice, and make ecologically valid, real-world attempts at practicing social and non-social skills. The approach can include moving from low difficulty sessions to high difficulty or real-world functioning sessions. The synergistic incorporation of multiple psychosocial therapies can provide several benefits, notably (i) extending the benefits of cognitive remediation to functional improvements; (ii) providing more ecological validity and real-time interactions than an in-person clinical setting; and (iii) developing an accessible digital treatment.

In this manner, the user can receive sessions relating to the condition with ease to help alleviate functional impairment relating to the condition as documented herein. This may reduce or eliminate barriers to the user from physically accessing their device while combating the condition. By selecting the sessions sent to the user to address the subject's difficulty with functioning, the quality of a human computer interactions (HCI) between the user and the device may be improved. In addition, since the sessions are more related to the user's condition, unnecessary consumption of computational resources (e.g., processing and memory) of the service and the user device and the network bandwidth may be reduced, relative to sending ineffective messages.

In addition, in the context of a digital therapeutics application, the selection of such sessions may result in the delivery of user-specific interventions to improve subject's adherence to the treatment. Furthermore, the multi-layer approach provided through the digital therapeutics application can provide potential improvements to the subject's functional impairment due to a condition (e.g., schizophrenia or an affective disorder) that the user has, as will be documented herein.

Aspects of the present disclosure relate to systems and methods for presenting interactive sessions to address functional impairment in users. The system may include a computing system having one or more processors coupled with memory. The computing system may identify a set of sessions to address an impairment associated with a condition of a user. Each session of the set of sessions can include a corresponding layer of a set of layers for the user. The computing system may provide a first session for a cognitive training layer by displaying one or more first images with which the user is to recognize one or more of a set of social cues associated with a social skill. The computer system may provide a second session for a virtual functional training layer for the user to apply the social skill in virtual social settings. The second session can include the computer system displaying a second image of a social setting with (a) a first prompt identifying a query associated with a character displaying one of the sets of social cues and (b) a set of interactive elements identifying a corresponding set of responses to the character. The second session can include the computer system receiving a first response for the set of responses selected by the user via at least one of the sets of interactive elements. The second session can include the computer system providing feedback to the user based on the query for the social setting and the response. The computer system can provide a third session for a functional training layer for the user to apply the social skills. The third session can include the computer system displaying a second prompt to direct the user to perform and activity. The third session can include the computer system receiving a second response associated with performance of the activity. The above can apply equally to non-social skills.

In some embodiments, the computing system may generate a performance metric for the user based on the first response received from the user during the second session at a first-time instance. The computing system may modify, based on the performance metric, at least one of a set of parameters defining presentation of at least one of the images, prompts, and interactive elements for the virtual functional training layer. The computing system may provide the second session for the virtual functional training layer at a second time instance.

In some embodiments, the computing system may provide the second session by displaying a third image of a second social setting with (i) a third prompt identifying a second query of a second character in the second social setting and (ii) a second set of interactive elements identifying a corresponding set of responses to the second character in accordance with the set of parameters. In some embodiments, the set of parameters can include at least one of (i) a type of modality for content, (ii) a context for social settings in images, (iii) a number of characters in social settings, (iv) a type of prompt, (v) a difficulty level for response, (vi) a type of response, or (vii) a number of responses. The above can apply equally to non-social skills.

In some embodiments, the computing system may generate a performance metric for the user based on a rate of correct selections in one or more sessions for the cognitive training layer. The computing system may determine, responsive to the performance metric satisfying a threshold, to transition the user from the cognitive training layer to the virtual functional training layer. The computing system may determine, responsive to the performance metric satisfying a threshold, to adjust the difficulty of the exercises within the cognitive training layer. In some embodiments, the computing system may generate a performance metric for the user based on a rate of correct responses in one or more sessions for the virtual functional training layer. The computing system may determine, responsive to the performance metric satisfying a threshold, to adjust the difficulty of the exercises within the virtual functional training layer. The computing system may determine, responsive to the performance metric satisfying a threshold, to transition the user from the virtual functional training layer to the functional training layer.

In some embodiments, the computing system may provide the first session by (i) displaying a first image of a social cue with a set of interactive elements identifying the corresponding set of types of social cues associated with the first image and (ii) receiving a selection of one of the set of social cues by the user via at least one of the set of interactive elements. The set of social cues for the first image in the first session for the cognitive training layer can include at least one of (a) a head movement, (b) a body language, (c) a gesticulation, or (d) an eye contact. The above can apply equally to non-social skills.

In some embodiments, the second session for the virtual functional training layer may include displaying a set of images of the social setting with the characters in accordance with a defined sequence. In some embodiments, the third session for the functional training layer can include displaying a third prompt for the user to select a time at which to provide a message prompting the user to perform the activity. In some embodiments, the computing system may identify a time at which to provide one of the set of sessions to the users in accordance with a session schedule. In some embodiments, the condition of the user can include schizophrenia, wherein the user is receiving a treatment, at least in partial concurrence with at least one of the first session, the second session, or the third session. The treatment can include at least one of a psychosocial intervention or a medication to address the schizophrenia. The sessions may increase the efficacy of the medication that the user is taking to address the condition. The above can apply equally to non-social skills.

Other aspects of the present disclosure are directed to methods of ameliorating a functional impairment in a user with schizophrenia in need thereof. A computing system may obtain a first metric associated with the user prior to a plurality of sessions. The computing system may repeat provision of one or more of the plurality of sessions to the user. The plurality of sessions may include a first session for a cognitive training layer by displaying one or more first images with which the user is to recognize one or more of a plurality of social cues associated with a social skill. The plurality of sessions may include a second session for a virtual functional training layer for the user to apply the social skill in virtual social settings, comprising (i) displaying a second image of a social setting with (a) a first prompt identifying a query associated with a character displaying one of the plurality of social cues and (b) a second set of interactive elements identifying a corresponding plurality of responses to the character, (ii) receiving a response from the plurality of responses selected by the user via at least one of the second set of interactive elements, and (iii) providing a feedback to the user based on the query for the setting and the response. The plurality of sessions may include a third session for a functional training layer, comprising (i) displaying a second prompt to direct the user to perform an activity and (ii) receiving a response associated with performance of the activity. The computing system may obtain a second metric associated with the user subsequent to at least one of the plurality of sessions. An amelioration in the functional impairment associated with schizophrenia may occur in the user, when the second metric is (i) decreased from the first metric by a first predetermined margin or (ii) increased from the first metric by a second predetermined margin. The above can apply equally to non-social skills.

In some embodiments, schizophrenia in the user may include at least one of (i) schizophrenia with positive symptoms including hallucinations or delusions; or (ii) schizophrenia with negative symptoms including a decrease in motivation or emotional expressions. In some embodiments, the functional impairment associated with the user may include at least one of (i) reduced attainment in education, (ii) reduced quality of life, (iii) difficulty in living independently, (iv) reduced social functioning, or (v) occupational dysfunction. In some embodiments, the user may be an adult aged at least 18 years or older, diagnosed with schizophrenia and experiencing the functional impairment.

In some embodiments, the amelioration in the functional impairment associated with schizophrenia may occur when the second metric is decreased from the first metric by the first predetermined margin. The first metric and the second metric may be Multnomah Community Ability Scale (MCAS) values. In some embodiments, the amelioration in the functional impairment associated with schizophrenia occurs when the second metric is increased from the first metric by the second predetermined margin. The first metric and the second metric may be Lawton Instrumental Activities of Daily Living (Lawton IADL) scale values.

In some embodiments, the amelioration in the functional impairment associated with schizophrenia occurs when the second metric is increased from the first metric by the second predetermined margin. In some embodiments, the first metric and the second metric may be Personal and Social Performance (PSP) scale values. In some embodiments, the amelioration in the functional impairment associated with schizophrenia may occur when the second metric is decreased from the first metric by the first predetermined margin. The first metric and the second metric may be World Health Organization Disability Assessment Schedule 2.0 (WHO-DAS 2.0) scale values. In some embodiments, the amelioration in the functional impairment associated with schizophrenia occurs when the second metric is decreased from the first metric by the first predetermined margin, and wherein the first metric and the second metric are Columbia-Suicide Severity Rating Scale (C-SSRS) values.

In some embodiments, the plurality of sessions further may include the second session for the virtual functional training at a first time instance, comprising displaying a third image of a second setting with (i) a third prompt identifying a second query of a second character in the second setting and (ii) a third set of interactive elements identifying a corresponding plurality of responses to the second character in accordance with a plurality of parameters. In some embodiments, at least one of the plurality of parameters may be modified based on a performance metric for the user using the response received from the user during the second session at a second time instance prior to the first-time instance. The above can apply equally to non-social skills.

In some embodiments, the plurality of parameters comprises at least one of (i) a type of modality for content, (ii) a context for settings in images, (iii) a number of characters in settings, (iv) a type of prompt, (v) a difficulty level for responses, (vi) a type of response, or (vii) a number of responses. In some embodiments, the computing system may determine, for at least one of the plurality of sessions, to transition from one layer to another layer based on a performance metric for the user across one or more of the plurality of sessions.

In some embodiments, the computing system may provide the first session to include (i) displaying a first image of a social cue with a set of interactive elements identifying the corresponding plurality of types of cues associated with the first image and (ii) receiving a selection of one of the plurality of types of social cues by the user via at least one of the second set of interactive elements. In some embodiments, the plurality of social cues for the first image in the first session for the cognitive training layer further comprises at least one of (a) a head movement, (b) a body language, (c) a gesticulation, or (d) an eye contact. The above can apply equally to non-social skills.

In some embodiments, the second session for the virtual functional training layer may include displaying a plurality of images of the setting with the characters in accordance with a defined sequence. In some embodiments, the third session for the functional training layer may include displaying a third prompt for the user to select a time at which to provide a message prompting the user to perform the activity. In some embodiments, the plurality of sessions may be provided over a period of time ranging between 2 weeks to 30 weeks. In some embodiments, the user may be receiving a treatment, at least in partial concurrence with at least one of the plurality of sessions. In some embodiments, the treatment may include at least one of a psychosocial intervention or a medication to address schizophrenia. The sessions may increase the efficacy of the medication that the user is taking to address the condition. The above can apply equally to non-social skills.

Other aspects of the present disclosure are directed to systems and methods of presenting interactive sessions to address impairment in verbal learning and memory as well as cognitive association in users. A computing system may identify a plurality of sessions to address an impairment associated with a condition of a user, each session of the plurality of sessions comprising a corresponding layer of a plurality of layers for the user. The computing system may provide a first session for a cognitive training layer associated with a verbal memory skill by presenting one or more first audio recordings with which the user is to recall one or more of a plurality of words. The computing system may provide a second session for a virtual functional training layer for the user to apply the verbal memory skill in virtual settings. The second session may include (i) presenting a second audio recording of a speech sample with (a) a first prompt identifying a query associated with the speech sample and (b) a set of interactive elements identifying a corresponding plurality of responses; (ii) receiving a first response from the plurality of responses selected by the user via at least one of the set of interactive elements; and (iii) providing a feedback to the user based on the query for the speech sample and the response. The computing system may provide a third session for a functional training layer for the user to apply the verbal memory skill, comprising (i) displaying a second prompt to direct the user to perform an activity and (ii) receiving a second response associated with performance of the activity. Instead of an audio recording, the foregoing could use a prompt, video, or image.

In some embodiments, the computing system may generate a performance metric for the user based on the first response received from the user during the second session at a first-time instance. The computing system may modify, based on the performance metric, at least one of a plurality of parameters defining presentation of at least one of audio recordings, prompts, and interactive elements for the virtual functional training layer. The computing system may provide the second session for the virtual functional training layer at a second time instance, comprising presenting a third audio recording of a speech sample between characters in a social setting with (i) a third prompt identifying a second query associated with the speech sample of the characters and the social setting and (ii) a second set of interactive elements identifying a corresponding plurality of responses in accordance with the plurality of parameters.

In some embodiments, the plurality of parameters may include at least one of (i) a type of modality for content, (ii) a context for social settings in audio recordings, (iii) a number of characters in social settings, (iv) a type of prompt, (v) a difficulty level for responses, (vi) a type of response, or (vii) a number of responses. In some embodiments, the computing system may present the second audio recording in accordance with a plurality of parameters. The plurality of parameters may include at least one of (a) an inclusion of a distraction, (b) an ability to repeat the second audio recording, (c) a modification in speed, (d) a time between each word, (e) a number of words in each sentence, (f) a length of the audio recording, or (g) an ability to control the distraction.

In some embodiments, the computing system may generate a performance metric for the user based on a rate of correct selections in one or more sessions for the cognitive training layer. The computing system may determine, responsive to the performance metric satisfying a threshold, to transition the user from the cognitive training layer to the virtual functional training layer. In some embodiments, the computing system may generate a performance metric for the user based on a rate of correct responses in one or more sessions for the virtual functional training layer. The computing system may determine, responsive to the performance metric satisfying a threshold, to transition the user from the virtual functional training layer to the functional training layer.

In some embodiments, the computing system may provide the first session by identifying, from a plurality of recordings, a set of recordings to present to the user, each of the plurality of recordings corresponding to one or more words; presenting, to the user, the set of recordings in accordance with a format to define a context in which the one or more words of each of the set of recordings are presented; displaying an interface to prompt the user to select at least one of a plurality of words as presented in the set of recordings; and receiving, via the interface, a selection of at least one word or image by the user.

In some embodiments, the third session for the functional training layer may include displaying a third prompt for the user to select a time at which to provide a message prompting the user to perform the activity. In some embodiments, the computing system may identify a time at which to provide one of the plurality of sessions to the users in accordance with a session schedule. In some embodiments, the condition of the user may include schizophrenia. The user may be receiving a treatment, at least in partial concurrence with at least one of the first session, the second session, or the third session. The treatment may include at least one of a psychosocial intervention or a medication to address schizophrenia.

Other aspects of the present disclosure are directed to a method of ameliorating a functional impairment associated with verbal memory in a user with schizophrenia in need thereof. A computing system may obtain a first metric associated with the user prior to a plurality of sessions. The computing system may repeat provision of one or more of the plurality of sessions to the user. The plurality of sessions may include a first session for a cognitive training layer associated with a verbal memory skill by presenting one or more first audio recordings with which the user is to recall one or more of a plurality of words. The plurality of sessions may include a second session for a virtual functional training layer for the user to apply the verbal memory skill in virtual settings, comprising (i) presenting a second audio recording with (a) a first prompt identifying a query associated with a speech sample and (b) a set of interactive elements identifying a corresponding plurality of responses, (ii) receiving a first response from the plurality of responses selected by the user via at least one of the set of interactive elements, and (iii) providing a feedback to the user based on the query for the speech sample and the response. The plurality of sessions may include a third session for a functional training layer for the user to apply the verbal memory skill, comprising (i) displaying a second prompt to direct the user to perform an activity and (ii) receiving a second response associated with performance of the activity. The computing system may obtain a second metric associated with the user subsequent to at least one of the plurality of sessions. Amelioration in the functional impairment associated with schizophrenia may occur in the user, when the second metric is (i) decreased from the first metric by a first predetermined margin or (ii) increased from the first metric by a second predetermined margin.

In some embodiments, the schizophrenia further comprises schizophrenia with negative symptoms including a decrease in motivation or emotional expressions. In some embodiments, the functional impairment associated with the user further may include at least one of (i) reduced attainment in education, (ii) reduced quality of life, (iii) difficulty in living independently, (iv) reduced social functioning, or (v) occupational dysfunction. In some embodiments, the user may be an adult aged at least 18 years or older and diagnosed with the schizophrenia experiencing the functional impairment. In some embodiments, the plurality of sessions may be provided over a period of time ranging between 2 weeks to 30 weeks.

In some embodiments, the amelioration in the functional impairment associated with schizophrenia may occur when the second metric is decreased from the first metric by the first predetermined margin, and wherein the first metric and the second metric are Multnomah Community Ability Scale (MCAS) values. In some embodiments, the amelioration in the functional impairment associated with schizophrenia may occur when the second metric is increased from the first metric by the second predetermined margin, and wherein the first metric and the second metric are clinical rating scale (CRS) values.

In some embodiments, the amelioration in the functional impairment associated with schizophrenia may occur when the second metric is decreased from the first metric by the first predetermined margin, and wherein the first metric and the second metric are Patient Global Impression of Improvement (PGI-I) scale values. In some embodiments, the amelioration in the functional impairment associated with schizophrenia may occur when the second metric is decreased from the first metric by the first predetermined margin, and wherein the first metric and the second metric are Clinical Global Impression of Improvement (CGI-I) scale values. In some embodiments the amelioration in the functional impairment associated with schizophrenia may occur when the second metric is decreased from the first metric by the first predetermined margin, wherein the difference between the first metric and second metric are PGI-I or CGI-I scale values. In some embodiments, the amelioration in the functional impairment associated with schizophrenia may occur when the second metric is increased from the first metric by the second predetermined margin, and wherein the first metric and the second metric are medication adherence rating scale (MARS-a) values. In some embodiments, the amelioration in the functional impairment associated with schizophrenia may occur when the second metric is decreased from the first metric by the first predetermined margin, and wherein the first metric and the second metric are Columbia-Suicide Severity Rating Scale (C-SSRS) values.

In some embodiments, the computing system may determine, for at least one of the plurality of sessions, to transition from one layer to another layer based on a performance metric for the user across one or more of the plurality of sessions. In some embodiments, the second session may include presenting the second audio recording in accordance with a plurality of parameters. The plurality of parameters may include at least one of (a) an inclusion of a distraction, (b) an ability to repeat the second audio recording, (c) a modification in speed, (d) a time between each word, (e) a number of words in each sentence, (f) a length of the audio recording, or (g) an ability to control the distraction.

In some embodiments, the first session may include identifying, from a plurality of recordings, a set of recordings to present to the user, each of the plurality of recordings corresponding to one or more words; presenting, to the user, the set of recordings in accordance with a format to define a context in which the one or more words of each of the set of recordings are presented; displaying an interface to prompt the user to select at least one of a plurality of words as presented in the set of recordings; and receiving, via the interface, a selection of at least one word or image by the user. In some embodiments, the user may be receiving a treatment, at least in partial concurrence with at least one of the plurality of sessions. The treatment may include at least one of a psychosocial intervention or a medication to address schizophrenia.

### BRIEF DESCRIPTION OF THE DRAWINGS

The foregoing and other objects, aspects, features, and advantages of the disclosure will become more apparent and better understood by referring to the following description taken in conjunction with the accompanying drawings, in which:
FIG. 1 depicts a block diagram of a system for presenting interactive sessions to address functional impairment in users in accordance with an illustrative embodiment;
FIG. 2 depicts a block diagram for a process to identify a set of sessions to address functional impairment in users and provide a first session for a cognitive training layer of the set of sessions in accordance with an illustrative embodiment;
FIGS. 3A-3H depict screenshots of sets of example user interfaces for providing a first session in accordance with an illustrative embodiment;
FIG. 4 depicts a block diagram for a process to provide a second session for a virtual functional training layer for the user to apply the social skill in virtual social settings in accordance with an illustrative embodiment;
FIGS. 5A-5G depicts screenshots of sets of example user interfaces for providing a second session in accordance with an illustrative embodiment;
FIG. 6 depicts a block diagram for a process to provide a third session for a functional training layer for the user to apply the social skill in accordance with an illustrative embodiment;
FIGS. 7A-7E depict screenshots of set of example user interfaces for providing a third session in accordance with an illustrative embodiment;
FIGS. 8A-8C depict a flow diagram of a method for presenting interactive sessions to address functional impairment in users in accordance with an illustrative embodiment;
FIG. 9 depicts a flow diagram of a method of ameliorating a functional impairment in a user with schizophrenia, in accordance with an illustrative embodiment; and
FIG. 10 illustrates the decreases in WHO-DAS 2.0 disability assessments following 4-week interaction with the CT-156 mobile app. WHO-DAS 2.0 assessed disability based on 36 separate items with scores of 1 (none) to 5 (extreme). The change in WHO-DAS screening from the beginning to the end of the study demonstrated significant reductions in disability assessments, indicating that participants saw improvements in their abilities across multiple functional domains.
FIG. 11 is a block diagram of a server system and a client computer system in accordance with an illustrative embodiment.

### DETAILED DESCRIPTION

For purposes of reading the description of the various embodiments below, the following enumeration of the sections of the specification and their respective contents may be helpful:
Section A describes systems and methods for presenting interactive sessions to address functional impairment in users;
Section B describes methods of ameliorating a functional impairment in a user with schizophrenia; and
Section C describes a network and computing environment which may be useful for practicing embodiments described herein.

### A. Systems and Methods for Presenting Interactive Sessions to Address Functional Impairment in Users

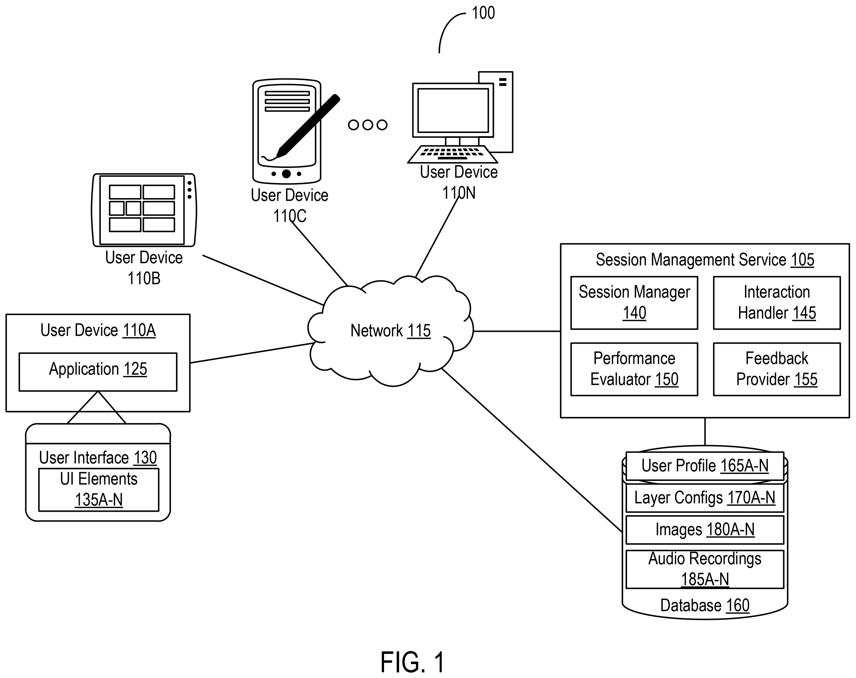

Referring now to FIG. 1, depicted is a block diagram of a system 100 for presenting interactive sessions to address functional impairment (e.g., social processing or non-social processing) in users. In an overview, the system 100 may include at least one session management service 105 and a set of user devices 110A-N (hereinafter generally referred to as user devices 110), communicatively coupled with one another via at least one network 115. At least one user device 110 (e.g., the first user device 110A as depicted) may include at least one application 125. The application 125 may include or provide at least one user interface 130 with one or more user interface (UI) elements 135A-N (hereinafter generally referred to as UI elements 135). The session management service 105 may include at least one session manager 140, one interaction handler 145, one performance evaluator 150, or at least one feedback provider 155, among others. The session management service 105 may include or have access to at least one database 160. The database 160 may store, maintain, or otherwise include one or more user profiles 165A-N (hereinafter generally referred to as user profiles 165), one or more layer configurations 170A-N (hereinafter generally referred to as layer configurations 170 or layers 170), one or more images 180A-N (hereinafter generally referred to as images 180), or one or more audio records 185A-N (hereinafter generally referred to as audio recordings 185), among others. The functionality of the application 125 may be performed in part on the session management service 105. Conversely, the functionality of the application 125 may also incorporate operations performed on the session management service 105. Collectively, the user device 110 and the session management service 105 may be part of a computing system to provide the application 125.

In further detail, the session management service 105 may (sometimes herein generally referred to as a service) be any computing device comprising one or more processors coupled with memory and software and capable of performing the various processes and tasks described herein. The session management service 105 may be in communication with the one or more user devices 110 and the database 160 via the network 115. The session management service 105 may be situated, located, or otherwise associated with at least one server group. The server group may correspond to a data center, a branch office, or a site at which one or more servers corresponding to the session management service 105 is situated. The session management service 105 may be situated, located, or otherwise associated with one or more of the client devices 110. Some components of the session management service 105 may be located within the server group, and some may be located within the client device. For example, the session manager 140 may operate or be situated on the user device 110, and the interaction handler 145 may operate or be situated on the server group.

Within the session management service 105, the session manager 140 may identify a set of sessions from the layer configurations 170 to present to a user by the application 125 on respective user devices 110. The session manager 140 may administer a set of sessions to address functional impairment in a user and may present one or more of stimuli (e.g., the images 180) in accordance with a session of the set of sessions. The interaction handler 145 may monitor for responses by the user on the user interface 130 in sessions provided via the user device 110. The performance evaluator 150 may determine a performance metric for the user in each session. The feedback provider 155 may provide feedback based on the performance to present to the user via the application 125 operating on the user device 110.

The user device 110 (sometimes herein referred to as an end user computing device or client device) may be any computing device comprising one or more processors coupled with memory and software and capable of performing the various processes and tasks described herein. The user device 110 may be in communication with the session management service 105 and the database 160 via the network 115. The user device 110 may be a smartphone, other mobile phone, tablet computer, wearable computing device (e.g., smart watch, eyeglasses), or laptop computer. The user device 110 may be used to access the application 125. In some embodiments, the application 125 may be downloaded and installed on the user device 110 (e.g., via a digital distribution platform). In some embodiments, the application 125 may be a web application with resources accessible via the network 115.

The application 125 executing on the user device 110 may be a digital therapeutics application and may provide the sessions (sometimes herein referred to as a therapy session) to address functional impairment associated with conditions. The user of the application 125 may have, be diagnosed with, or at risk of a condition. The condition may include any number of disorders that cause a functional impairment in the user. The functional impairment may include social processing or non-social processing impairment, across any number of domains. A social processing impairment can correspond to limitations or restrictions of the user in performing daily activities, such as social interactions with others. For instance, the functional impairment may include a reduced attainment in education, reduced quality of life, difficulty in living independently, reduced social functioning, or occupational dysfunction, among others.

In addition, a non-social processing impairment can correspond to difficulties or limitations in cognitive processes, such as mental operations related to attention, memory, language processing, executive functions, or sensory processing, among others. The condition may include, for example, a neurological disorder (e.g., schizophrenia with positive or negative symptoms or multiple sclerosis) or an affective disorder (e.g., major depressive disorder, anxiety disorder, bipolar disorder, or a post-traumatic stress disorder (PTSD)), among others. The condition may impede or hinder social skills, such as behaving in a socially appropriate manner or expressing emotions or needs. In some embodiments, the processing impairment may include deficiency in verbal memory on the part of the user. The verbal memory may correspond to an ability by the user to encode, store, and retrieve information related to language and verbal communication. The verbal memory may include memory recall and cognitive association, among others. The memory recall may refer to the user's ability to retrieve information after being provided (e.g., within 5-10 minutes). The cognitive association may refer to the user's ability to connect or link between information received verbally and another piece of information (e.g., in the form of a cue).

The application 125 may be used to present sessions prompting the user to perform actions to reduce functional impairments associated with the condition of the user. The actions may be presented to the user as a result of sending a request to begin a session, detected measurements of the user received from the client device, or a scheduled time or period, among others. Behaving in a socially appropriate manner can include performing ordinary, daily interactions without undue stress or awkwardness. In some cases, social interactions which may be understood by others without the condition can be very difficult, stressful, or confusing to perform by those with conditions such as schizophrenia. For example, purchasing a coffee in a socially appropriate manner may cause stress or discomfort for a subject experiencing schizophrenia. Initially, the user may not know an appropriate tone of voice, pattern of words, or bodily physicality associated with the action of interacting with a barista in order to buy a coffee. This can lead to difficulty for the subject with functioning in public or social settings and can hinder the lifestyle of the subject. Other behaviors may cause or be related to a condition of the user. By providing the user the digital therapeutic sessions through the application 125, the limitation of social and non-social skills due to the functional impairment arising from the condition can be addressed.

The user may be at least partially concurrently receiving a treatment to address the condition, at least partially concurrently with the sessions through the application 125. The user may be receiving a treatment at least partially concurrently with a first session, a second session, a third session, or any combination thereof. The treatment can include a taking of a medication. The medication may be at least orally administered, intravenously administered, or topically applied. For example, for schizophrenia, the user may include a typical antipsychotic (e.g., haloperidol, chlorpromazine, fluphenazine, perphenazine, loxitane, thioridazine, or trifluoperazine) or an atypical antipsychotic (e.g., aripiprazole, risperidone, clozapine, quetiapine, olanzapine, ziprasidone, lurasidone, paliperidone, or iclepertin), among others. For affective disorders (e.g., PTSD or depression), the user may be on a serotonin reuptake inhibitor (SRI) or a mood-stabilizing drug (e.g., lithium, valproic acid, divalproex sodium, carbamazepine, or lamotrigine), among others. The application 125 may increase the efficacy of the medication that the user is taking to address the condition. The treatment can include a psychosocial intervention, such as psychoeducation, group therapy, cognitive-behavioral therapy (CBT), early intervention for first-episode psychosis (FEP), cognitive rehabilitation, or educational plans, among others.

The application 125 can include, present, or otherwise provide a user interface 130 including the one or more UI elements 135 to a user of the user device 110 in accordance with a configuration on the application 125. The UI elements 135 may correspond to visual components of the user interface 130, such as a command button, a text box, a check box, a radio button, a menu item, and a slider, among others. In some embodiments, the application 125 may be a digital therapeutics application and may provide one or more sessions (sometimes referred to herein as a therapy session) via the user interface 130 for addressing functional impairments in users.

The application 125 can receive an instruction for presentation of a session to the user. The session can include or be defined by a corresponding layer configuration 170 provided from the database 160. The layer configuration 170 can correspond to or include layers, therapy techniques, images, prompts, or other displays for presentation to the user via the application 125 during a session. The session can include an interactive interface, through the user interface 130, to engage the user in one or more therapies designed to improve cognitive functioning of the user associated with the condition. For example, the user may play a game on the user device 110 presented by the application 125 which incorporates one or more therapies to address functional impairment. Each session can correspond to a layer. In some embodiments, each session corresponds to one layer. The layer can refer to the difficulty and type of content being displayed during the session. For example, a first session can include a first layer. The first layer may be configured to display a set of first images corresponding to a first therapy technique. The layer can include instructions for presentation of the images 180, the audio recordings 185, the prompts, the UI elements 135, or other visual displays associated with the session.

In some embodiments, the application 125 can present three sessions, each corresponding to a respective layer, to address an impairment associated with the condition of the user. Each layer associated with its respective session can increase in difficulty, such as in actions or queries prompted. The first layer can include prompts and images 180 or the audio recordings 185 for presentation to the subject through a mobile device of the subject, such as the user device 110. For addressing social processing impairment, for example, the image 180 can be of a character and a prompt may direct the subject to select a social cue of the character depicted in the image 180 (or the audio recording 185) to train the subject to recognize social cues. For addressing non-social processing impairment, for example, the image 180 (or the audio recording 185) can be of an object and a prompt may direct the subject to select which object is depicted to address attention and memory in the subject. The first layer can be a cognitive training layer to provide cognitive exercises to teach the subject social skills through repetition. The subject can be presented with a first image 180A of the images 180 depicting a social setting, character, or social cues. In some embodiments, the first layer may be a cognitive training layer associated with a non-social skill such as verbal memory to present one or more audio recordings 185 in which the user is to recall one or more of a plurality of words presented in the audio recordings 185.

The first session associated with the first layer can provide a prompt regarding the social cues depicted in the first image 180A (or the audio recording 185A or both the first image 180A and the audio recording 185A) and the UI elements 135. The subject can select one or more of the UI elements 135 based on the social settings or cues depicted. One or more of the UI elements 135 can be associated with a correct selection and one or more of the UI elements 135 can be associated with an incorrect selection. In some cases, each selection may include varying degrees of correctness. For example, the first session can include two images. A first image 180A can depict a person actively cooking and a second image 180B can depict a person simply sitting on a couch. The subject may be prompted to select the image which depicts a busy person. In this illustrative example, the correct selection would be the first image 180A depicting the person cooking. A series of images 180 (or audio recordings 185) and prompts such as the aforementioned example can be provided in the first layer to enforce knowledge of the social and non-social skills through repetition.

A second layer of the three layers can correspond to a second session. The second session can provide, via the mobile device of the user, second images 180B (or audio recordings 185B or both the second images 180B and the audio recordings 185B) and second prompts. The second session can include a set of interactive elements, such as the UI elements 135. The second image 180B can present a social setting as a virtual environment. For example, the second image 180B can present a virtual social setting through the application 125. In this manner, the second layer can provide virtual training which incorporates relatable, realistic aspects of daily living, with the aim of addressing functional impairment. The second session can include a prompt indicating a question associated with a character depicted in the social setting and exhibiting the social cues.

For a social processing example, the second layer can include a presentation of text depicting a social setting in which a character is standing in a doorway, unknowingly and unintentionally blocking the subject from leaving a room. The second prompt may include a question, such as "How can you leave the room?" In this example, the interactive elements can include text such as "1. Ask the person politely to move," "2. Push past the person," or "3. Say nothing and wait for the person to move." A correct response by the subject can include a selection, via the interactive elements, indicating an appropriate social interaction based on the social situation depicted. In this example, the correct selection can be selection 1.

For addressing non-social processing, for example, the image 180B can be of multiple characters in a setting and a prompt may direct the subject to select a response, with the objective of training the user to exercise executive functions (e.g., planning and problem solving) in the virtual environment. In another example, with the objective of training the user to exercise verbal memory, an audio recording 185 of a conversation may be presented to the user, and the user may be prompted to provide a response regarding information conveyed in the conversation.

A third layer can correspond to a third session in which a third image 180C (or audio recording 185C, or both the image 180C and the audio recording 185C) is presented to the user. The third session may not include the third image 180C and may include only a prompt. The third image 180C can include a third prompt for an activity (e.g., action) for the user to complete in the real world. For example, to address social processing impairment that leads to functional impairments, the third prompt can instruct the user to smile at a stranger, or to ask a librarian where to find a book. The third session can prompt the subject for a response associated with performance of the activity. For example, the third session can prompt the subject for an indication of the completion of the task, such as an evaluation of how the activity went or whether or not the subject completed the activity. For addressing non-social processing impairment that leads to functional impairments, the prompt can direct the subject to perform an activity along with planning to perform the activity by a specified time, with the aim of training the user to exercise executive functions (e.g., planning) in the real-world environment. As an example, to exercise verbal memory in a real-world environment, the prompt can ask the user to follow directions in a how-to video such as making a sandwich or can ask the user to have a conversation and later recalling the person's name.

The images 180 can be or include a display to be presented, as an image, as a video, or other visual presentation to the user. The images 180 can be subdivided into sets, such as a first set of images 180A or a second set of images 180B. In some embodiments, a set of images 180N can include still images, videos, text, or a combination thereof. One or more images 180 can repeat across the sets. The images 180 can include live, pre-recorded, or generated videos or animations, such as video recordings, animated shorts, or animated images, among others. The image 180 can include audio or haptic presentations. For example, the images 180 can include a sound, phrase, word, or other auditory noise to present to the user. The images 180 can be in any size or orientation executable by the user interface 130. The images 180 can include text, such as a word or sentence to be presented to the user via the user interface 130. The images 180 can include instructions for the user to perform an action as a part of an ecologically valid cognitive remediation. For example, the images 180 can include text, graphics, or an auditory instruction which depict an action for the user to take or perform in relation to the session. The images 180 can depict social settings, people, animals, objects, among others, or a combination thereof. The images 180 can be coupled with or correspond to one or more prompts.

The audio recordings 185 can be or include one or more files corresponding to audio content to be presented to the user. The audio recordings 185 can include live, pre-recorded, or generated audio, such as sound recordings of speech from an individual or a conversation between two or more human individuals. For example, each audio recording 185 may be a recording of one or more words, one or more sentences spoken by a single speaker, or a set of sentences in conversational form by two or more individuals, among others. In some embodiments, the audio recordings 185 may include audio generated using speech synthesizer or generative audio. In some embodiments, at least one audio recording 185 may be associated with an image 180. In some embodiments, at least one audio recording 185 may be associated with or correspond to one or more prompts. The files used for the audio recordings 185 may include, for example, a waveform audio file format (WAV), an MPEG file format (MP3), audio interchange file format (AIFF), or Ogg Vorbis (OGG), among others.

The prompts can include instructions or an indication for the user to perform an action or make a selection to address the functional impairments associated with the condition. The prompts can be associated with the images 180 (or audio recordings 185, or both the images 180 and the audio recordings 185). The prompt can be displayed within the image 180, overlapping the image 180, or adjacent to the image 180, among others. The prompt can include a query related to one or more of the images 180 displayed through the application 125 operating on the user device 110A. The prompt can include text instructing the user to interact with one or more of the UI elements 135. For example, the prompt can propose a query to the user based on the image 180 displayed on the user interface 130 by the application 125 and can instruct the user to make a selection of the UI elements 135 based on the image 180 and the query. The prompt can include instructions for an action or task to be performed by the user in accordance with a session.

An action related to the session can be included in the layer configurations 170. The action can include interacting or not interacting with the user device 110. For example, the action can include tilting the user interface 130 or selecting a UI element 135 presented by the user device 110. The action can include a physical task to be performed by the user. For example, the action can include the user leaving the house, conversing with a cashier, or purchasing milk. The action can include instructions for the user to address the condition. The action can be included in the session to address the functional impairment associated with the user's condition. For example, the prompt can instruct the user to perform an action outside of the virtual environment, such as physically going to a coffee shop and buying a coffee. One or more actions can be associated with the layer configurations 170 stored in the database 160.

The database 160 may store and maintain various resources and data associated with the session management service 105 and the application 125. The database 160 may include a database management system (DBMS) to arrange and organize the data maintained thereon. The database 160 may be in communication with the session management service 105 and the one or more user devices 110 via the network 115. While running various operations, the session management service 105 and the application 125 may access the database 160 to retrieve identified data therefrom. The session management service 105 and the application 125 may also write data onto the database 160 from running such operations.

Such operations may include the maintenance of the user profile 165 (sometimes herein referred to as a subject profile). The user profile 165 can include information pertaining to a condition of a user, as described herein. For example, the user profile 165 may include information related to the severity of the condition, occurrences of the condition (such as occurrences of symptoms associated with the condition affecting the cognitive functioning of the user), medications or treatments the user takes for the condition, and/or a duration of the condition, among others. The user profile 165 can be updated responsive to a schedule, periodically (e.g., daily, weekly), responsive to a change in user information (e.g., input by the user via the user interface 130 or learned from the user device 110), or responsive to a clinician (e.g., a doctor or nurse) addressing the user's condition, among others.

The user profile 165 can store and maintain information related to a user of the application 125 through user device 110. Each user profile 165 may be associated with or correspond to a respective subject or user of the application 125. The user profile 165 may contain or store information for each session performed by the user. The information for a session may include various parameters, actions, the images 180, prompts, the layer configurations 170, or selections or actions of previous sessions performed by the user and may initially be null. The user profile 165 can enable streamlined communications to the user by presenting a session to the user which, based on at least the user profile 165, is most likely to aid the user in reducing the functional impairment or improving cognitive functioning. This directed approach can reduce the need for multiple communications with the user, thereby reducing bandwidth and increasing the benefit of the user-computer interaction.

In some embodiments, the user profile 165 may identify or include information on a treatment regimen undertaken by the user, such as a type of treatment (e.g., therapy, pharmaceutical, or psychotherapy), duration (e.g., days, weeks, or years), and frequency (e.g., daily, weekly, quarterly, annually), among others. The user profile 165 may be stored and maintained in the database 160 using one or more files (e.g., extensible markup language (XML), comma-separated values (CSV) delimited text files, or a structured query language (SQL) file). The user profile 165 may be iteratively updated as the user provides responses, makes selections, and performs actions related to the session, the layer configurations 170, or the images 180, among others.

The layer configuration 170 can identify or include a set of instructions defining each layer to be provided as a session through the user interface 130 for the application 125 on the user device 110. The layer configuration 170 can correspond to or include layers, therapy techniques, images, prompts, or other displays for presentation to the user via the application 125 during a session. In some embodiments, the layer configuration 170 may be for social skills training. For example, for the first layer, the layer configuration 170 can identify files corresponding to images 180 of characters and define prompts for candidate choices of social cues detected in the image 180. For the second layer, the layer configuration 170 can identify files corresponding to images 180 of social settings with one or more characters and define prompts for candidate choices of responses to the setting depicted in the image 180. For the third layer, the layer configuration 170 can identify prompts for directing users of the application 125 to perform activities in the users' surrounding (e.g., an interaction with another).

In some embodiments, the layer configuration 170 may be for non-social skills training such as verbal memory training to address impairment in verbal learning and memory as well as cognitive association. For instance, for the first layer, the layer configuration 170 can identify files corresponding to images 180 and audio recordings 185 and define prompts for candidate choices of one or more words presented in the audio recordings 185. For the second layer, the layer configuration 170 can identify files corresponding to images 180 or audio recordings 185 of conversations between individuals in virtual settings and define prompts for candidate choices of responses associated with information presented in the conversation. For the third layer, the layer configuration 170 can identify prompts for directing users of the application 125 to perform activities in the users' surrounding (e.g., an interaction with another). The layer configuration 170 can be maintained in the database 160 using one or more files (e.g., extensible markup language (XML), comma-separated values (CSV) delimited text files, or a structured query language (SQL) file, among others).

Identifications of the images 180, the audio recordings 185, the layer configurations 170, the actions, or the prompts may be stored and maintained on the database 160. For example, the database 160 may maintain the images 180 and the audio recording 185 using one or more data structures or files. Each of the images 180 may prompt the user via the application 125 to perform an action or make a selection via the application 125. For example, the application 125 may receive instructions to present a layer including one or more images 180. The layers and sessions may be used to provide therapies to improve cognitive functioning such as social or non-social skills, symptoms of the condition, or other cognitive or behavioral effects of the condition, among others. The sessions may be presented as games, activities, or actions to be performed by the user via the user interface 130. For example, one or more of the sessions may be presented as a sequence of the images 180 with prompts including queries for the user to select an interactive element (e.g., of the UI elements 135) associated with the query and images 180.

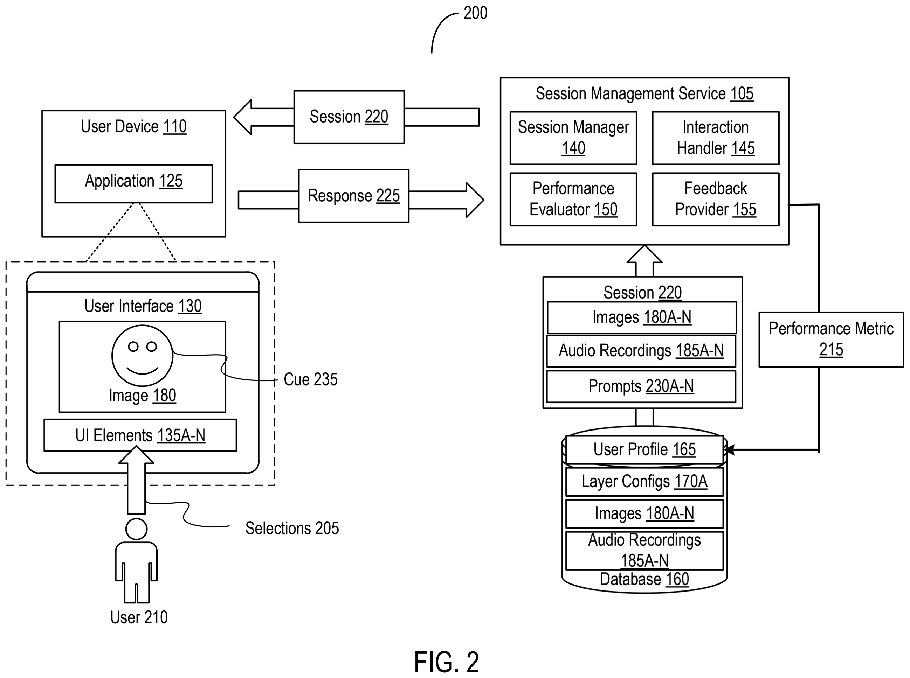

Referring now to FIG. 2, depicted is a block diagram for a process 200 to identify a set of sessions to address functional impairment in users and to provide a first session for a cognitive training layer of the set of sessions. The process 200 may include or correspond to operations performed in the system 100 to present interactive sessions to address functional impairment in users. Under the process 200, the session manager 140 executing on the session management service 105 may access the database 160 to retrieve, fetch, or otherwise identify the user profile 165 for a user 210 (sometimes herein referred to as a subject, patient, or person) of the application 125 on the user device 110. The user profile 165 may identify or define information associated with the user 210, the instance of the application 125 on the user device 110, and the user device 110, among others. For example, the user profile 165 may identify that user 210 has a functional impairment, symptoms associated with a condition, or other cognitive or behavioral results from the condition. The user profile 165 may identify taking of medication by the user 210 to address the condition or associated symptoms of the condition in the user 210, among others.

The session manager 140 may determine or identify a set of sessions for the user 210 for addressing functional impairments associated with the condition. Each session may correspond to a respective layer to address an impairment associated with the condition of the user 210. Each layer may correspond to one or more cognitive remediation trainings to help the user 210 overcome or improve the functional impairment. For example, each layer can include a different therapy designed to teach the user 210 social skills or to recognize social cues. The session manager 140 can identify the sessions to functional impairments associated with the condition of the user 210 associated with the user profile 165.

The user profile 165 may include information on the images 180, the layer configurations 170, prior sessions (such as previous selections identified for the user 210), a performance associated with the session already identified for the user 210, or a taking of medication by the user 210 to address the condition of the user, among others. The user profile 165 may also identify or include information on recorded performance of functional impairment, such as a number of occurrences of failed social interactions, symptoms associated with the condition, a number of occurrences of engaging in a social setting, durations of prior occurrences, and taking of medication, among others. The user profile 165 may initially lack information about prior sessions and may build information as the user 210 engages in the sessions via the application 125. The user profile 165 can be used to select the one or more sessions to provide via the application 125 to the user 210 in the session.

The session manager 140 may initiate a session responsive to receiving a request from the user 210 via the application 125. The user 210 may provide, via the user interface 130 to execute through the application 125, a request to start a session. The request may include information related to the functional impairment. The request can include attributes associated with the condition, such as an identification of the user 210 or the user profile 165, symptoms associated with the condition of the user 210, a time of the request, or a severity of the condition, among others. The application 125 operating on the user device 110 can generate the request to start the session to send to the session management service 105 in response to an interaction by the user 210 with the application 125, such as by the user 210 choosing one or more selections 205 through the UI elements 135. In some embodiments, the session manager 140 may initiate or identify the sessions responsive to a scheduled session time, responsive to the completion of a prior session, or based on the user 210 taking a prescribed medication to address the condition, among others.

In some embodiments, the session manager 140 may identify the sessions based on a predefined schedule of sessions. For example, the session manager 140 may identify a first session to include a cognitive training skills layer associated with the condition in accordance with the predefined schedule of tasks. In this illustrative example, the session manager 140 can identify a second session based on the subsequent session of the predefined schedule. For example, a predefined schedule of sessions may include a first, second, and third session corresponding to different psychosocial therapies. The session manager 140 may define a schedule or time at which to identify or provide the sessions or at which to mark the sessions for presentation. In some embodiments, the session manager 140 can identify the sessions based on a set of rules. The rules may be configured to provide the sessions to target the underlying causes of the condition or train the user 210 to improve social and non-social skills in the user 210 in a systematic, objective, and therapeutically effective manner. The rules may be based around time of completion of an action or selection 205 associated with the sessions, the user profile 165, the prompts, or other attributes of the system 100.

Upon identification, the session manager 140 may provide, send, or otherwise transmit one or more of the sessions to the user device 110. In some embodiments, the session manager 140 may send an instruction for presentation of the sessions and their corresponding images and prompts via the user interface 130 for the application 125 on the user device 110. The instruction may include, for example, a specification as to which UI elements 135 are to be used and may identify content to be displayed on the UI elements 135 of the user interface 130. The instructions can further identify or include the images 180. The instructions may be code, data packets, or a control to present the session to the user 210 via the application 125 running on the user device 110.

The session manager 140 can create, write, or otherwise generate an instruction for a session 220 in accordance with the layer configuration 170 for the identified session 220. The session 220 can be a first session and can include a cognitive training layer, such as stored in the layer configuration 170A. For the session 220, the session manager 140 may select or identify a set of images 180A-N (hereinafter generally referred to as the images 180), a set of audio recordings 185A-N (hereinafter generally referred to as audio recordings 185), and prompts 230A-N (hereinafter generally referred to as prompts 230) for display during the session 220. The session manager 140 can select the images 180 and the prompts 230 based on the layer configuration 170A, the user profile 165, or a prior session, among others. For example, the session manager 140 can select the images and the prompts 230 to address verbal memory, social skills, or other domains in which the user may suffer from an impairment. The session manager 140 may identify or select the images 180 and the prompts 230 as a part of a session to provide a cognitive training layer for the user 210 experiencing the functional impairment associated with the condition.

In some embodiments, the session manager 140 may select or identify a set of audio recordings 185 for the session 220. The selection may be based on the layer configuration 170A, the user profile 165, or a prior session, among others. Each recording 185 may correspond to or be associated with one or more words. For example, the set of selected recordings 185 may correspond to a list of words to be presented to the user 210. The session manager 140 may also identify or specify a format in which the set of audio recordings 185 are to be presented. The format may identify or define a context in which the words in each audio recording 185 are to be presented. For example, the format may include a list of words or a sentence, among others.

The session manager 140 may provide the instructions for the session 220 to display of the images 180, the audio recordings 185, and the prompts 230 on the application 125. The session manager 140 may transmit the session 220 to the user device 110 for execution through the application 125. The session manager 140 may provide the images 180 or the audio recordings 185 (or both) as a part of the session 220. The session manager 140 may provide the prompts 230A-N as a part of the session 220 in at least partial concurrence with the presentation of the images 180 or the audio recording 185. The instructions may also include the prompts instructing the user 210 to make a selection 205 or perform an activity in relation to their session. For example, the prompts 230 may display a message instructing the user 210 to make a selection 205 of the UI elements 135. The images 180 may include a text, image, audio, or video presented by the user device 110 via the application 125. For example, the images 180 may include presentation of an image instructing the user 210 to interact with the application 125 via the user interface 130.

The session manager 140 may provide the prompts 230 as a part of the session 220 in at least partial concurrence with the presentation of the images 180 or the audio recording 185. The instructions may also include the prompts 230 instructing the user 210 to make a selection 205 to indicate which one or more words were presented during the presentation of the audio recordings 185. For example, the prompts 230 may display a message instructing the user 210 to make a selection 205 of the UI elements 135. At least some of the images 180 may correspond to the words presented in the audio recordings 185, and at least some of the images 180 may not correspond to any of the words presented through the audio recordings 185. For example, the images 180 may depict different fruits, when at least one of the audio recordings 185 presented the word "banana".

The application 125 on the user device 110 may render, display, or otherwise present one or more of the sessions. The sessions 220 may be presented via the one or more UI elements 135 of the user interface 130 of the application 125 on the user device 110. The presentation of the UI elements 135 can be in accordance with the instructions provided by the session manager 140 for presentation of the session 220 to the user 210 via the application 125. In some embodiments, the application 125 can render, display, or otherwise present aspects of the session 220, such as an image 180, the audio recordings 185, or prompts, independently of the session management service 105.

The application 125 may render, display, or otherwise present the images 180 and the audio recordings 185 for the session 220. The images 180 can include one or more cues 235 to teach or train the user 210 to recognize such cues 235 for a social skill. The cues 235 can relate to or include social cues. A social cue, as described herein, can be an indication of social behavior exhibited by a character or person. In some embodiments, neurotypical people (e.g., people not experiencing the condition associated with the user 210), can easily recognize social cues and can make social decisions or interactions based on observed social cues. For example, the user 210 experiencing the condition may have difficulty understanding social cues.

The cues 235 can include at least one of a head movement (e.g., nodding, shaking, tilting, or other head gestures), a body language (e.g., facial expressions, overall body posture, and proximity with respect to another character), a gesticulation (e.g., pointing using a finger or expressing emotion through hands or fingers), or an eye contact (e.g., orientation of eye with respect to another person to show engagement or emotion), among others. For example, the image 180 displayed on the user interface 130 via the application 125 can depict social cues 235 such as a character nodding her head, a character smiling, a character making eye contact, a character avoiding eye contact, a character with her arms crossed, a character waving, a character frowning, a character talking with her hands, or a character shaking her head, among others. In some embodiments, the social cues 235 can be textually described in the image. For example, the image 180 can include text describing a character's body language. In some embodiments, the image 180 presented may lack the cue 235. For example, when the application 125 played back a set of recordings 185, the image 180 displayed by the application 125 may lack any social cues 235.

In conjunction, the application 125 may present the prompts 230A-N associated with the session 220. The prompts 230 can include text or images associated with the images 180. The prompts 230 can indicate a selection 205 for the user 210 to choose. In some embodiments, the prompts 230 can include or be coupled with the UI elements 135A-N. The UI elements 135A-N can identify one or more of the types of the social cues 235 depicted in the image 180. The types of the social cues 235 can include the physicality of the cue 235 (e.g., head movement, body language, a gesticulation, or an eye contact, among others) or a context of the cue 235 (e.g., the cue 235 indicating that a character is busy, annoyed, in a hurry, not paying attention, etc.). The application 125 may share or have the same functionalities as the session manager 140, the interaction handler 145, the performance evaluator 150, or other components of the session management service 105 as discussed above. For example, the application 125 may maintain a timer to keep track of time elapsed since presentation of a previous session.

In some embodiments, the application 125 may display, render, or otherwise present the images 180 or prompts for different time periods. The application 125 may present a first image 180A for a first time period and a second image 180B for a second time period. For example, the application 125 may present the first image 180A during the first time period and then present the second image 180B during the second time period. In some cases, the application 125 may delay the presentation of the second image 180B during the session after displaying the first image 180A. The application 125 may present the images 180A and 180B or the prompts concurrently. Presenting the images 180 or the prompts concurrently can refer to displaying the images 180 or the prompts during a concurrent time period, or with a concurrent display position on the user interface 130. In conjunction, the application 125 may display the prompts 230A-N to direct the user 210 to interact with the display. The images 180 or the UI elements 135A-N may include prompts for the user 210 to perform an action or selection 205 associated with the session 220. The selection 205 can include actions such as a physical manipulation of the user device 110, actuation of the UI elements 135, or viewing of a video or image of the images 180, among others.

In some embodiments, the session manager 140 can provide a series of images 180 (or audio recordings 185) and prompts 230 for the user 210 to provide the selections 205 based on the cues 235 depicted in the images 180. The user 210 can make a selection, via the UI elements 135, indicating the cue 235. For example, the image 180 can include a picture of a person shaking her head. The prompts 230 can be coupled with the UI elements 135 or separate and can instruct the user 210 to choose one or more of the UI elements 135 based on the cue 235 (e.g., head shaking, audio) exhibited in the image 180. For example, a first prompt 230A may be coupled with a first UI element 135A and may include text stating "Select me if she is saying 'yes'" and a second prompt 230B may be coupled with a second UI element 135B including text stating "Select me if she is saying 'no.'" In this manner of prompt selection based on the cue 235 associated with the presented image 180, the user 210 can iteratively perform training exercises to teach the user 210 to recognize social cues 235.

In some embodiments, the application 125 may play back or present the set of audio recordings 185 (e.g., via a loudspeaker or headphones) to the user 210. The presentation of the audio recordings 185 may be in accordance with the specified format defining the context in which the words are to be presented. For instance, the application 125 may sequentially play the set of audio recordings 185, when the format specifies that the words corresponding to the audio recordings 185 are a list of words. The application 125 may also play a single audio recording 185, when the format specifies that the words form a sentence to be presented to the user 210. In conjunction with or subsequent to the presentation of the audio recordings 185, the application 125 may present or display a prompt 230 on the user interface 130 for the user 210. The prompt 230 may allow the user 210 to provide a selection 205 to identify at least one of a set of words as presented in the set of recordings 185. The words may be presented in the form of text or images 180 representing the objects. The prompt 230 may include any number of inquiries, such as whether a word was presented; which word was presented; which word was not presented; which of these words were presented; one word from list, one from not; n number of words from list, one from list; and one from list and one not from list, among others. The prompt 230 may be presented in conjunction with or subsequent to the presentation of the audio recordings 185. In some embodiments, the application 125 may present at least one UI element 135 to allow the user 210 to replay the audio recordings 185.

In some embodiments, the audio recording 185 may accompany one or more images 180 to teach the user 210 to understand, comprehend, and remember auditory information such as words, sentences, and stories. As an illustrative example, a third prompt 230C may be coupled with multiple UI elements, such as the UI elements 135. The third prompt 230C may indicate to the user to select the UI element 135 which corresponds to the audio cue played for the user via a speaker coupled with the client device. For example, the audio cue may play the word "cat," and the third prompt 230C may ask the user 210 to select the UI element 135 corresponding to the audio cue. The UI elements 135C-E may include a respective text such as "horse," "dog," or "cat," and the user may select the UI element 135 which is believed to correspond to the third prompt 230C including the word "cat". In this manner of prompt selection based on the cue 235 associated with the presented image 180, the user 210 can iteratively perform training exercises to aid the user.

The application 125 may monitor for at least one selection 205 with the UI elements 135A-N. The application 125 can monitor during the session responsive to presentation of the images 180, presentation of the prompts 230, or responsive to receiving the selection 205. The application 125 can monitor for receipt of the selection 205. The application 125 can monitor for the selection 205 through the user interface 130 or through sensors associated with the user device 110, among others. The application 125 can receive multiple selections 205 during a session. For example, the application 125 can monitor for a series of selections 205 provided by the user 210 during the session. The application 125 may monitor and record information related to the received selections 205. For example, the application 125 may monitor and record a time of a selection 205, a duration of a selection 205, a sequence of selections 205, the prompt 230 or image 180 associated with the selection 205, and/or the delay time between the presentation of the prompts 230 or the image 180 and the selection 205, among others.

Upon the user 210 providing the selection 205, the application 125 may generate at least one response 225. The response 225 can identify the selection 205. The response 225 can include the information about the selection 205, such as a duration of the selection 205, a time of the selection 205, the image 180 or prompts 230 associated with the selection 205, and/or a delay time between the presentation of the image 180 or the prompts 230 and the selection 205, among others. The application 125 can generate the response 225 for transmittal to the session management service 105. The response 225 can be in a format readable by the session management service 105, such as an electronic file readable by the session management service 105 or data packets readable by the session management service 105, among others.

The interaction handler 145 can receive, identify, or otherwise detect a response 225 identifying the selection 205. The interaction handler 145 can receive the response 225 from the application 125. The interaction handler 145 can receive the response 225 at scheduled time intervals or as the selections 205 occur during the session 220. The interaction handler 145 can query or ping the application 125 for the response 225. The interaction handler 145 can receive multiple responses 225 during a time period. For example, the interaction handler 145 can receive a first response 225 indicating a first selection 205 and a second response 225 indicating a second selection 205.

The interaction handler 145 can store and maintain the response 225 including the selection 205 in the database 160. The interaction handler 145 can store information related to the response 225, including a time of the response 225, actions associated with the selection 205, the user profile 165 associated with the response 225, and the images 180 or prompts 230 associated with the response 225, among others. The response 225 may include or identify the selection 205 by the user 210 with the UI elements 135. The response 225 may identify a type of social cue 235 depicted in the image 180. In some embodiments, the response 225 may identify the one or more words presented through the audio recordings 185. The response 225 may include a time for task completion. For example, the response 225 may include that the user spent 4 minutes performing the action associated with the presentation of the session 220.

The response 225 can include a total time for completion of the session 220 and may also include a time of initiation of the session 220 and a time of completion of the session 220. The response 225 may include the UI elements 135 interacted with during the duration of the presentation of the session 220. For example, the response 225 may include a listing of buttons, toggles, or other UI elements 135 selected by the user 210 at specified times during the presentation of the session 220. The response 225 may include other information, such as a location of the user 210 while performing the session, such as a geolocation, IP address, GPS location, or triangulation by cellular towers, among others. The response 225 may include measurements such as measurements of time, location, or user data, among others.

In some embodiments, the performance evaluator 150 can generate a performance metric 215 based at least upon the selections 205, the response 225, the user profile 165, or prior sessions, among others. The performance metric 215 can be a qualitative (e.g., "poor" "average" "good") or quantitative (e.g., numerical) score to indicate the performance of the user 210 during the session 220 or a series of sessions. The performance evaluator 150 can generate a performance metric 215 based on a rate of correct selections 205 of the cognitive training layer. For example, the user 210 can be provided a series of images 180 (or audio recordings 185) and prompts 230 as a part of the session 220. The user 210 can provide one or more selections 205 during the session 220. In some cases, the selections 205 made by the user 210 can correctly identify the social cue 235 depicted in the image 180 and in some cases the selections 205 made by the user 210 may not correctly identify the social cue 235. In some embodiments, the selections 205 by the user 210 may identify (e.g., correctly, or incorrectly) the word presented via the audio recording 185. The performance evaluator 150 can determine the performance metric 215 based on a ratio of correct selections 205 to incorrect selections 205, a ratio of correct selections 205 to total selections 205, a ratio of incorrect selections 205 to total selections 205, a rate of correct or incorrect selections 205 during a time period, a rate of correct or incorrect selections 205 during the duration of the session 220, among others.

The performance evaluator 150 can determine whether the user 210 is performing at or above a threshold performance. The performance evaluator 150 can compare the determined performance metric 215 to a threshold performance. Upon the performance evaluator 150 determining the performance metric 215 to be below the threshold performance, the performance evaluator 150 may instruct the session manager 140 to maintain the current session 220. Conversely, when the performance evaluator 150 determines the performance metric 215 to be at or above the threshold performance, the session 220 can be considered complete or passed. Upon completing or passing the session 220, the session manager 140 may determine to present a second session to the user 210 via the application 125. For example, upon the performance metric 215 satisfying a threshold, the session manager 140 can determine to transition the user 210 from the cognitive training layer associated with the session 220 to a different layer, such as a virtual functional training layer.

In addition, the feedback provider 155 may produce, output, or otherwise generate feedback for the user 210 to receive via the application 125 operating on the user interface 130. The feedback provider 155 may generate the feedback based on at least the performance metric 215, the response 225, the prompt 230 including a query, the user profile 165, or the historic session, image 180, the audio recording 185, or prompt presentation. The feedback may include text, video, or audio to present to the user 210 via the application 125 displaying through the user interface 130. The feedback may include a presentation of the performance metric. The feedback may display a message, such as a motivating message, suggestions to improve performance, a congratulatory message, or a consoling message, among others. In some embodiments, the feedback provider 155 may generate the feedback during the session being performed by the user 210. In some embodiments, the feedback may include an indication of the correct response for the query in the prompt 230.

The interaction handler 145 may provide, send, or otherwise transmit the feedback to the application 125 for display on the user interface 130. The interaction handler 145 may provide instructions for rendering or display of the feedback. The interaction handler 145 may transmit data packets, signals, or other instructions to indicate the presentation of the feedback to the application 125. Upon receipt, the application 125 can present the feedback to the user 210 via the user interface 130. The presentation of the feedback may include the UI elements 135. For example, the user 210 may make a selection related to the feedback, such as to increase the difficulty of the session or to resume the session 220. In some embodiments, the application 125 may also present the feedback to indicate the correct response. For example, if the user's selection is incorrect, the application 125 may set a color of the UI element 135 corresponding to the user's selection to indicate that the user's selection as incorrect (e.g., set to red). The application 125 may also set a color of the UI element 135 corresponding to the correct response to a different color (e.g., set to green). Otherwise, if the user's selection is correct, the application 125 may set a color of the UI element 135 corresponding to the user's selection to indicate that the user's selection as correct (e.g., set to green).

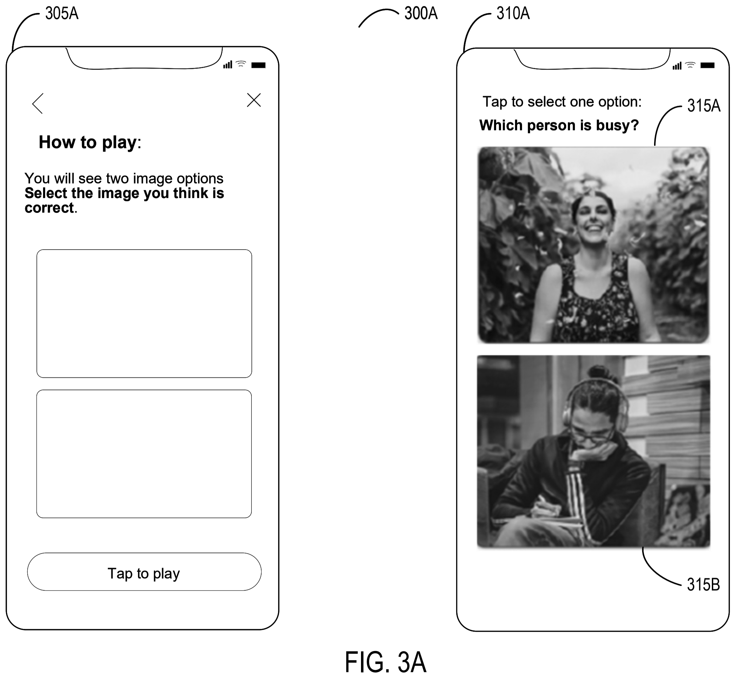

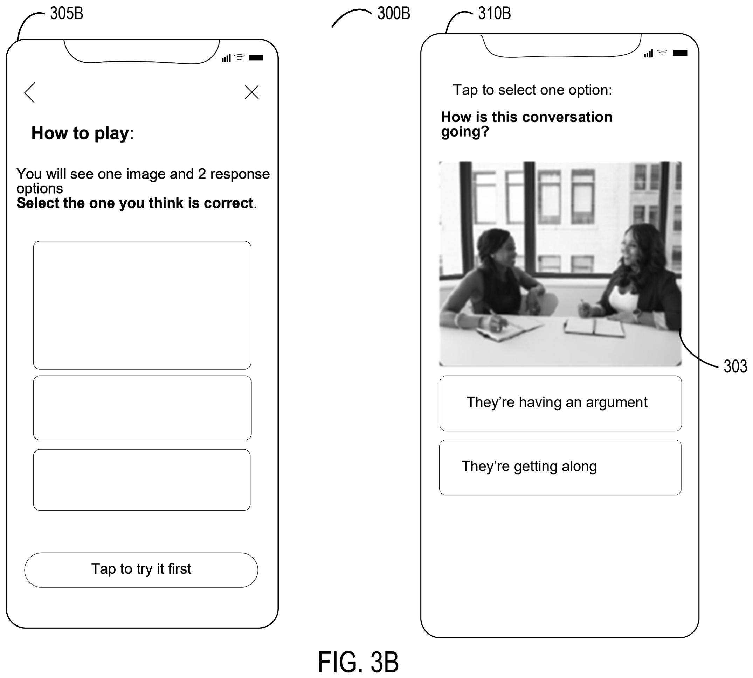

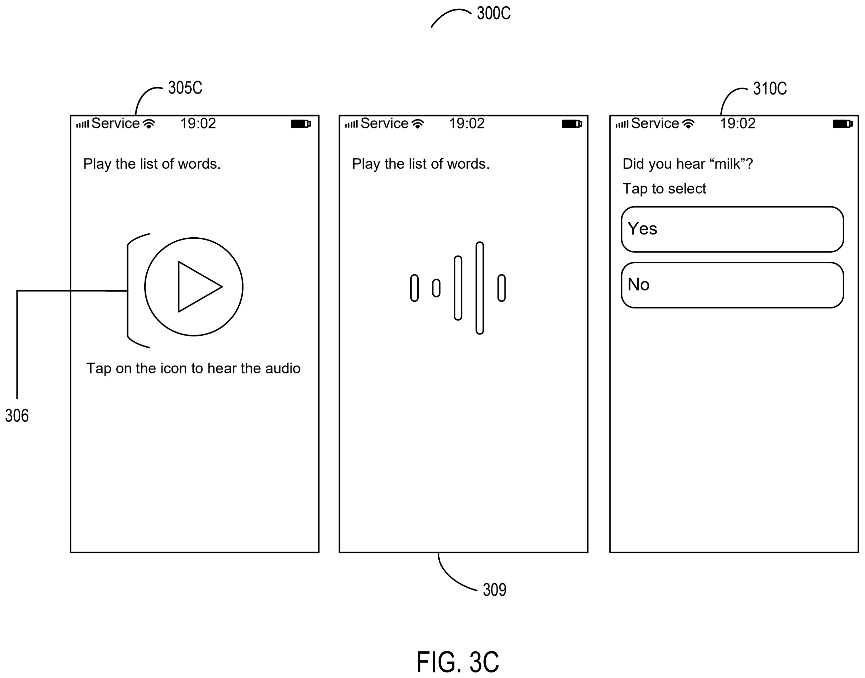

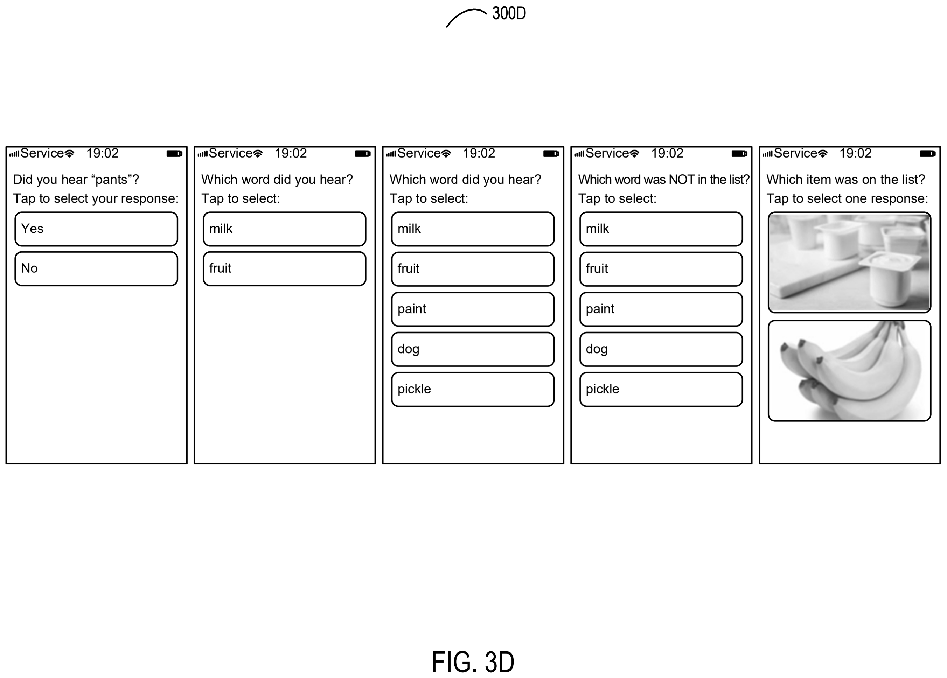

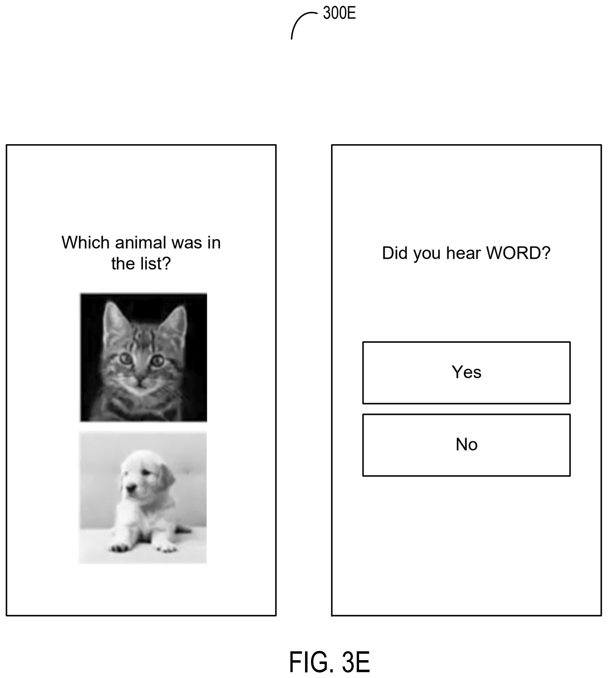

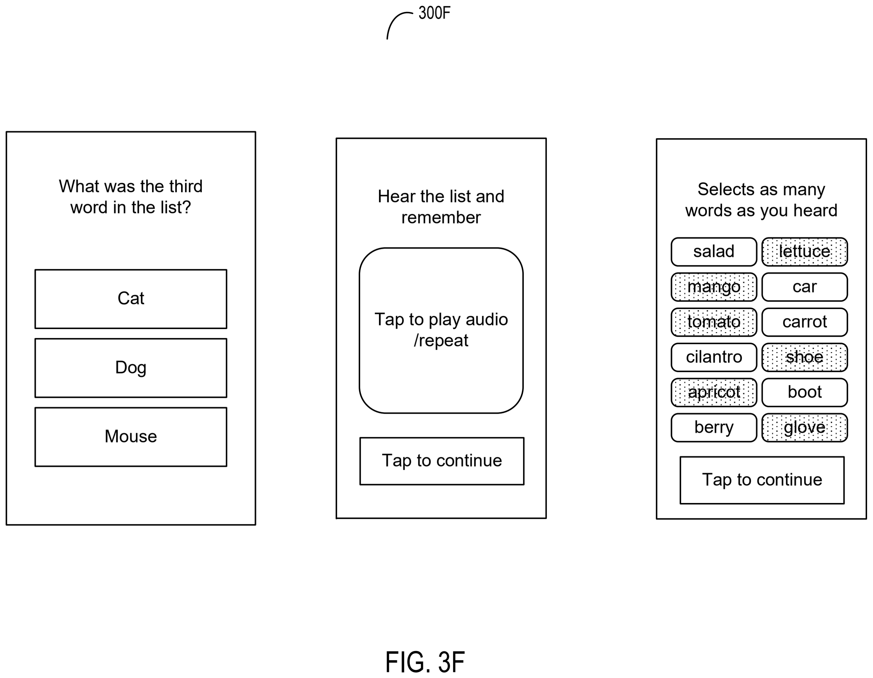

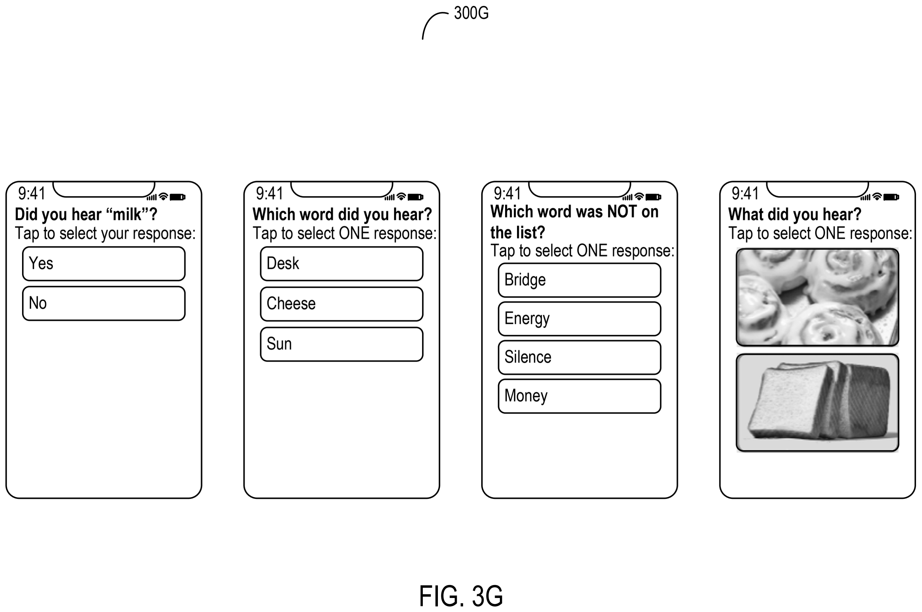

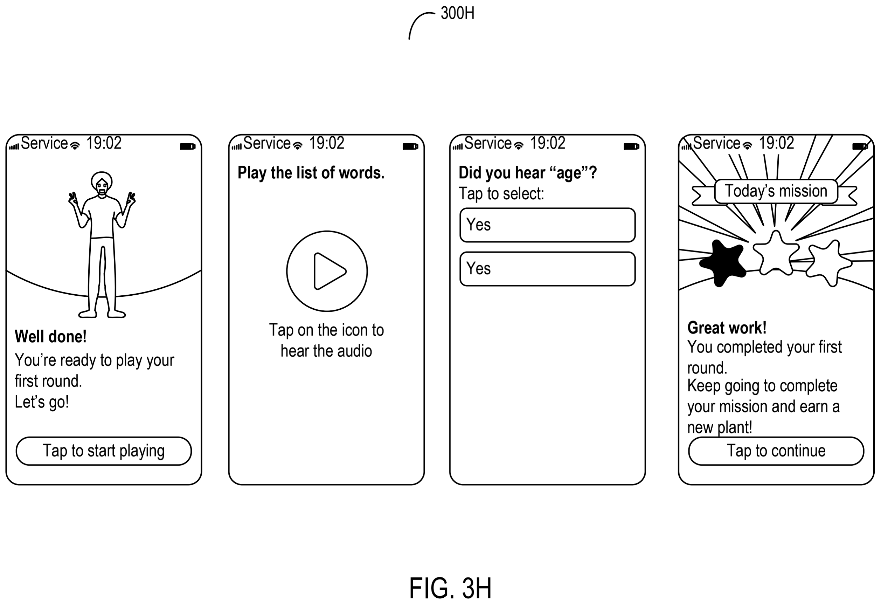

Referring now to FIGS. 3A-3H, depicted are example sets 300A-H of user interfaces for providing a first session. The set 300 can be presented via the application 125. The application 125 may present on a user interface or display such as the user interface 130 of the user device 110 in connection with the first layer. The first session depicted by the set 300 can relate to a cognitive training layer, such as depicted with reference to FIG. 2. In sets 300A-B, a user (e.g., the user 210) may interact with the user interfaces 305A-310B. The user interfaces 305A-310B may include one or more user elements, such as the UI elements 135, to accept input by the user 210 using at least one hand 525. The interface 305A and 305B can be a prompt to inform the user how to perform the subsequent task. The interface 310A and 310B can include a pair of images 315A and 315B or a single image 303 with characters, along with a prompt for the user to select a character with a particular social cue (e.g., busy, state of conversation flow, and attention).

In set 300C, the user interface 305C may include a play button 306 to start playing a set of audio recordings 185 corresponding to a list of words. Upon the button being pressed, the application 125 may present the user interface 309, in conjunction with the playback of the set of audio recordings 185. Upon completion of the playback of the audio recordings, the application 125 may present the user interface 310C to prompt the user 210 whether a certain word (e.g., "milk") was presented. The set 300D may be a continuation of the set 300C. Through user interfaces, the application 125 may continue to prompt the user 210 to select whether a certain word was played. The application 125 may also present the user interface to prompt the user 210 whether a certain word was not played. In addition, the application 125 may present the user interface to prompt whether a certain word was played using images depicting various words (e.g., yogurt and banana as depicted). The user interfaces shown in sets 300E-H may have the user go through similar exercises. For example, in set 300E, the user may be prompted to indicate whether a word was presented, and in set 300F, the user may be prompted to indicate whether a word was presented at a particular position within the list of words played.

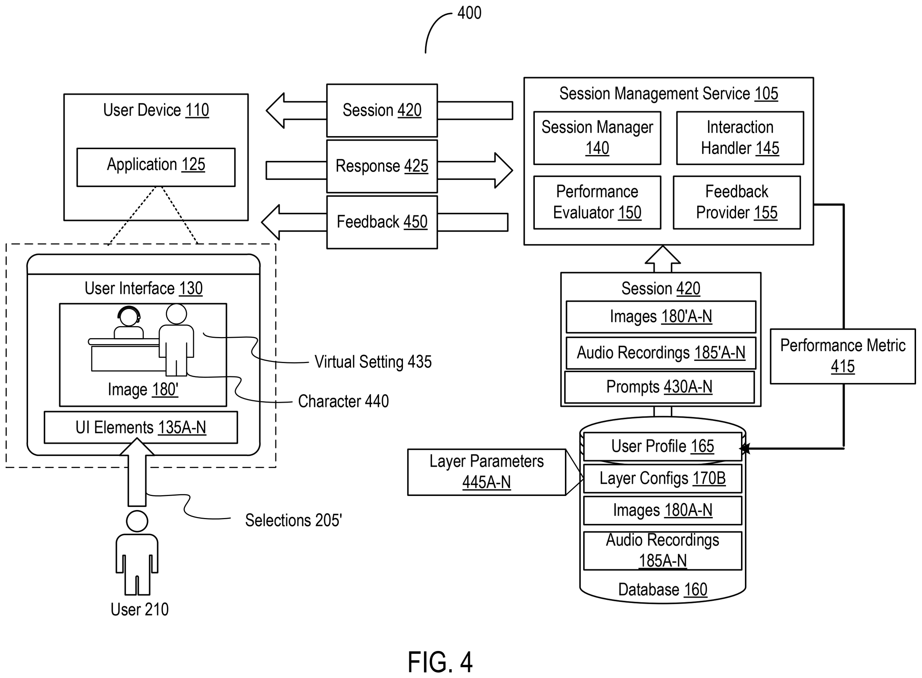

Referring now to FIG. 4, depicted is a block diagram for a process 400 to provide a second session for a virtual functional training layer for the user to apply the social skill in virtual social settings. The process 400 may include or correspond to operations performed in the system 100 or the process 200. Under the process 400, the session manager 140 may provide a session 420 corresponding to a virtual functional training layer. The interaction handler 145 can receive a response 425 indicating a set of selections 205' from the user 210. The performance evaluator 150 can generate or determine a performance metric 415 for the session 420. The feedback provider 155 may generate feedback 450 to provide to the user 210 based on their performance during the session 420. The interaction handler 145 may transmit the feedback 450 to the application 125 for presentation to the user 210.

The session manager 140 can provide the session 420 for presentation to the user 210 via the application 125. The session manager 140 can provide the session 420 using similar operations as in the process 200 as described in conjunction with the session 220 in FIG. 2. The session 420 can include the images 180'A-N (hereinafter referred to generally as the images 180'), the audio recordings 185'A-N (hereinafter referred to generally as the audio recordings 185'), and the prompts 430A-N (hereinafter referred to generally as the prompts 430). The images 180' and the prompts 430 can be like or similar to the images 180' and the prompts 230.

The session 420 can be a second session corresponding to a virtual functional training layer. The virtual functional training layer can provide a virtual setting 435 including one or more characters 440 for presentation to the user 210 within the image 180'. The virtual functional training layer can serve to provide virtual, artificial, or otherwise generated environments to display through the user interface 130 to train the user 210 to identify and react to a virtual setting 435. In some embodiments, the second session may be a virtual functional training layer for the user 210 to apply a social skill (e.g., social perception) or non-social skill (e.g., verbal memory). In this manner, by providing a second session with a virtual setting 435, the session management service 105 can build upon the teaching of the prior session 220.

The virtual setting 435 can correspond to or refer to a virtual social setting. A social setting can include an environment in which one or more persons or characters 440 socially interact with each other. For example, a social setting can include a party, a shop, a school, an office, a workplace, a park, or other setting in which interactions with other characters 440 is common or expected. Interaction with other characters 440 can include conversation, written communications, physical movements (e.g., handshaking or hugging), or other forms of verbal or non-verbal communication. In some embodiments, the images 180' can depict the virtual setting 435 such as an office, shop, party, etc., including the one or more characters 440 via the user interface 130 as a part of the session 420. In some embodiments, the virtual setting 435 may be presented via the set of audio recordings 185' of conversations between two or more individuals. The virtual setting 435 may lack the images 180' and may lack depictions of characters 440 within the images 180'.

The one or more characters 440 can include persons displayed in the image 180'. The characters 440 can include librarians, cashiers, passersby, coworkers, children, police, clerks, waiters, mail people, among other persons who may be encountered in a social setting. The image 180' may depict the character 440 interacting with the virtual setting 435. For example, the image 180' may depict the character 440 purchasing a coffee, speaking with a policeman, or passing another character in a store. The image 180' may depict the character 440 performing or exhibiting social cues, such as the cues 235 depicted in relation to FIG. 2.

The session manager 140 may provide the session 420 including the prompts 430. The prompts 430 can correspond to the image 180' or facets thereof, such as the virtual setting 435 or the character 440. In some embodiments, the prompt 430 can include a query or question. The query or question may be displayed as text or a symbolic image or presented as audio via the user device 110. The query can correspond to the image 180'. The query may be associated with the character 440 depicted in the virtual setting 435. In some embodiments, the query identified by the prompt 430 can relate to cues depicted by the character 440 in the image 180'. For example, the prompt 430 may include a query such as "What should the character say to order a coffee?" or "Is the barista busy?" The prompt can include any queries related to the characters 440 depicted in the virtual setting 435 to enable the user 210 to determine an appropriate or correct response based on the image 180'.

In some embodiments, the session manager 140 may provide the images 180' (or the audio recordings 185') for presentation via the user interface 130 of the application 125 in a defined sequence. The session manager 140 may present one or more images 180' during the session 420. The images 180' of the defined sequence may include the same characters 440, the same virtual setting 435, different characters 440, different virtual settings 435, or a combination thereof. In some embodiments, the sequence of images 180' may depict a sequential or causal set of social cues or interactions. For example, a first image 180A' may depict a character ordering a coffee and a second image 180B' may depict the character subsequently receiving their coffee. As another example, a first image 180A' may depict a character obstructing another character, and a second image 180B' may depict the character moving out of the way of the other character. In some embodiments, the sequence of images 180' can be determined by the session manager 140 in accordance with a defined set of images 180', or the session manager 140 can determine the sequence of images 180' based on the user profile 165, a response 425 from the user 210, or a performance metric 415 associated with the user 210 during the session 420.

In some embodiments, the session manager 140 may provide instructions for the session 420 to present the images 180', the audio recordings 185', and the prompts 430 on the application 125. The session manager 140 may provide the images 180' or the audio recordings 185' (or both) as a part of the session 420. The session manager 140 may provide the prompts 430 as a part of the session 420 in at least partial concurrence with the presentation of the images 180' or the audio recording 185'. In some embodiments, the session manager 140 may provide one or more audio recordings 185' of a speech sample from at least one speaker in relation to a virtual setting 435. The speech sample may correspond to one or more sentences by the speaker, such as a statement, question, exclamation, request, command, or suggestion, among others. In some embodiments, the recording may be of a conversation between two or more speakers. The conversation may be speech between characters in a virtual setting 435. In some embodiments, the session manager 140 may provide instructions to include one or more images 180' associated with the set of audio recordings 185'. For instance, the image 180' may include a depiction of the virtual setting 435 and avatars corresponding to the speakers in the conversation presented through the audio recording 185'. In some embodiments, the image 180' may include a depiction of the virtual setting 435, without any characters. For example, the audio recording 185' may be a conversation about filling up a car, and the image 180' accompanying the presentation of the audio recording 185' may be of a gas station.

In some embodiments, the instructions may also include the prompts 430 instructing the user 210 to make a selection 205' to indicate which information was presented during the presentation of the audio recordings 185. The information may be deduced or associated with the words presented during the session 420. For example, the prompts may display a message instructing the user 210 to make a selection 205' of the UI elements 135. At least some of the images 180 may correspond to the information presented in the audio recordings 185, and at least some of the images 180 may not correspond to any of the information presented through the audio recordings 185. In some embodiments, the prompt 430 may identify or include a query associated with the conversation presented in the audio recordings 185'. The query of the prompt 430 may be to recall information conveyed in the speech sample in the audio recording 185 in the virtual setting 435.

Upon presentation of the one or more images 180' including the virtual setting 435 and the character 440, the user 210 can make a selection based on the prompts 430. The user 210 can make a selection 205' through the UI elements 135 to answer a prompt including a query related to the image 180'. In some embodiments, the UI elements 135 can include other prompts 430. The user 210 make select a UI element 135 in response to the presentation of the prompt 430 including the query, the image 180', the character 440, the virtual setting 435, or a combination thereof.

The application 125 can monitor for one or more selections 205' through the UI elements 135. The application 125 can monitor for the one or more selections 205' in a similar manner as described in relation to the selection 205 in FIG. 2. Upon detecting the selection 205', the application 125 can generate at least one response 425. The response 425 can identify the selections 205'. The response 425 can include information related to the selection 205', such as a duration of the selection 205', a time of the selection 205', the image 180' or prompts 430 associated with the selection 205', and/or a delay time between the presentation of the image 180' or the prompts 430 and the selection 205', among others. The application 125 can generate the response 425 for transmittal to the session management service 105. The response 425 can be in a format readable by the session management service 105, such as an electronic file readable by the session management service 105 or data packets readable by the session management service 105, among others.

In some embodiments, the application 125 may play back or present the set of audio recordings 185' (e.g., via a loudspeaker or headphones) to the user 210 as part of the session 420. The set of audio recordings 185' may include a recording of the speech sample from at least one speaker in relation to a social setting. In some embodiments, the recording may be of a conversation between two or more speakers. The speech sample may include one or more pieces of information that the user 210 will be asked about, subsequent to or in conjunction with the presentation of the audio recordings 185'. In some embodiments, the application 125 may display one or more images 180' in conjunction with the presentation of the audio recordings 185'. The images 180' may depict the social setting in which the characters are conversing.

In conjunction with or subsequent to the presentation of the audio recordings 185', the application 125 may present or display a prompt 430 on the user interface 130 for the user 210. The prompt 430 may allow the user 210 to provide a selection 205 to identify information presented in the conversation from the set of audio recordings 185'. The information may correspond to the semantic content deducible or otherwise associated with the one or more words presented in the set of audio recordings 185'. The prompt 430 may inquire the user 210, for example, to indicate whether a particular statement is true about the content presented in the audio recording 185'. The prompt 430 may be presented in conjunction with or subsequent to the presentation of the audio recordings 185'. In some embodiments, the application 125 may present at least one UI element 135 to allow the user 210 to replay the audio recordings 185'.

The interaction handler 145 can receive, identify, or otherwise detect the response 425 identifying the selection 205'. The interaction handler 145 can receive the response 425 from the application 125. The interaction handler 145 can receive the response 425 at scheduled time intervals or as the selections 205' occur during the session 420. The interaction handler 145 can query or ping the application 125 for the response 425. The interaction handler 145 can receive multiple responses 425 during a time period. For example, the interaction handler 145 can receive a first response 425 indicating a first selection 205' and a second response 425 indicating a second selection 205'.

The interaction handler 145 can store the response 425 including the selection 205' in the database 160. The interaction handler 145 can store information related to the response 425, including the time of the response 425, actions associated with the selection 205', the user profile 165 associated with the response 425, and the images 180', the audio recordings 185', or prompts 430 associated with the response 425, among others. The response 425 may include or identify the selection 205' by the user 210 with the UI elements 135. In some embodiments, the response 425 may identify a type of social cue depicted in the image 180, an appropriate social interaction to take given the virtual setting 435, or a social cue associated with the character 440. In some embodiments, the response 425 may indicate the information presented in the set of audio recordings 185'. The response 425 may include a time for task completion. For example, the response 425 may include that the user 210 spent four minutes performing the action associated with the presentation of the session 420.

In some embodiments, the response 425 can include a total time for completion of the session 420 and may also include a time of initiation of the session 420 and a time of completion of the session 420. The response 425 may include the UI elements 135 interacted with during the duration of the presentation of the session 420. For example, the response 425 may include a listing of buttons, toggles, or other UI elements 135 selected by the user 210 at specified times during the presentation of the session 420. The response 425 may include other information, such as a location of the user 210 while performing the session, such as a geolocation, IP address, GPS location, or triangulation by cellular towers, among others. The response 425 may include measurements such as measurements of time, location, or user data, among others.

The performance evaluator 150 can calculate, generate, or otherwise determine a performance metric 415 associated with the session 420 based on the response 425. The performance metric 415 can be or include the performance metric 215, depicted in FIG. 2. In some embodiments, the performance evaluator 150 can determine the performance metric 415 based on the prior performance metric 415. In some embodiments, the performance evaluator 150 may maintain separate performance metrics for each session or may produce a combined performance metric. In some embodiments, a high performance metric 415 can correspond to the user performing well (e.g., selecting correct selections 205') in the session 420.

The correct selections 205' can refer to selections which denote a correct response to the query posed by the prompt 430 regarding the virtual setting 435 and the character 440. A correct response 425 can correlate to a socially acceptable or polite, behaviorally normal, moral, or emotionally stable social interaction for the presented virtual setting 435. For example, a correct response to the query "How can you order coffee?" in a virtual setting 435 may be indicated by a UI element 135A depicting the words "Say 'I'd like to order a medium black coffee.'" In some embodiments, a correct response 425 may correspond to a selection of information deducible from the conversation in the set of audio recordings 185' presented to the user 210. For example, in a conversation where two interlocutors are discussing which item to buy at a grocery, the correct response 425 may be associated with an action related to buying the item discussed in the conversation.

Conversely, an incorrect response may be indicated by a UI element 135B depicting the words "Say 'Give me coffee'" or a UI element 135C depicting "Take someone else's coffee." In this manner, an incorrect response can be associated with a socially inappropriate action, an immoral action, a behaviorally abnormal action, or an emotionally unstable action. Examples of incorrect responses can relate to shouting, stealing, physical altercations, lying, or rudeness. In some embodiments, an incorrect response 425 may correspond to a selection of information not deducible from the conversation in the set of audio recordings 185' presented to the user 210. For instance, in a conversation where two individuals are discussing vacation plans, the incorrect response 425 may be associated with an incorrect amount for the budget for the vacation.

Based on whether the response 425 is correct or incorrect, the performance evaluator 150 may calculate, generate, or otherwise evaluate the performance metric 415 for the user 210 based on the selections 205' associated with the response 425. For example, the performance evaluator 150 can set the performance metric 415 for a given response 425 or session 420 as "1" when correct and "-1" when incorrect. In some embodiments, the performance evaluator 150 may identify a reaction time or a correctness of the user 210 in selecting the selections 205'. For example, the performance evaluator 150 may determine, from the response 225, that the user 210 is not performing one or more actions indicated by the prompts 430 or the image 180', or that the user 210 is not performing the actions within a threshold time. The threshold time may correspond or define an amount of time in which the user 210 is expected to make a selection 205' with one of the UI elements 135 and can range from 5 seconds to 10 minutes. With the determination, the performance evaluator 150 can modify or adjust the performance metric 415 using the response time compared to the threshold time.

In some embodiments, the performance evaluator 150 can calculate, generate, or otherwise determine the performance metric 415 related to an increase in cognitive functioning or a decrease in the functional impairment (e.g., social processing or non-social processing) for the user 210. The performance evaluator 150 can determine the performance metric 415 based on delay times between the presentation of the image 180' and the receipt of the response 425. For example, the delay time between subsequent presentations of the images 180' (or the audio recordings 185') and the receipt of the selection 205' may decrease. This decrease in delay time can indicate an increase in cognitive functioning or a decrease in the functional impairment for the user 210.

The session manager 140 may modify the session 420 (e.g., including the presentation of the images 180', the audio recordings 185', and the prompts 430) based on the performance metric 415. In some embodiments, the performance evaluator 150 may generate the performance metric 415 during a first time period based on the response 425. The session manager 140 may modify layer parameters 445A-N associated with the layer of the session 420. The layer parameters 445A-N (hereinafter generally referred to as the layer parameters 445 or the parameters 445) can define the presentation of the session 420. The parameters 445 can correspond to the presentation of the images 180', the audio recordings 185', the prompts 430, or the UI elements 135, among others.

The parameters 445 can include a type of modality for content. A type of modality for content can include the way in which the session 420 is presented, such as auditorily, visually, haptically, or textually. For example, the session manager 140 can modify the image 180' to include an auditory reading of the text depicted therein. The parameters 445 can include a context for social settings in images. The context for social settings in images can relate to information provided about the virtual setting 435 during the session, such as the location of the virtual setting 435 or the social interaction displayed in the image 180'. The parameters 445 can include a number of characters 440 in the virtual setting 435. For example, the session manager 140 can add or remove characters 440 to or from the virtual setting 435. The parameters 445 can include a type of prompt. A type of prompt can include a query, an action, or an instruction, among others.

In some embodiments, the session manager 140 can modify the prompt 430 to display alternate text or images, or to be associated with alternate UI elements 135. The session manager 140 can change the type of prompt from a query to an action or can rephrase a type of query. The parameters 445 can include a difficulty level for responses. The difficulty level can relate to how often a set of users selects the correct selection for a given prompt. For example, a first prompt eliciting more incorrect responses than a second prompt can be more difficult than the second prompt. The parameters 445 can include a type of response. The type of response can refer to the UI elements 135 selected by the user 210 or can refer to a classification of the response. For example, a first response may be classified as an "aggressive" response and a second response may be classified as a "calm" response, based on the selections of UI elements 135. The parameters 445 can include a number of responses. For example, the session manager 140 can modify the presentation of the prompts 430 to include more prompts based on a number of responses received by the session manager 140 thus far.

In some embodiments, the session manager 140 may modify the presentation of the audio recordings 185' in accordance with the set of parameters 445. The set of parameters 445 may include a set of controls or constraints to be applied to the presentation of the audio recordings 185'. In some embodiments, the session manager 140 may use the constraints defined by the set of parameters 445 when selecting new audio recordings 185' for subsequent sessions. The parameters 445 may include an inclusion of a distraction. For example, the distraction may include addition of noise (e.g., Gaussian noise) or inclusion of interrupting speech within the speech sample in the audio recordings 185'. The parameters 445 may include an ability to repeat the presentation of the audio recordings 185'. For instance, the constraint may limit the number of times that the user 210 is able to press (e.g., one of the UI elements 135) repeat to hear the audio recordings 185'. The parameter 445 may include a modification in speed. For example, the constraint may specify a playback speed of the audio in the audio recordings 185'. The parameters 445 may include a volume (or intensity) of the speech in the audio recordings 185'.

In addition, the parameter 445 may also identify a time between each word (or sentence) in the audio recordings 185'. For instance, the parameter 445 may define an amount of time between the presentation of one phrase followed by a subsequent phrase while presenting the audio recordings 185'. The parameter 445 may include a number of words in each sentence. For example, the constraint may specify a minimum or maximum number of words presented in each sentence of the audio recordings 185'. The parameter 445 may include a length of the audio recording 185'. The length may specify a minimum or maximum duration in time for the audio recording 185'. The parameter 445 may include an ability to control the distraction. For example, the user interface 130 may include a UI element 135 to include or exclude distractions in the presentation of the audio recording 185'.

The session manager 140 can provide the modified session for presentation at a second time instance. The session manager 140 may provide the modified session including modified images 180', the audio recordings 185', modified prompts 430, modified UI elements 135, or any combination thereof. In some embodiments, providing the session 420 at a second time instance can include displaying a third image 180C'. The third image 180C' can include a second virtual setting 435, a second prompt 430B, or a second set of UI elements 135B. The second prompt 430B can identify a second query for a second character depicted in the second virtual setting 435. For example, the session manager 140 can modify the prompt to display a second or different query for a second or different character 440 during the session 420 by modifying the layer parameters 445. The set of UI elements 135 can identify a corresponding set of responses related to the second character. In some embodiments, the session manager 140 may provide the modified session with prompts 430 to configure, set, or otherwise modify the parameters 445 applied to the presentation of the audio recordings 185'. For example, the session manager 140 may specify that the prompt 430 is to include options to reduce distractions, slow speech, or increase volume, among others.

In accordance with the parameters 445, the application 125 can display the UI elements 135 corresponding to a set of selections 205' related to the second character in the second virtual setting. The application 125 may present the audio recordings 185' with the one or more constraints applied as defined by the parameters 445. For instance, the application 125 may remove the UI element 135 corresponding to the replay button and increase the speed of the playback of the audio recordings 185'. By modifying the parameters 445 of the session 420, the session manager 140 can provide curated sessions for the user 210 based on the user's performance as indicated by the performance metric 415. This approach to cognitive remediation can reduce computational resources allotted to irrelevant sessions and can further improve adherence to the digital therapeutics regimen. In some embodiments, the application 125 may present the prompt 430 via the UI elements 135 to allow the user 130 to modify the parameters 445 applied to the presentation of the audio recordings 185'. For example, the user interface 130 for the prompt 430 may include UI elements 135 to reduce distractions, increase volume, decrease speed, and repeat at least a portion of the conversation, among others.

In addition, the feedback provider 155 may produce, output, or otherwise generate feedback 450 for the user 210 to receive via the application 125 operating on the user interface 130. The feedback provider 155 may generate the feedback 450 based on at least the performance metric 415, the response 425, the prompt 430 including a query, the user profile 165, or the historic session, image 180', the audio recording 185', or prompt presentation. The feedback 450 may include text, video, or audio to present to the user 210 via the application 125 displaying through the user interface 130. The feedback 450 may include a presentation of the performance metric 415. The feedback 450 may display a message, such as a motivating message, suggestions to improve performance, a congratulatory message, or a consoling message, among others. In some embodiments, the feedback provider 155 may generate the feedback 450 during the session being performed by the user 210. In some embodiments, the feedback provider 155 may generate the feedback 450 to provide text including an explanation of the correct response. The text of the explanation may be maintained and stored on the database 160 (e.g., using one or more files), and retrieved from the database 160 for the feedback 450.

The interaction handler 145 may provide, send, or otherwise transmit the feedback 450 to the application 125 for display on the user interface 130. The interaction handler 145 may provide instructions for rendering or display of the feedback 450. The interaction handler 145 may transmit data packets, signals, or other instructions to indicate the presentation of the feedback 450 to the application 125. Upon receipt, the application 125 can present the feedback 450 to the user 210 via the user interface 130. The presentation of the feedback 450 may include the UI elements 135. For example, the user 210 may make a selection related to the feedback 450, such as to increase the difficulty of the session or to resume the session 420. In some embodiments, the application 125 may present the feedback 450 to display the text of the explanation about the correct response.

With the determination of the performance metric 415 by the performance evaluator 150, the session manager 140 can determine to transition to a third session. The session manager 140 can determine to transition the user 210 from the virtual functional training layer associated with the session 420 to a functional training layer associated with another session. The session manager 140 can provide the third session responsive to the performance evaluator 150 determining that the performance metric 415 is at or above a threshold. The performance evaluator 150 can determine that the performance metric 415 is at or above a performance threshold for the session 420. Conversely, upon determining that the performance metric 415 is at or above the performance threshold for the session, the session manager 140 can provide another session. The performance evaluator 150 can determine that the performance metric 415 is below a performance threshold for the session 420. Upon determining that the performance metric 415 is below the performance threshold, the session manager 140 can maintain the session 420.

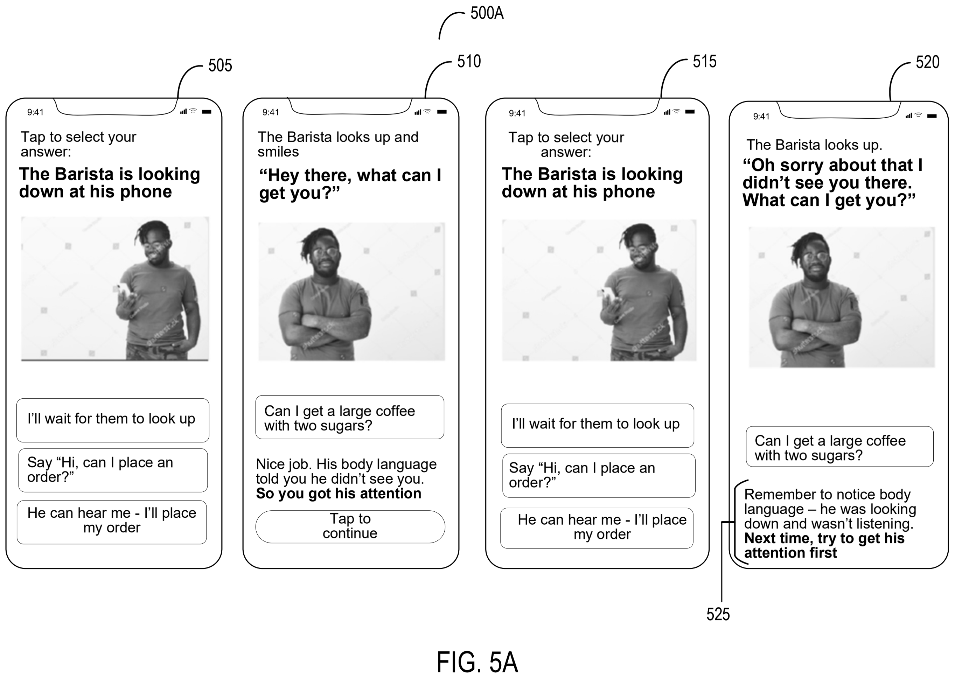

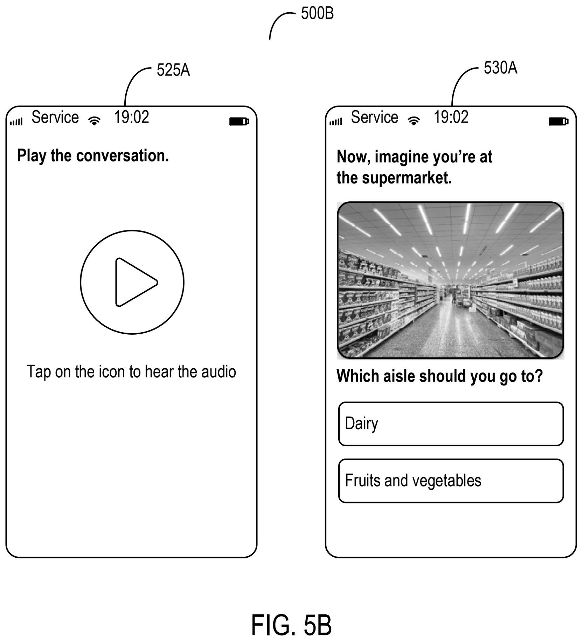

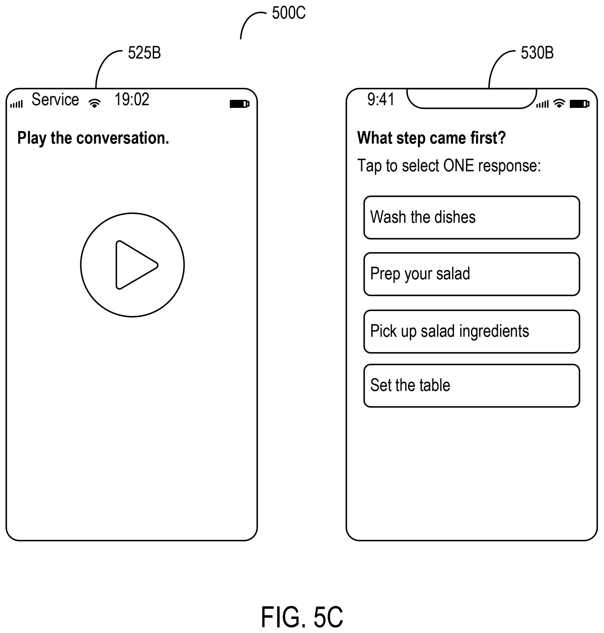

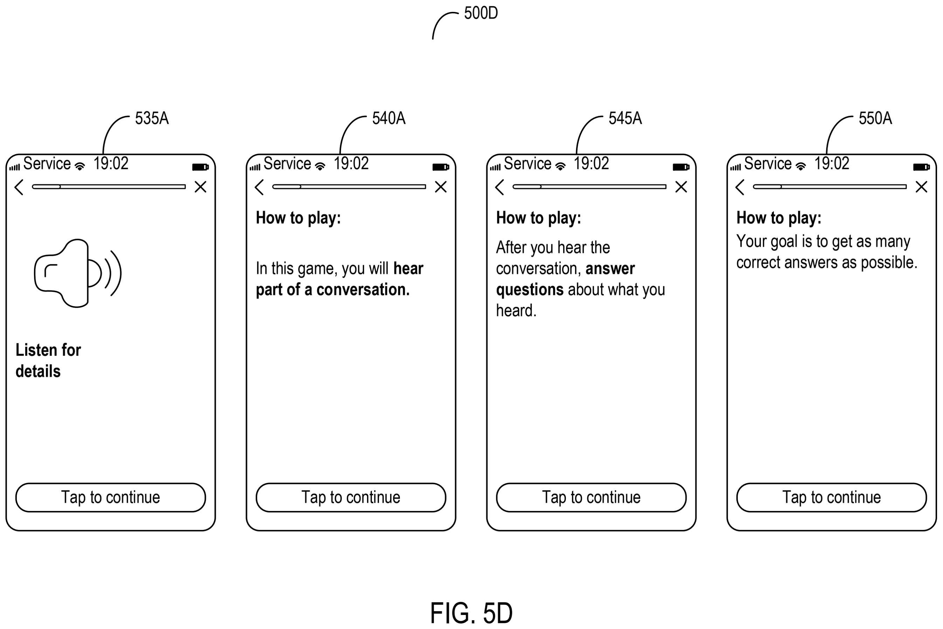

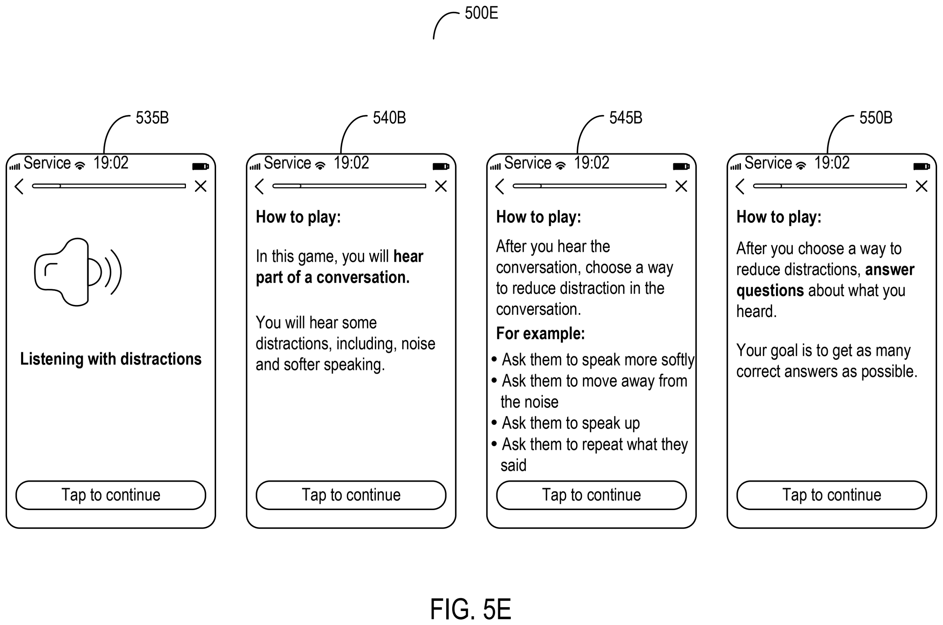

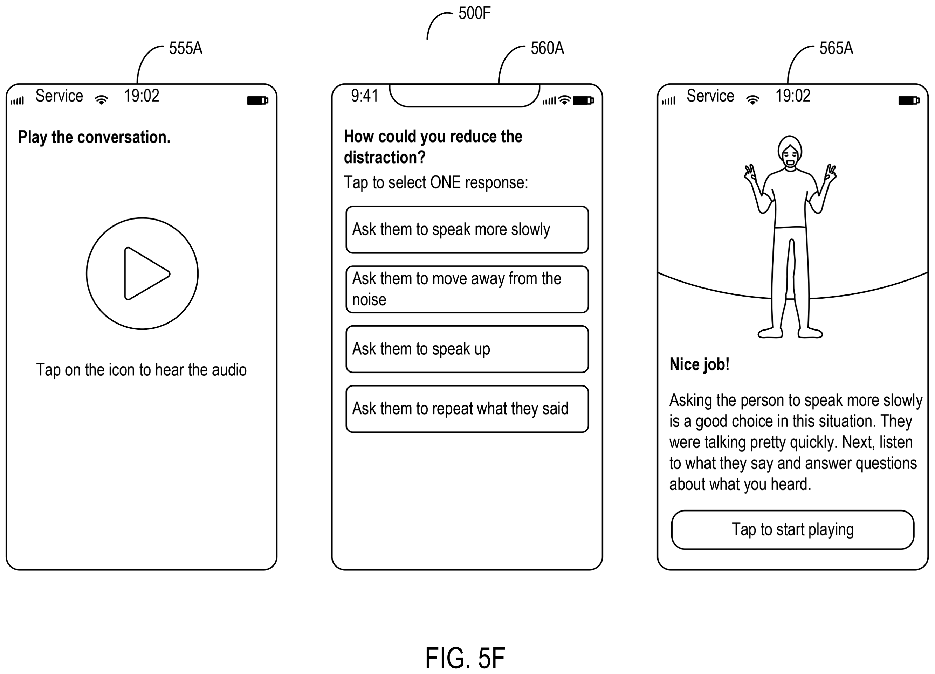

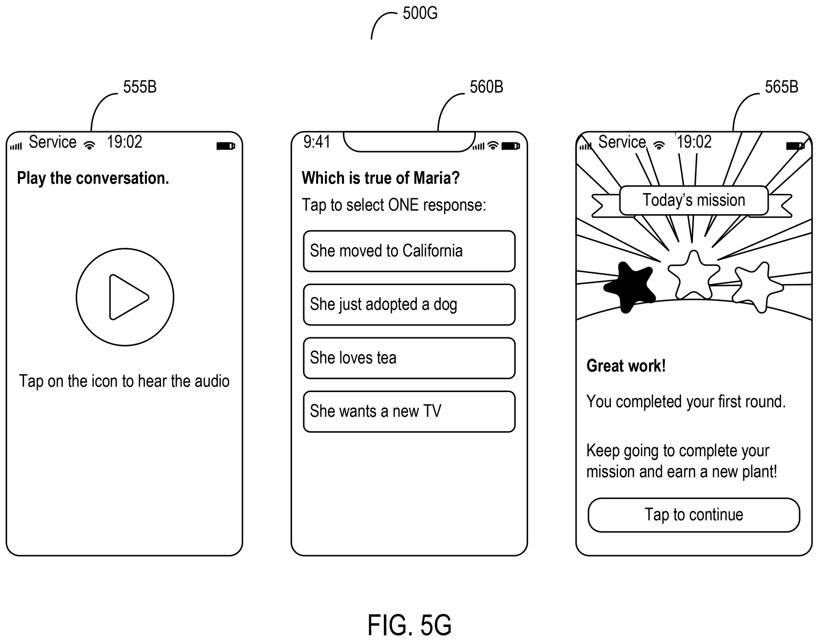

Referring now to FIGS. 5A-5G, depicted is a set 500A-G of example user interfaces for providing a second session. The set 500A can include user interfaces 505-520. In some embodiments, the set 500 can be a part of or included in the second session providing a virtual functional training layer. The user interfaces 505-520 can depict an example virtual setting, such as the virtual setting 435 depicted with reference to FIG. 4. The user interface 520 can include feedback 525 for user's selections in the previous interfaces. The feedback 525 can be presented by the service responsive to the receipt of a response by the user at the interface 515.

Through the set 500A of interfaces provided in the second session (e.g., the session 420), the user can be trained on social skills or non-social skills. Social skills can include performance of social interactions in a social situation, such as ordering a coffee at a shop, asking a stranger for directions, or trying to reach an object when another individual is in the way. Non-social skills can include time management and memory skills, such as verbal memory skills like remembering spoken words or associating an image with a spoken phrase. A social setting can include an observed interaction between one or more individuals, or an interaction the subject is participating in with one or more other individuals. For example, the subject may read or observe a social setting in which a customer is arguing with a cashier, or the subject may be involved in an interaction such as a transaction, conversation, exchange of objects, dining, or other social interaction with a variety of individuals such as a cashier, teller, neighbor, friend, coworker, or other individual.

A social setting can include settings such as parties, offices, public spaces, transit, or other environments in which the subject may observe or participate in social interactions. Social interactions and social settings do not necessitate speech, and can include written communications, non-verbal communications, or sign language, among others. Social interactions and social settings can include social cues, such as an individual's body language, tone of voice, volume of voice, eye contact, or facial expressions, among others. In some cases, an appropriate social interaction for a social setting can be determined by the subject through the social cues presented by a person in the social setting. The sessions provided by the service can facilitate training the subject to determine or choose their social interactions based on the social cues of the social situation. For example, as depicted in user interfaces 505-520, the subject may, upon successful completion of a regimen of sessions, determine to politely gain a cashier's attention if the body language, eye contact, and facial expression of the cashier indicate to the subject that the cashier is not busy.

In set 500B, the application 125 may display the user interface 525A with a play button to start playing a set of audio recordings 185' corresponding to a conversation associated with a social setting. The conversation may be about obtaining an item in a supermarket. Upon the button being pressed, the application 125 may play back the audio recordings 185'. Subsequent to playback, the application 125 may display the user interface 530A with an image 180' of the supermarket to prompt the user 210 about information conveyed in the conversation. In the depicted example, the application 125 may include a question about which aisle the user 210 should go to obtain the item referenced in the conversation.

In set 500C, the application 125 may display the user interface 525B with a play button to start playing a set of audio recordings 185' corresponding to a conversation associated with a social setting. The conversation may include directions to make a salad as well as obtaining ingredients for making the salad. Upon the button being pressed, the application 125 may play back the audio recordings 185'. Subsequent to playback, the application 125 may display the user interface 530B to prompt the user 210 about information conveyed in the conversation. In the depicted example, the application 125 may include a question about which step came up first in the directions to make a salad.

In set 500D, the application 125 may display the user interface 535A with a play button to start playing a set of audio recordings 185' corresponding to a conversation associated with a social setting. Subsequent to the user pressing the button "tap to continue," the application 125 may present the user interface 540A to prompt the user 210 to listen to the conversation. Afterwards, the application 125 may present the user interface 545A prompting the user to recall information about the conversation presented through the audio recordings 185'. Upon the user pressing to continue, the application 125 may display the user interface 550A to provide instructions to the user 210 to attempt to obtain as many correct answers as possible. The application 125 may proceed to play back the set of audio recordings 185'.

The set 500E may be similar to the user interfaces presented in set 500D, with the addition of parameters 445 applied to the presentation of audio recordings 185'. In set 500E, the application 125 may display the user interface 535B with a play button to start playing a set of audio recordings 185' corresponding to a conversation associated with a social setting. Subsequent to the user pressing the button "tap to continue," the application 125 may present the user interface 540B to prompt the user 210 to listen to the conversation, and notify the user 210 of potential distractions including noise and other speech. Afterwards, the application 125 may present the user interface 545B notifying the user about the ability to reduce distraction in the conversation. Upon the user pressing to continue, the application 125 may display the user interface 550A to provide instructions to the user 210 to attempt to obtain as many correct answers as possible. The application 125 may proceed to play back the set of audio recordings 185', with the additional distractions.

In set 500F, the application 125 may display the user interface 555A with a play button to start playing a set of audio recordings 185' corresponding to a conversation. Subsequent to the user pressing the button, the application 125 may play back the audio recordings 185', with one or more parameters 445 such as noise, low volume, or fast speech, among others. Subsequent to the presentation of the audio recordings 185', the application 125 may present the user interface 560A to prompt the user 210 regarding the parameters 445 to modify the presentation of the conversation in the set of audio recordings 185'. With the selection of one of the choices, the application 125 may present the user interface 565A to provide feedback 450 about the selection by the user 210.

In set 500G, the application 125 may display the user interface 555B with a play button to start playing a set of audio recordings 185' corresponding to a conversation. Subsequent to the user pressing the button, the application 125 may play back the audio recordings 185'. Subsequent to the presentation of the audio recordings 185', the application 125 may present the user interface 560A to prompt the user 210 regarding information conveyed in the conversation, and provide a list of choices regarding the information. With the selection of one of the choices, the application 125 may present the user interface 565B to provide feedback 450 about the selection by the user 210.

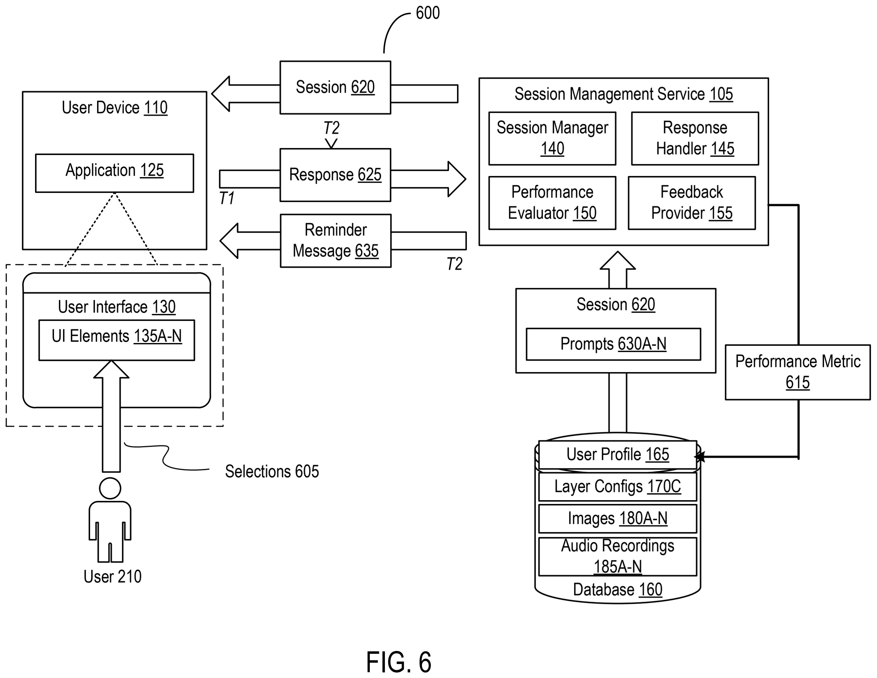

FIG. 6 depicts a block diagram for a process 600 to provide a third session for a functional training layer for the user to apply the social skill. The process 600 may include or correspond to operations performed in the system 100, the process 200, or the process 400. Under the process 600, the session manager 140 may provide a session 620 corresponding to a functional training layer. The interaction handler 145 can receive a response 625 indicating the performance of an action of the functional training layer. The performance evaluator 150 can determine a performance metric 615 based on the response 625. The feedback provider 155 can provide feedback to the user 210 based on the performance metric 615.

The session manager 140 can provide a session 620 to the application 125. The session 620 can be a third session 620 and can correspond to a third layer. The third layer can be a functional training layer. A functional training layer can be a therapeutic layer in which one or more prompts 630 are displayed to the user via the user interface 130 to direct the user to perform an activity. The activity can include activities in a real-world environment, different from the virtual setting 435 of FIG 4. The activity can include the user 210 engaging in a social setting such as a library, shop, or park. For example, the activity can include the user 210 purchasing a coffee in-person at a coffee shop, asking a librarian in-person for help locating a book in the library, or engaging in an in-person conversation on a bus. By partaking in activities in a real-world environment, the user 210 can combine the teachings of prior sessions to practice social skills in the real world in order to reduce the functional impairment. The functional training layer can also further non-social skills such as verbal memory in the previous layers, such as following recipe directions in a tutorial video on how to bake a cake or having a conversation and recalling the person's contact information.

The user interface 130 can display one or more prompts 630A-N directing the user 210 to perform one or more activities. Upon presentation of the session 620, the user 210 may select to perform the activity then or may select to delay the activity for later. In some embodiments, upon presentation of the prompts 630A-N, the user 210 may select a time, using the UI elements 135, to perform the activity. For example, the user 210 can make a selection 605 using the UI elements 135, to indicate a time at which to perform the activity. The response 625 can be transmitted at a first time T1. The response 625 may indicate a second time T2 at which the user selects to perform the activity. In some embodiments, the second time T2 may indicate a time at which the user 210 will perform the activity. In some embodiments, the second time T2 may indicate a time at which the session management service 105 may transmit a reminder message 635 to direct the user 210 to perform the activity.

The interaction handler 145 may transmit the reminder message 635 at the time T2 indicated by the user 210 in the response 625. The interaction handler 145 may transmit the reminder message 635 automatically upon reaching the time T2. The time T2 can be a countdown (e.g., delay for one hour, one day) or the time T2 can be a concrete time or date (e.g., 6PM on Friday). The interaction handler 145 may transmit the reminder message 635 including the prompt 630 for the activity to be performed. The reminder message 635 may display on the user interface 130. The user 210 may snooze (e.g., delay until a later time) the reminder message 635. In some cases, the reminder message 635 may be snoozed a threshold number of times. Upon exceeding the threshold number of snoozes, the user 210 may no longer be able to snooze the reminder message 635.

The user 210 may perform the activity indicated in the prompt 630 or the reminder message 635. Upon performance of the activity by the user 210, the user may choose one or more selections 605 to indicate the performance of the activity. In some cases, the session management service 105 or the application 125 may prompt for an indication of the performance of the activity, regardless of the user 210 indicating completion of the activity. The session management service 105 or the application 125 may prompt for an indication of the performance of the activity responsive to the elapse of a period of time, responsive to a selection 605 made by the user 210, or responsive to a schedule of prompts. Prompting for an indication of the performance of the activity can include providing the UI elements 135 on the user interface 130. In some embodiments, the user 210 may be provided with multiple possible selections of the UI elements 135 corresponding to the performance of the activity by the user 210. For example, the user interface 130 may display a first UI element 135A indicating that the activity went well, a second UI element 135B indicating that the activity went poorly, or a third UI element 135C indicating that the user 210 did not perform the activity. In some embodiments, the UI elements 135 may include a text box, in which the user 210 can dictate or type a response indicating the performance of the activity.

The performance of the activity can identify the state of the user 210 (e.g., emotional state, response, or difficulty of task) during the performance of the activity. For example, the performance of the activity can include whether or not the user performed the activity, the length of time the activity was performed, challenges encountered during the activity, general feelings or reflections on the activity, or a numerical rating of how the user 210 feels they did performing the activity, among others. The user 210 may indicate their performance in the activity through the selections 605 of the UI elements 135. The application 125 may detect the selections 605 and transmit the response 625 indicating the performance of the activity to the interaction handler 145.

The performance evaluator 150 may determine a performance metric 615 for the session 620 based on the response 625. The performance evaluator 150 may determine the performance metric 615 in the same or a similar manner as described in relation to the performance metric 215 of FIG. 2 or the performance metric 415 of FIG. 4. The performance evaluator 150 may determine the performance metric 615 based on the response 625 including an indication of the performance of the activity. The performance evaluator 150 may determine, based on the indication of the performance, the performance metric 615. In some embodiments, the user 210 may indicate that the activity went well. Upon the user 210 indicating that the activity went well, the performance evaluator 150 may determine a high performance metric 615. Conversely, upon the user 210 indicating that the activity did not go well, or that the user 210 did not perform the activity, the performance evaluator 150 may determine a low performance metric 615.

The session management service 105 may repeat the functionalities described above (e.g., processes 200, 400, and 600) over multiple sessions. The number of sessions may be over a set number of days, weeks, or even years, or may be without a definite end point. By iteratively providing different sessions corresponding to different layers based at least on the performance metrics 215, 415, and 615, the user profile 165, or the responses 225, 425, and 625, the user 210 may be able to receive training to improve functional impairments (e.g., social processing or non-social processing) associated with their condition. This may alleviate symptoms faced by the user 210, even when having a condition which could otherwise inhibit the user from seeking treatment or even physically accessing the user device 110. Furthermore, from participating in the sessions when presented through the user interface 130 of the application 125, the quality of human computer interactions (HCI) between the user 210 and the user device 110 may be improved.

Since the sessions build upon one another to provide a comprehensive, approachable regimen, the user 210 may be more likely to participate in the sessions when they are presented via the user device 110. This may reduce unnecessary consumption of computational resources (e.g., processing and memory) of the service and the user device 110 and lower the usage of the network bandwidth, relative to sending otherwise ineffectual or irrelevant sessions. Furthermore, in the context of a digital therapeutics application, the individualized selection of the sessions including the images and prompts may result in the delivery of user-specific interventions to improve subject's adherence to the treatment. This may not only result in higher adherence to the therapeutic interventions but also lead to potential improvements to the user's condition or cognitive or functional impairment.

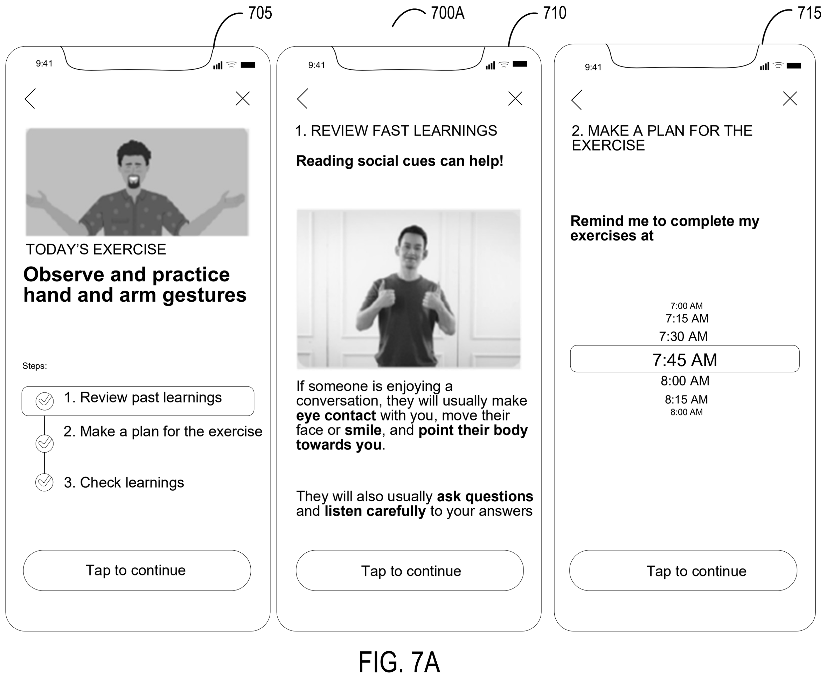

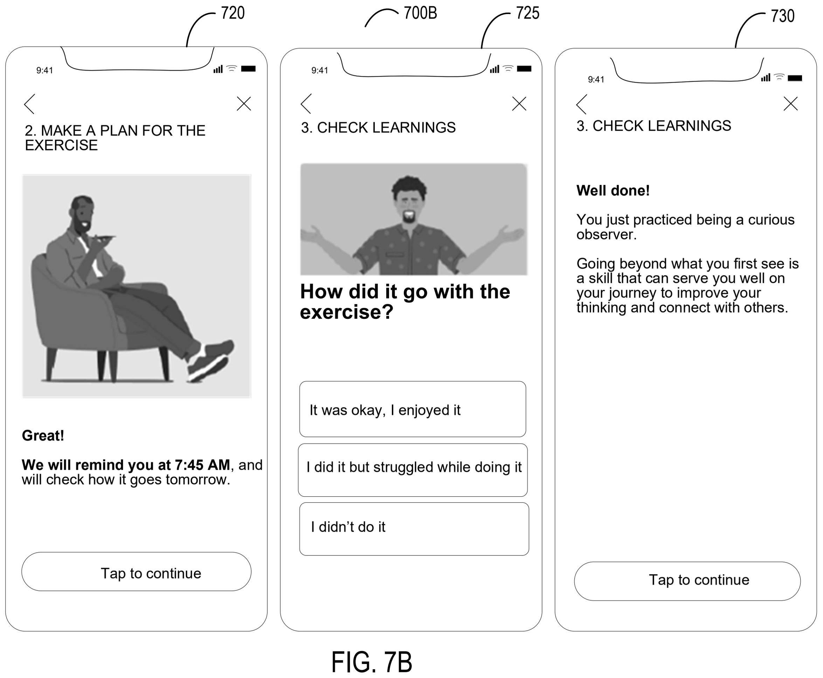

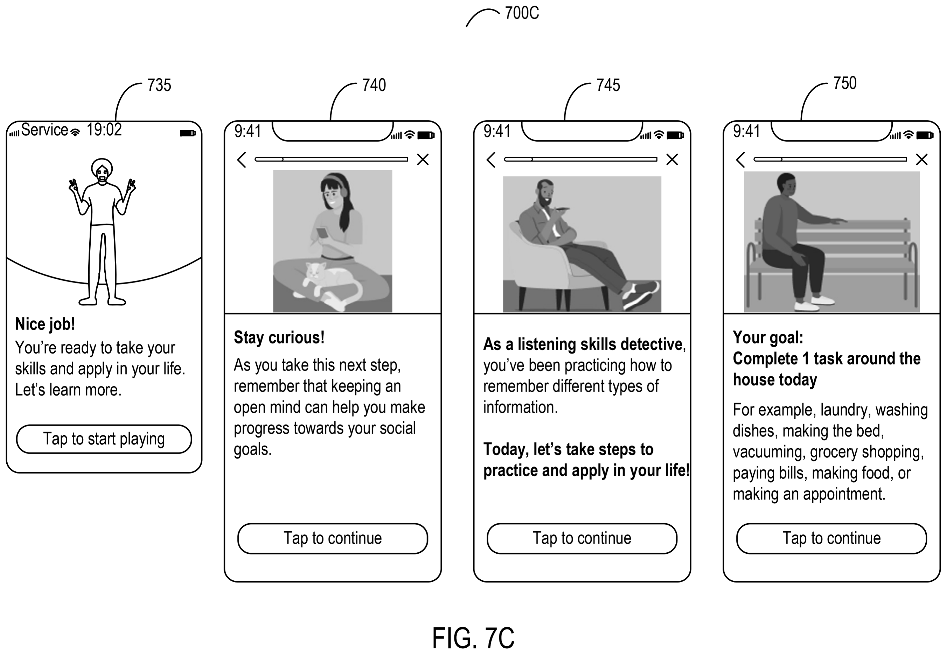

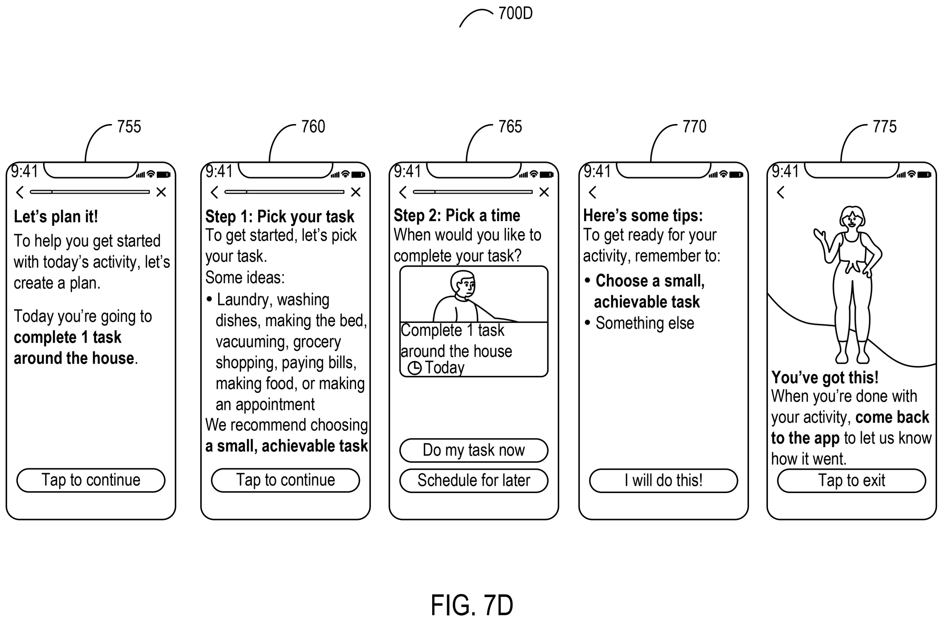

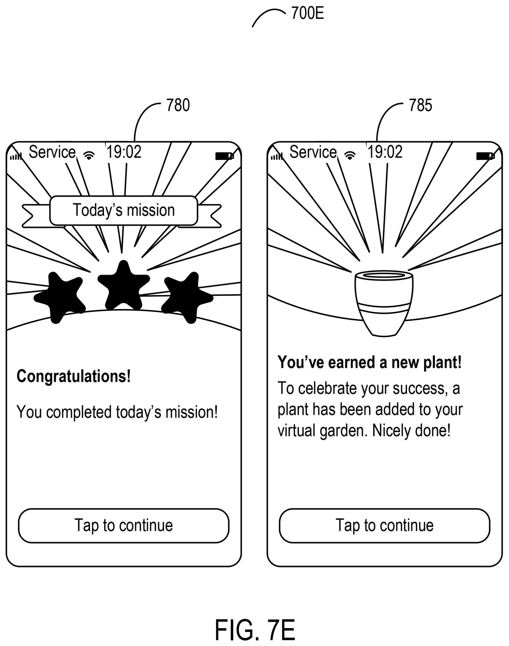

FIGS. 7A-7E depict screenshots of sets 700A-E of example user interfaces for providing a third session. In set 700A, the application 125 can present user interfaces 705-730. The set 700 can be a part of or included in the third session providing a functional training layer. The user interfaces 705-730 depict an activity for the user to perform, such as the activities described herein. The interface 705 can be a prompt directing the user to perform a particular activity (e.g., observing and practicing gestures). The second interface 710 can include information for the user to recognize social cues in the real-world setting. The third interface 715 can include a prompt to select a time at which to send reminders.

In set 700B, the application 125 can present user interfaces 720-730. The user interface 720 can allow the user 210 a time at which to send reminder messages. The user interface 725 can prompt the user 210 to indicate feedback regarding a particular activity performed in a surrounding of the user 210. The user interface 730 can provide feedback to the user 210 regarding the response in the user interface 725. In set 700C, the application 125 may present user interface 735 to indicate to the user 210 the start of a new activity to apply verbal memory-based skills in the surroundings of the user. When an interaction with the start button is detected, the application 125 may present user interface 740 to cue the users 210 regarding the activity. Continuing on, the application 125 may present the user interface 745 to provide instructions on a listening skills activity. The application 125 may present the user interface 750 to provide on a specific objective of the listening skills activity.

In set 700D, the application 125 may present the user interface 755 to direct the user 210 to perform a task in the surrounding area (e.g., the user's house). Upon the user pressing continue, the application 125 may present the user interface 760 to provide additional instructions regarding the task to be performed. The application 125 may also display the user interface 765 to allow the user 210 to select a time at which to perform the activity. Upon continuing, the application 125 may present the user interface 770 to provide tips regarding the performance of the activity. The application 125 may display the user interface 775 to notify the user to return to the application 125 to carry out the task and to record via the application 125. In set 700E, after the completion of the activity, the application 125 may present user interfaces 780 and 785 to notify the user 210 about the successful completion of the task.

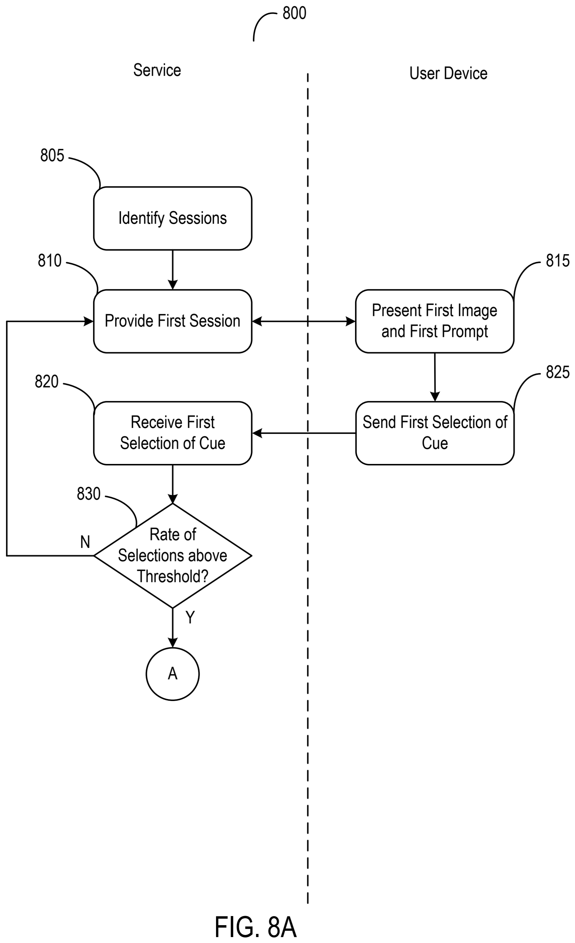

FIGS. 8A-8C depict a flow diagram of a method 800 for presenting interactive sessions to address functional impairment in users. The method 800 may be implemented or performed using any of the components detailed herein, such as the session management service 105 and the user device 110, or any combination thereof. Under the method 800, a service (e.g., the session management service 105) may identify a set of sessions (805). Each session may correspond to a respective layer and may include one or more images and prompts. The service may provide a first session of the set of sessions to the user device (e.g., the user device 110) (810). Providing the first session may include providing images and prompts associated with the first session. The user device may present a first image and a first prompt using a display device of the user device (815). Upon presentation of the first image and the first prompt, the application operating on the user device may detect and send a first selection related to a cue indicated in the first session (825). The cue can be or include the cues 235 indicated in FIG. 2. The service may receive the first selection of cue (820). Upon receipt of the first selection of cue, the service may determine whether the rate of selections is above a threshold (830). The rate of selections can refer to a rate of correct selections received by the service from the user device. If the rate of selections is below the threshold, the service may return to providing the first session (810). If the rate of selections is at or above the threshold, the service may provide a second session (835). The second session can include a virtual functional training layer. The user device may present a second image, second prompt, and interactive elements associated with the second session (840).

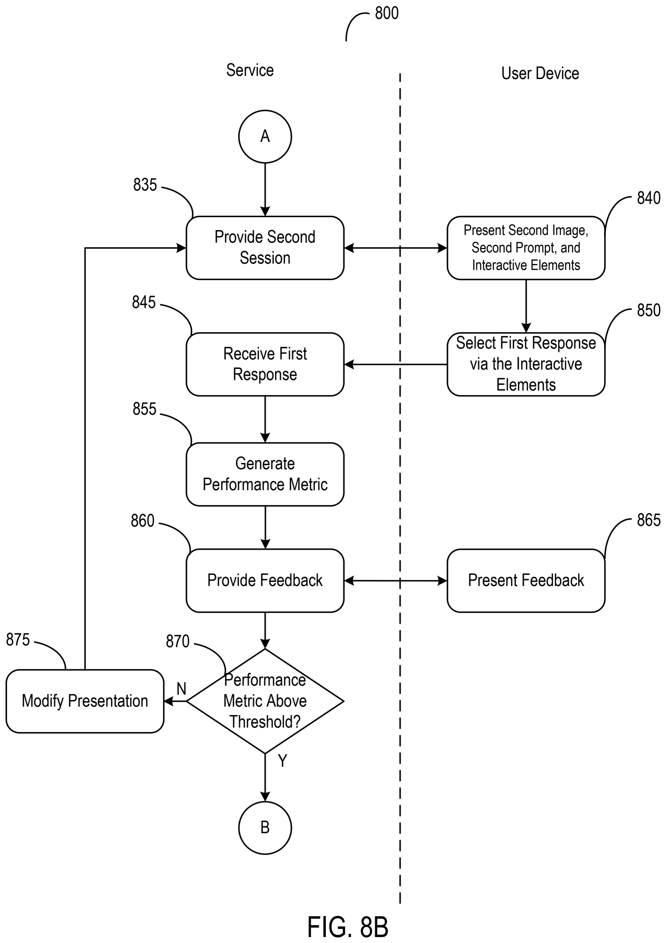

Continuing the method 800 in FIG. 8B, responsive to presenting the second image, the second prompt, and the interactive elements (840), the application operating on the user device may detect a selection of a response via the interactive elements (850). In some embodiments, the user 210 may select a first response corresponding to the second image or the second prompt using the interactive elements. In some embodiments, the application may detect selections of the interactive elements and may generate the response based on the selections for transmittal to the service. The service can receive the first response (845). The service can receive the first response indicating the selections associated with the second session. The service can generate a performance metric (855). The service may generate the performance metric based at least on the first response. The service may provide feedback (860). The service may provide feedback to the user device via the application. The feedback may be generated by the service based on at least one of the first response, the performance metric, the selections, the second images, or the second prompts. The user device may present the feedback (865). The user device may present the feedback through the application operating on the user device.

Upon providing the feedback, the service may determine whether the performance metric is above a threshold (870). If the performance metric is not above the threshold, the service may modify the presentation of the session (875). If the performance metric is at or above the threshold, the service may provide the third session (880). The third session may include or correspond to a functional training layer and may include one or more third prompts. The user device may present the third prompt (885).

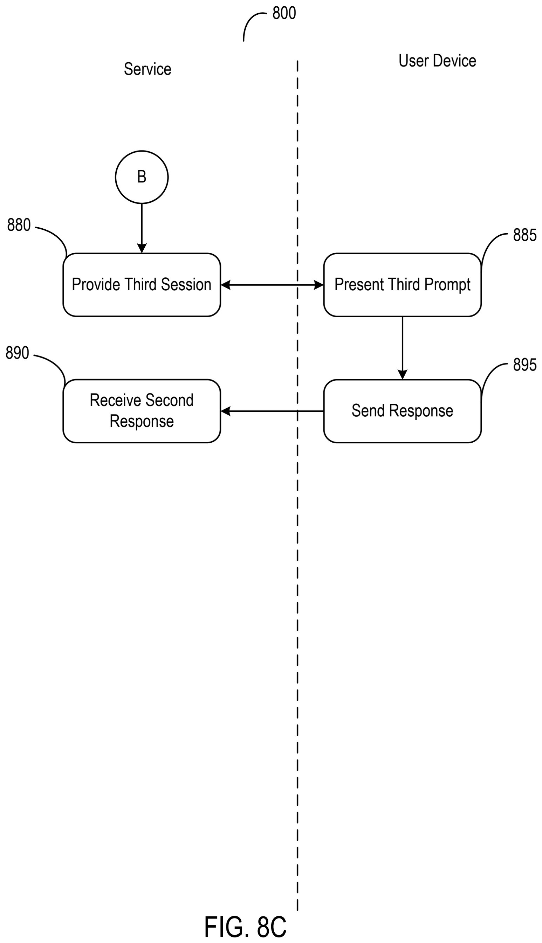

Continuing the method 800 in FIG. 8C, the user device may present the third prompt (885). Presenting the third prompt can include presenting a prompt to direct the user to perform an activity. The user device can send a response (895). The response can include an indication of the performance of the activity, or the response can include a time at which to remind the user to perform the activity. The service can receive the second response (890). Upon receiving the second response, the service can transmit a reminder message at a time indicated in the second response, store the response, or generate a performance metric for the user based on the response.

### B. Method of Ameliorating Functional Impairment in Users with Schizophrenia

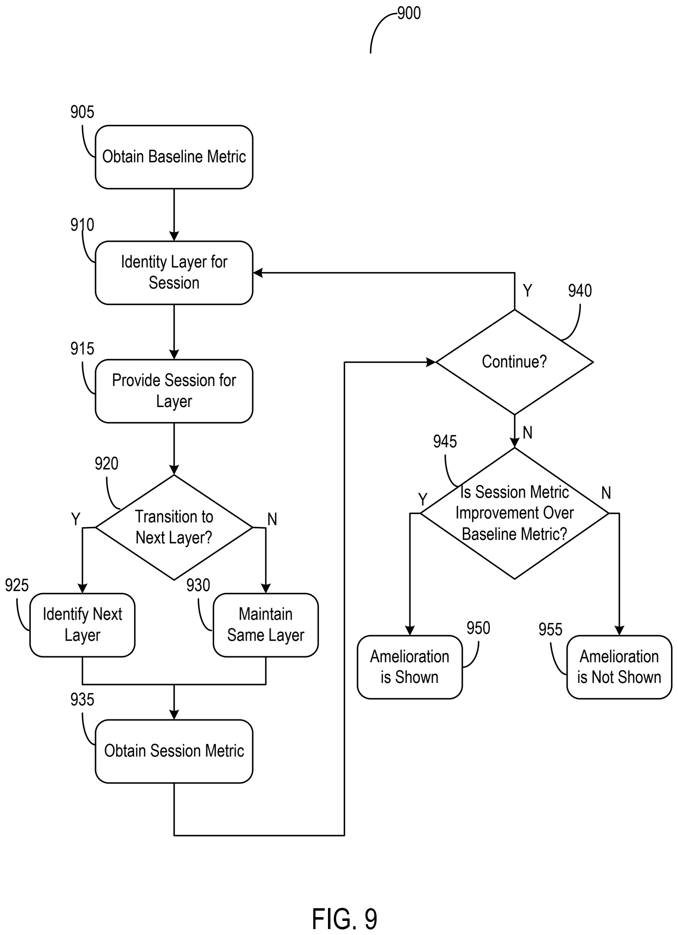

Referring now to FIG. 9, depicted is a flow diagram of a method 900 of ameliorating a functional impairment in a user with schizophrenia in need thereof. The method 900 may be performed by any components or actors described herein, such as the session management service 105, the user device 110, or the user 210, among others. The method 900 may be used in conjunction with any of the functionalities or actions described herein in Section A and the Examples in Section B. The method 900 may include a method of ameliorating a functional impairment in a user with schizophrenia in need thereof. The functional impairment may include social processing (e.g., social perception and emotional processing) or non-social processing (e.g., verbal memory, mentalizing, theory of mind, and problem solving), among others. In brief overview, the method 900 may include obtaining a baseline metric (905). The method 900 may include identifying a layer for a session (910). The method 900 may include providing the session for the layer (915). The method 900 may include determining whether to transition to a next layer (920). The method 900 may include identifying a next layer, when the determination is to transition (925). Otherwise, the method 900 may include maintaining the current layer, when the determination is to not transition (930). The method 900 may include obtaining a session metric (935). The method 900 may include determining whether to continue (940). The method 900 may include determining whether the session metric is an improvement over the baseline metric (945). The method 900 may include determining that amelioration is shown when the session metric is determined to be an improvement over the baseline metric (950). The method 900 may include determining that amelioration is not shown when the session metric is determined to be not an improvement over the baseline metric (955).

In further detail, the method 900 may include retrieving, identifying, otherwise obtaining a baseline metric (905). The baseline metric may be associated with a user (e.g., the user 210) with or diagnosed with schizophrenia. Schizophrenia in the user further may include positive symptoms including hallucinations or delusions, or negative symptoms including a decrease in motivation or emotional expressions. Schizophrenia in the user may lead to a functional impairment (e.g., social processing or non-social processing). For example, the functional impairment associated with the user may include one or more of reduced attainment in education, reduced quality of life, difficulty in living independently, reduced social functioning, or occupational dysfunction, among others.

The baseline metric may be obtained (e.g., by a computing system such as the session management service 105 or the user device 110 or both) prior to providing any of the sessions to the user via a digital therapeutics application (e.g., the application 125 or the Study App described herein). The baseline metric may indicate a degree of severity of the functional impairment due to schizophrenia in the user. The baseline measure may include, for example, a Multnomah Community Ability Scale (MCAS) value, a Lawton Instrumental Activities of Daily Living (Lawton IADL) value, a Personal and Social Performance (PSP) scale value, time use survey value, Patient Global Impression of Improvement (PGI-I) scale value, Clinical Global Impression of Improvement (CGI-I) scale values, or a World Health Organization Disability Assessment Schedule 2.0 (WHO-DAS 2.0) value, among others. Other metrics may be used to show other related effects (e.g., to show medicine adherence or as an exclusion criteria) such as a Clinician Rating Scale (CRS) value, a Medication Adherence Rating Scale (MARS) value, or Columbia-Suicide Severity Rating Scale (C-SSRS) value, among others. The scales used to measure the degree of severity of the functional impairment may differ based on whether social processing or non-social processing is addressed. For social processing (e.g., social perception), the metrics may include MCAS, Lawton IADL, PSP, or WHO-DAS 2.0 scale values, among others. For non-social processing (e.g., verbal memory), the metrics may include MCAS, CRS, CGI-I, or MARS-a, among others. Further explanation of the scales is provided below.

Multnomah Community Ability Scale (MCAS): The Multnomah Community Ability Scale is used to assess functioning of adults who have psychiatric disabilities and live in the community. Both instruments (Clinician and Self-Report versions) are based on standard methodology for rating scale development. For the purpose of this study, the clinician-rated version of the Multnomah Community Ability Scale will be used and referred to moving forward. The expanded version that includes behavioral anchors and additional interview probes that have been normed in people with schizophrenia and other serious mental illnesses will be used. Trained non-clinicians can also serve as raters and have been shown to have high reliability. The Multnomah Community Ability Scale measures the degree of functional ability in the past month through seventeen indicators. These indicators are rated on a 5-point scale that ranges from 5 (no impairment) to 1 (extreme impairment). The maximum score, indicative of a high functioning level, is 85. It takes an average of 20 minutes to complete the trainer-rated Multnomah Community Ability Scale.

Lawton Instrumental Activities of Daily Living: The Lawton Instrumental Activities of Daily Living (Lawton IADL) Scale assesses a person's independent living skills in eight categories: using a telephone, shopping, cooking, housekeeping, laundry, using transportation, taking their medications as prescribed, and financial management. Each category has a list of tasks that the person is rated on as 0 (low function, dependent) or 1 (high function, independent). A total score is obtained with a range of 0 (low function, dependent) to 8 (high function, independent) for women and 0 (low function, dependent) to 5 (high function, independent) for men.

Personal and Social Performance Scale (PSP): The PSP is a validated clinician-rated scale that measures personal and social functioning in four domains: socially useful activities (e.g., work and study), personal and social relationships, self-care, and disturbing and aggressive behaviors. Each area is scored on a 0-100 scale, with anchors for every 10-point interval. An overall score is obtained within the 10-point scale.

World Health Organization Disability Assessment Scale (WHO-DAS 2.0): The WHODAS 2.0 is a 36-item self-assessment scale to measure a participant's function and disability across six domains of life: cognition (understanding and communicating), mobility (moving and getting around), self-care (hygiene, dressing, eating, staying alone), getting along (interacting with others), life activities (domestic responsibilities, leisure, work and school), and participation (community and society).

Clinician Rating Scale (CRS): The CRS is a 1-item scale where the clinician assesses the level of medication or treatment adherence observed by the patient. The item is rated on a scale of 1-7 where 1 indicates complete refusal to adhere to medication or treatment and 7 indicates active participation in their treatment.

Medication Adherence Rating Scale (MARS): The MARS is a 10-item scale where the patient is asked about their adherence to psychiatric medications. For each item, patients are presented with a question related to their behaviors or attitudes towards their medication in the past week. Patients are asked to answer "yes" or "no" to each item. The sum of the items yields a final score ranking from 0 (poor adherence to psychiatric medications) to 10 (good adherence to psychiatric medications).

Time Use Survey: The Time Use Survey is a semi-structured interview to assess how the patient spends their time over the past month. Patients are asked about work, education, voluntary work, leisure, sports, hobbies, socializing, resting, housework/chores, childcare, and sleep. Time spent on each activity is calculated in terms of the number of hours per week allocated to that activity over the past month. The interview takes about 30-45 minutes to complete.

Columbia-Suicide Severity Rating Scale (C-SSRS): The Columbia-Suicide Severity Rating Scale (C-SSRS) is an assessment tool that evaluates suicidal ideation and behavior. The scale has been successfully implemented across many settings, including schools, college campuses, military, fire departments, the justice system, primary care and for scientific research. This scale is intended to be used by individuals who have received training in its administration. The questions contained in the Columbia-Suicide Severity Rating Scale are suggested probes to determine risk of suicide. Ultimately, the determination of the presence of suicidal ideation or behaviors depends on the judgment of the individual administering the scale.

Patient Global Impression of Improvement (PGI-I): The PGI-I is a single-item patient-reported outcome measuring participative subjective improvement in experiential negative symptoms severity on a 7-point scale. Higher scores on the PGI-I indicate a subjective report of disease worsening over the course of treatment. Response choices include: 1 = Very much better, 2 = Much better, 3 = A little better, 4 = No change, 5 = A little worse, 6 = Much worse, and 7 = Very much worse.

Clinical Global Impression of Improvement (CGI-I): The CGI-I is a single-item standardized clinician-rated measure that evaluates how much of the patient's illness has improved or worsened in the past seven days after enrolling in the study or treatment. The measure uses a 7-point Likert scale where a higher score indicates worsening of the illness. Response choices include: 1 = Very much improved, 2 = Much improved, 3 = Minimally improved, 4 = No change, 5 = Minimally worse, 6 = Much worse, and 7 = Very much worse.

Clinical Global Impression of Severity (CGI-S): The CGI-S is a single-item standardized, clinician-rated global rating scale that measures experiential negative symptom severity in the past seven days using a 7-point Likert scale. A higher score on the CGI-S represents a higher severity of disease. Response choices include: 0 = not assessed; 1 = normal, not at all ill; 2 = borderline mentally ill; 3 = mildly ill; 4 = moderately ill; 5 = markedly ill; 6 = severely ill; and 7 = among the most extremely ill participants.

Medication Adherence Rating Scale (MARS-a): The MARS-a is a 10-item scale where the patient is asked about their adherence to psychiatric medications. For each item, patients are presented with a question related to their behaviors or attitudes toward their medication in the past week. Patients are asked to answer "yes" or "no" to each item. The sum of the items yields a final score ranking from 0 (poor adherence to psychiatric medications) to 10 (good adherence to psychiatric medications).

Benefit Assessment: Trial participants may receive direct benefit from the interactive, software-based intervention featuring cognitive training and messaging. CT-156 is an adaptation of cognitive remediation training supplemented with ecological generalization of cognitive training, which is well validated as a therapeutic option for treating functional impairment in people diagnosed with schizophrenia. It integrates multiple psychosocial therapeutic techniques that work together to treat functional impairment associated with schizophrenia, as described in the psychological model of symptomatology.

The user may be of any demographic or trait, such as by age (e.g., an adult (above age of 18) or late adolescent (between ages of 18-24)) or gender (e.g., male, female, or non-binary), among others. At least in partial concurrence with one or more of the sessions, the user may be receiving treatment for schizophrenia. The treatment may include a psychosocial intervention or a medication to address schizophrenia. The psychosocial intervention may include, for example, psychoeducation, group therapy, cognitive-behavioral therapy (CBT), or early intervention for first-episode psychosis (FEP), among others. The medication may include, for example, a typical antipsychotic (e.g., haloperidol, chlorpromazine, fluphenazine, perphenazine, loxitane, thioridazine, or trifluoperazine) or an atypical antipsychotic (e.g., aripiprazole, risperidone, clozapine, quetiapine, olanzapine, ziprasidone, lurasidone, paliperidone, or iclepertin), among others.

The method 900 may include selecting, determining, or otherwise identifying at least one of a set of layers for one or more sessions (e.g., the session 220, 420, or 620) for the user (910). A computing system (e.g., the session management service 105 or the user device 110, or both) may execute a digital therapeutic application (e.g., the application 125) to provide an enhanced life skills by cognitive intervention (ELSCI). The computing system can identify the layer for the session based on a schedule or a user profile (e.g., the user profile 165). The layers may include the first layer for cognitive training, the second layer for virtual functional training, and the third layer for ecological functional training, among others. The schedule or the profile may identify which layer the user is currently tasked with. The user may first start with the first layer, and then progress onto the second and third layers upon completion of the prior layers. From the schedule or profile, the computing system may identify the layer for the user.

The method 900 may include providing or presenting the session for the identified layer (915). The computing system may provide an instruction for the session in accordance with the identified layer. Each session may correspond to the identified layer in which the user is to be trained with a particular social or non-social skill aimed at addressing the functional impairment due to schizophrenia. The instruction may define images (e.g., the images 180) and prompts according to a configuration for the layer (e.g., the layer configuration 170). Upon receipt, the application may present the images and the prompts via one or more elements of a user interface (e.g., the user interface 130).

For the session of the first layer, the computing system may display one or more images (e.g., the images 180) with which the user is to recognize one or more of a plurality of social cues associated with a social skill. In some embodiments, the computing system may display an image of a social cue (e.g., cue 235) with a prompt (e.g., the prompt 230) including a set of interactive elements identifying the corresponding plurality of types of cues for the image. The computing system may receive a selection of one of the plurality of types of social cues by the user via at least one of the sets of interactive elements. The social cues may include a head movement (e.g., nodding, shaking, tilting, or other head gestures), a body language (e.g., facial expressions, overall body posture, and proximity with respect to another character), a gesticulation (e.g., pointing using a finger or expressing emotion through hands or fingers), or an eye contact (e.g., orientation of eye with respect to another person to show engagement or emotion), among others.

In some embodiments, for the session of the first layer associated with a non-social skill such as verbal memory, the computing system may present one or more first audio recordings (e.g., the audio recordings 185) with which the user is to recall one or more of a plurality of words. The computing system may identify a set of audio recordings to present to the user. Each recording may correspond to one or more words. With the identification of the set of audio recordings, the computing system may present the set of audio recordings in accordance with a format. The format may define a context in which the words of the audio recordings are presented. The computing system may display a user interface to prompt the user to select at least one of the words as having been presented in the audio recordings. The computing system may receive a selection of the word from the user.

For the session of the second layer, the computing system may display an image (e.g., the image 180') of a social setting (e.g., the virtual setting 435). The image may be presented with a prompt (e.g., the prompt 430) identifying a query associated with a character (e.g., the character 440) displaying one of the pluralities of social cues and a set of interactive elements identifying a corresponding plurality of responses to the character. In some embodiments, the computing system may display a set of images of the setting with the characters in accordance with a defined sequence. The computing system may receive a response (e.g., the response 425) from the plurality of responses selected by the user via at least one of the second set of interactive elements. The computing system may provide feedback (e.g., the feedback 450) to the user based on the query for the setting and the response.

In some embodiments, the computing system may present the second layer in accordance with a set of parameters (e.g., the parameters 445). The parameters may include, for example, a type of modality for content, a context for settings in images, a number of characters in settings, a type of prompt, a difficulty level for responses, a type of response, or a number of responses, among others. In some embodiments, the computing system may modify the provision of the session for the second layer at one instance using a response from the user in the session for the second layer in a prior instance. For instance, the computing system may display an image of a social setting with a prompt identifying a query of a character in the setting and a set of interactive elements identifying a corresponding plurality of responses to the second character in accordance with a plurality of parameters.

In some embodiments, for the session of the second layer to apply the verbal memory skill in the virtual social setting, the computing system may present an audio recording (e.g., the audio recording 185') of a speech sample. The speech sample may be from at least one speaker, and may correspond to one or more sentences by the speaker, such as a statement, question, exclamation, request, command, or suggestion, among others. In some embodiments, the computing system may modify the presentation or selection of the audio recordings in the second layer in accordance with a set of parameters. The set of parameters may include an inclusion of a distraction; an ability to repeat the presentation; modification in speed; volume of speech in the audio recording; time between each word or sentence; number of words in each sentence; a length of the audio recording; and an ability to control inclusion or exclusion of distractions, among others. In addition, the computing system may present a prompt identifying a query associated with the speech sample and a set of interactive elements identifying a set of responses. The query may ask the user to recall information about at least a portion of the speech sample. The computing system may receive a response selected by the user from the set of responses via at least one of the user interface elements. The computing system may generate and provide feedback to the user based on the query and the response.

For the session of the third layer, the computing system may display a prompt to direct the user to perform an activity in a surrounding or environment of the user. The computing system may subsequently receive a response (e.g., the response 625) associated with performance of the activity. In addition, the computing system may present a prompt for the user to select a time at which to provide a message (e.g., the reminder message 635) prompting the user to perform the activity.

The method 900 may include determining whether to transition to next layer (920). The computing system may generate, calculate, or otherwise determine a performance metric (e.g., the performance metric 215 or 415) for the user in the current session. With the determination, the computing system may compare the performance metric with a threshold. The threshold may delineate or identify a value for the performance metric at which to transition the user to the next layer (e.g., from the first layer to the second layer or from the second layer to the third layer). The method 900 may include identifying a next layer, when the determination is to transition (925). When the performance metric satisfies (e.g., is greater than or equal to) the threshold, the computing system may determine to transition the user to the next layer. Otherwise, the method 900 may include maintaining the current layer, when the determination is to not transition (930). When the performance metric does not satisfy (e.g., is less than) the threshold, the computing system may determine to not transition the user to the next layer and maintain the user in the current layer.

The method 900 may include retrieving, identifying, otherwise obtaining a session metric (935). The session metric may be obtained (e.g., by the computing system) subsequent to providing at least one session to the user via the digital therapeutics application. The session measure may indicate a degree of severity of the functional impairment due to schizophrenia in the user, after being provided at least one session in one or more of the layers. The session measure may include, for example, a Multnomah Community Ability Scale (MCAS) value, a Lawton Instrumental Activities of Daily Living (Lawton IADL) value, a Personal and Social Performance (PSP) scale value, time use survey value, Patient Global Impression of Improvement (PGI-I) scale value, Clinical Global Impression of Improvement (CGI-I) scale values, or a World Health Organization Disability Assessment Schedule 2.0 (WHO-DAS 2.0) value, among others. Other metrics may be used (e.g., to show medicine adherence or as an exclusion criteria) such as a Clinician Rating Scale (CRS) value, a Medication Adherence Rating Scale (MARS) value, or Columbia-Suicide Severity Rating Scale (C-SSRS) value, among others. The session metric may be of the same type of metric or scale as the baseline metric.

The method 900 may include identifying or determining whether to continue (940). The determination may be based on the set length (e.g., days, weeks, or years) of the trial, a set number of time instances during which to perform one or more sessions, or a set number of sessions to be provided to the user. For example, the set number of time instances may range between 2 to 30 weeks, relative to obtaining the baseline metric or the start of the initial session by the user. When the amount of time from obtaining the baseline metric exceeds the set length, the determination may be to stop providing additional tasks. In contrast, when an amount of time has not exceeded the set length, the determination may be to continue providing additional tasks and repeat from (910).

The method 900 may include identifying or determining whether the session metric is an improvement over the baseline metric (945). The improvement may correspond to an amelioration in the degree of severity of the functional impairment due to schizophrenia in the user. The functional impairment may include social processing (e.g., social perception) or non-social processing (e.g., verbal memory). The improvement may be shown when the session metric is increased compared to the baseline metric by a first predetermined margin or when the session metric is decreased compared to the baseline metric by a second predetermined margin. The margin may identify or define a difference in value between the baseline and session metrics at which to determine that the user shows improvement in the degree of severity of the functional impairment due to schizophrenia. Whether the improvement is shown by increase or decrease may depend on the type of metric used to measure the user with respect to the degree of severity of the functional impairment due to schizophrenia. The margin may also depend on the type of metric used and may in general correspond to the difference in value showing noticeable difference by the clinician or user with respect to the degree of severity of the functional impairment due to schizophrenia, or showing a statistically significant result in the difference in the values between the baseline and session metrics.

The method 900 may include determining that amelioration is shown when the session metric is determined to be an improvement over the baseline metric (950). In some embodiments, the amelioration may be determined (e.g., by the computing system or a clinician examining the user) to occur when the session MCAS metric is decreased from the baseline MCAS metric by the first predetermined margin. In some embodiments, the amelioration may be determined to occur when the session Lawton IADL metric is increased from the baseline Lawton IADL metric by the second predetermined margin. In some embodiments, the amelioration may be determined to occur when the session PSP metric is increased from the baseline PSP metric by the second predetermined margin.

Continuing on, in some embodiments, the amelioration may be determined to occur when the session WHO-DAS 2.0 metric is decreased from the baseline WHO-DAS 2.0 metric by the first predetermined margin. In some embodiments, improvement to medication adherence may be determined to occur when the session CRS metric is increased from the baseline CRS metric by the second predetermined margin. In some embodiments, the improvement to medication adherence may be determined to occur when the session MARS metric is increased from the baseline MARS metric by the second predetermined margin.

In some embodiments, the amelioration in the functional impairment associated the user with schizophrenia may be determined to occur, when the session MCAS metric is decreased from the baseline MCAS metric by the first predetermined margin. In some embodiments, the amelioration in the functional impairment associated with the user with schizophrenia may be determined to occur, when the session CRS metric is increased from the baseline CRS metric by the second predetermined margin. In some embodiments, the amelioration in the functional impairment associated with the user with schizophrenia may be determined to occur, when the session PGI-I metric is decreased from the baseline PGI-I metric by the first predetermined margin. In some embodiments, the amelioration in the functional impairment associated with the user with schizophrenia may be determined to occur, when the session MARS-a metric is increased from the baseline MARS-a metric by the second predetermined margin.

The method 900 may include determining that amelioration is not shown when the session metric is determined to be not an improvement over the baseline metric (955). In some embodiments, the amelioration may be determined (e.g., by the computing system or a clinician examining the user) to not occur when the session MCAS metric is not decreased from the baseline MCAS metric by the first predetermined margin. In some embodiments, the amelioration may be determined to not occur when the session Lawton IADL metric is increased from the baseline Lawton IADL metric by the second predetermined margin. In some embodiments, the amelioration may be determined to not occur when the session PSP metric is not increased from the baseline PSP metric by the second predetermined margin.

Continuing on, in some embodiments, the amelioration may be determined to not occur when the session WHO-DAS 2.0 metric is not decreased from the baseline WHO-DAS 2.0 metric by the first predetermined margin. In some embodiments, the amelioration may be determined to not occur when the session CRS metric is not increased from the baseline CRS metric by the second predetermined margin. In some embodiments, the amelioration may be determined to not occur when the session MARS metric is not increased from the baseline MARS metric by the second predetermined margin. In some embodiments, the amelioration may be determined to not occur when the session time use survey metric is not increased from the baseline time use survey metric by the second predetermined margin. In some embodiments, the amelioration may be determined to not occur when the session C-SSRS metric is not decreased from the baseline C-SSRS metric by the first predetermined margin.

In some embodiments, the amelioration in the functional impairment associated with the user with schizophrenia may be determined to not occur, when the session MCAS metric is not decreased from the baseline MCAS metric by the first predetermined margin. In some embodiments, the amelioration in the functional impairment associated with the user with schizophrenia may be determined to not occur, when the session CRS metric is not increased from the baseline CRS metric by the second predetermined margin. In some embodiments, the amelioration in the functional impairment associated with the user with schizophrenia may be determined to not occur, when the session PGI-I metric is not decreased from the baseline PGI-I metric by the first predetermined margin. In some embodiments, the amelioration in the functional impairment associated with the user with schizophrenia may be determined to not occur, when the session MARS-a metric is not increased from the baseline MARS-a metric by the second predetermined margin.

### Example 1: Use of ELSCI in a Digital Therapeutics to Target Social Perception

In one example, the CT-156 mobile app (e.g., the application 125) was given to individuals who have schizophrenia per International Classification of Diseases 11th Revision (ICD-11) or Diagnostic and Statistical Manual of Mental Disorders, Fifth Edition (DSM-5), are experiencing mild-to-moderate functional impairment as evidenced by WHO-DAS 2.0, and are prescribed antipsychotic medication(s). The treatment was 2-30 weeks long. It was expected that users would show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

This study was a multi-center, exploratory, double-arm study to evaluate the overall effects of an abbreviated version of CT-156 as a treatment for mild-to-moderate functional impairment in participants 18 years of age and older diagnosed with schizophrenia. Eligible participants must have had a diagnosis of schizophrenia per the International Classification of Diseases, Eleventh Edition (ICD-11) or Diagnostic and Statistical Manual of Mental Disorders, Fifth Edition (DSM-5) and have been experiencing mild-to-moderate functional impairment based on the WHO-DAS 2.0.

Participants who met eligibility criteria were enrolled in the study and were offered the option to opt in to one of the study arms (CT-156 or CT-156+UXR) at the Screening Visit. Participants in either arm of the study used the same Study App. The difference in study arms was the qualitative interviews and surveys.

Screening Period: All participants entered an up to 7-day screening period to determine eligibility following the informed consent process. Participants who met all applicable inclusion criteria and no exclusion criteria at the time of the Screening Visit were introduced to the digital mobile application by downloading and installing the application onto their personal iPhone or Android smartphone device. Approximately 50 eligible participants were enrolled across approximately 12 study centers in the US during an in-person clinic visit on Day 1. A subset of participants (up to 15 participants) were offered the opportunity to opt in to the UXR arm, which consisted of the same treatment with additional user research interviews and surveys. Approximately 35 participants were enrolled in the CT-156 arm.

Baseline Visit: During the Baseline Visit on Day 1, participant eligibility was confirmed. Participants were considered eligible to activate the Study App if they continued to meet all inclusion and no exclusion criteria.

Intervention Period: Assessments and activities during this period were performed during in-person clinic visits or remotely by telephone visits.

Follow-up Period: Participants entered an up to 1-week follow-up period in which they attended a visit to complete follow-up assessments. Participants did not perform any activities within the application.

Inclusion Criteria: A participant was eligible for entry into the study if all of the following criteria were met:
1. Willing and able to provide written informed consent to participate in the study, attend study visits, and comply with study-related requirements and assessments.
2. Is at least 18 years of age at the time of informed consent.
3. Fluent in written and spoken English, confirmed by ability to read and understand the informed consent form.
4. Lives in the United States.
5. Meets diagnostic criteria for a primary diagnosis of schizophrenia as defined in the International Classification of Diseases, Eleventh Edition (ICD-11) or Diagnostic and Statistical Manual of Mental Disorders, Fifth Edition (DSM-5) for at least 6 months prior to screening.
6. Has outpatient treatment status at the time of screening, with no psychiatric inpatient hospitalization within 13 weeks (3 months) prior to screening.
7. Is currently prescribed at least one typical and/or atypical antipsychotic medication and has been on the same antipsychotic medication(s) for at least 13 weeks (3 months) prior to enrollment (Day 1). Dose adjustments are permitted during the study as outlined in the respective package insert(s).
8. Has an average domain score 2'2 in at least 2 domains of Understanding and Communicating, Getting Along with People, Life Activities - Household, or Participation in Society on the WHO-DAS 2.0.
9. Participant is the only user of an iPhone with iPhone operating system (iOS) version 15 or later or a smartphone with an Android operating system (OS) version 12 or later and agrees to download and use the digital mobile application as required by the protocol.
10. Is willing and able to receive short message service (SMS) text messages and push messages on their smartphone.
11. Is the owner of or has regular access to an email address.
12. Has regular access to the internet via cellular data plan and/or Wi-Fi.
13. Has stable housing and has remained at the same residence for at least 13 weeks (3 months) prior to screening and does not anticipate any housing changes for the duration of the study.
14. Understands the use of Study App during the Screening Period and at the Baseline Visit, per investigator judgment.

Exclusion Criteria: A participant was not eligible for study entry if any of the following criteria were met:
1. Has positive symptoms of schizophrenia that, in the opinion of the investigator, would preclude effective engagement in the treatment to improve functional impairment.
2. Is currently receiving or has received concomitant therapy, defined as individual or group-based structured treatment (e.g., Cognitive Behavioral Therapy, Social Skills Training, Motivational Interviewing, or Vocational/Occupational Therapy), within 3 months (13 weeks) prior to screening per investigator assessment.
3. Is currently treated with more than 2 antipsychotic medications (including more than 2 dosage forms).
4. Meets ICD-11 or DSM-5 criteria for diagnoses not under investigation that will impact compliance to the protocol, including schizophreniform, schizoaffective, or psychosis non-specific disorders (post-traumatic stress disorder [PTSD], bipolar disorder, major depressive disorder, developmental disorders).
5. Meets ICD-11 or DSM-5 criteria for a current episode of depression, mania or hypomania.
6. Meets ICD-11 or DSM-5 criteria for a current substance or alcohol use disorder (excluding caffeine and nicotine) that would interfere with compliance to the protocol, per investigator judgment. Diagnoses classified as in sustained remission are permitted.
7. In the investigator's opinion, currently needs or will likely require prohibited concomitant medications and/or therapy during the study.
8. Is at moderate to high risk for suicide, defined by any of the following:
   a. A "yes" response to either item 4 or 5 on the Columbia-Suicide Severity Rating Scale (C-SSRS) Suicidal Ideation Items within the last 13 weeks (3 months) prior to screening or at Baseline (Day 1)
   b. A "yes" response on the C-SSRS Suicidal Behavior Items within the last 26 weeks (6 months) prior to screening or at Baseline (Day 1)
   c. In the opinion of the investigator, presents a serious risk of suicide
9. Has participated in another clinical study (interventional or observational) in the last 13 weeks (3 months).
10. Has previously participated in any of the following studies: CT-155-C-001, CT-155-C-002, CT-155-C-003, CT-155-P-00x, CT-155-A-001, CT-155-R-001, CT-156-D-001, CT-156-C-001, CT-156-P-00x.

Primary Endpoints: The primary endpoint was the degree of participant engagement with the Study App using predefined engagement metrics.
- Number of days the Study App is opened out of total days of treatment
- Number of times the Study App is opened during the treatment period
- Number of medication check-ins completed during the treatment period out of total assigned
- Number of assigned tasks completed during the treatment period
- Average duration of each App-use session

### Exploratory Endpoints:

- Change from baseline to Week 8 in the MCAS - expanded version
- Change from screening to Week 8 in the WHO-DAS 2.0
- Change from baseline to Week 8 in the CRS
- Change from baseline to Week 8 in the MARS-a
- PGI-I at Week 8
- CGI-I at Week 8
- Change from baseline to Week 8 in the CGI-S
- Change in strength of digital working alliance from baseline to Week 8 as assessed by mARM
- Participant ratings of the Study App quality and satisfaction as measured by the MARS-b at the Week 8/ET Visit
- Participant feedback captured in user research qualitative interviews

### Schedule of Activities and Assessments:

| **Study Day and Visit Window** | **Screenin g Period** | **Intervention Period** | | | | | | | | | **Follo w-Up Perio d** |
|---|---|---|---|---|---|---|---|---|---|---|---|
| | **Screen ing** | **Baseli ne** | **We ek 1** | **We ek 2** | **We ek 3** | **We ek 4** | **We ek 5** | **We ek 6** | **We ek 7** | **Wee k 8/E T** | **Wee k 9** |
| | **Day -7 to Day -1^{a}** | **Day 1** | **Day 7 ± 3 day s** | **Day 14 ± 3 day s** | **Day 21 *±* 3 day s** | **Day 28 ± 3 day s** | **Day 35 ± 3 day s** | **Day 42 ± 3 day s** | **Day 49 ± 3 day s** | **Day 56 ± 3 days** | **Day 57 to Day 63^{b}** |
| **Study Visit Number** | **1** | **2** | **3** | **4** | **5** | **6** | **7** | **8** | **9** | **10** | **11** |
| **Clinic Visit** | X | X | | | | X | | | | X | |
| **Remote Visit** | | | X | X | X | | X | X | X | | X |
| Informed Consent | X | | | | | | | | | | |
| Medical History | X | | | | | | | | | | |
| Demographic s | X | | | | | | | | | | |
| Diagnostic history and interview (ICD-11 or DSM-5 diagnosis of schizophrenia ) | X | | | | | | | | | | |
| Inclusion/Exc lusion Criteria | X | X | | | | | | | | | |
| Study App Installation | X | | | | | | | | | | |
| Study App Activation | | X | | | | | | | | | |
| WHO-DAS 2.0 | X | | | | | | | | | X | |
| MCAS | | X | | | | X | | | | X | |
| Study App Engagement | X^{c} | X^{c} | | | | | | | | | |
| MARS-a | | X | | | | | | | | X | |

| **Study Day and Visit Window** | **Screen ing Period** | **Intervention Period** | | | | | | | | | **Follo w-Up Perio d** |
|---|---|---|---|---|---|---|---|---|---|---|---|
| | **Screen ing** | **Baseli ne** | **We ek 1** | **We ek 2** | **We ek 3** | **We ek 4** | **We ek 5** | **We ek 6** | **We ek 7** | **Wee k 8/E T** | **Wee k 9** |
| | **Day -7 to Day -1^{a}** | **Day 1** | **Day 7 ± 3 day s** | **Day 14 ± 3 day s** | **Day 21 ± 3 day s** | **Day 28 ± 3 day s** | **Day 35 ± 3 day s** | **Day 42 ± 3 day s** | **Day 49 ± 3 day s** | **Day 56 ± 3 days** | **Day 57 to Day 63^{b}** |
| **Study Visit Number** | **1** | **2** | **3** | **4** | **5** | **6** | **7** | **8** | **9** | **10** | **11** |
| CRS | | X | | | | | | | | X | |
| PGI-I | | | | | | | | | | X | |
| CGI-I | | | | | | | | | | X | |
| CGI-S | | X | | | | | | | | X | |
| mARM | | | X | | | X | | | | X | |
| MARS-b | | | | | | | | | | X | |
| Qualitative Interview^{f} | | | | X | | X | | X | | X | |
| Qualitative Survey^{d, f} | | | X | X | X | X | X | X | X | X | |
| C-SSRS | X | | | | | X | | | | X | |
| Study App Deactivation | | | | | | | | | | X^{e} | |
| Concomitant Medications | X | X | | | | X | | | | X | X |
| Adverse Events and/or Unanticipated Device Effects | X | X | | | | X | | | | X | X |

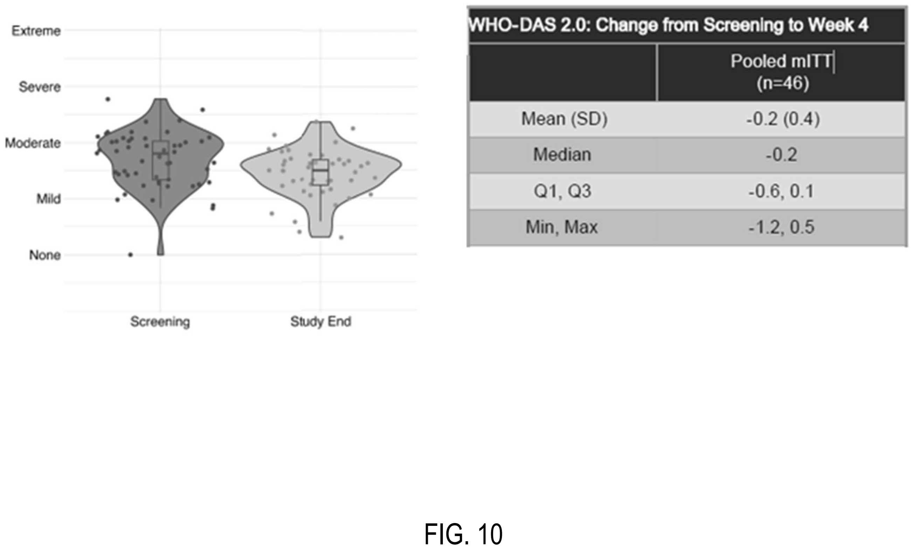

As shown in FIG. 10, a study was performed using the CT-156 mobile app in adults (18+ years) with a diagnosis of schizophrenia on antipsychotic medication with functional impairment. It was shown that self-reported disability (WHO DAS 2.0) improved significantly, and the primary efficacy endpoint (MCAS) moved in the right direction.

### Example 2: Use of ELSCI in a Digital Therapeutics to Target Problem Solving

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

### Example 3: Use of ELSCI in a Digital Therapeutics to Target Verbal Memory

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

### Example 4: Use of ELSCI in a Digital Therapeutics to Target Mentalizing/Theory of Mind

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

### Example 5: Use of ELSCI in a Digital Therapeutics to Target Emotion Processing

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

### Example 6: Use of ELSCI in a Digital Therapeutics to Target Social Perception and Verbal Memory

In one example, the CT-156 mobile app (e.g., the application 125) will be given to individuals who have schizophrenia per International Classification of Diseases 11th Revision (ICD-11) or Diagnostic and Statistical Manual of Mental Disorders, Fifth Edition (DSM-5), are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

### Example 7: Use of ELSCI in a Digital Therapeutics to Target Social Perception, Verbal Memory, Problem Solving, and Emotional Processing

In one example, the CT-156 mobile app (e.g., the application 125) will be given to individuals who have schizophrenia per International Classification of Diseases 11th Revision (ICD-11) or Diagnostic and Statistical Manual of Mental Disorders, Fifth Edition (DSM-5), are experiencing mild-to-moderate functional impairment as evidenced by WHO-DAS 2.0, and are prescribed antipsychotic medication(s).

In some aspects, the individuals are initially surveyed prior to entering treatment. Individuals may undergo a cognitive assessment as well as a values and goals assessment to determine what the patient's values and goals are. These Values and Goals determinations will be used after this through the app to maintain motivation and engagement. A determination will be made to determine the combination and order of interventions, including Social Perception, Verbal Memory, Problem Solving, and Emotional Processing. The individuals will be informed of the recommendation for the first intervention, and the individuals will have the option to accept the recommended first intervention or choose a different option.

The treatment will be 2-30 weeks long. The subjects will proceed through each of the four interventions provided above. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

### Example 8: Use of ELSCI in a Digital Therapeutics to Target Social Perception, Verbal Memory, Emotional Processing, and Problem Solving

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

Utilizing the CT-156 mobile app, individuals who have schizophrenia will carry out the method provided in FIG. 9 utilizing the interventions of Social Perception, Verbal Memory, Emotional Processing, and Problem Solving in order. The CT-156 mobile app may be used in conjunction with any of the functionalities or actions described herein in Section A and the Examples in Section B. The individuals will first obtain a baseline metric for Social Perception, followed by identifying a layer for a session and providing the session for the layer. Next, the individual using the CT-156 app will determine a next layer when the determination is to transition or to maintain the current layer. Finally, a session metric for Social Perception will be obtained that may or may not be an improvement over the baseline metric.

Following an improvement over the baseline metric, the individual will proceed to the next intervention, which is Verbal Memory. The process described above will be repeated for Verbal Memory. The individual will then move to the intervention of Emotional Processing, and the process described above will be repeated for Emotional Processing. Finally, the individual will move to the intervention of Problem Solving, and the process described above will be repeated for Problem Solving.

Upon completion of the four interventions in sequence, the users will experience an amelioration of functional impairments of schizophrenia, as measured by the MCAS value, the Lawton IADL value, the PSP scale value, the WHO-DAS 2.0 value, the C-SSRS value, or the time use survey value, among others, over the course of using the CT-156 mobile app.

### Example 9: Use of ELSCI in a Digital Therapeutics to Target Verbal Memory, Social Perception, Problem Solving, and Emotional Processing

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

Utilizing the CT-156 mobile app, individuals who have schizophrenia will carry out the method provided in FIG. 9 utilizing the interventions of Verbal Memory, Social Perception, Problem Solving, and Emotional Processing in order. The CT-156 mobile app may be used in conjunction with any of the functionalities or actions described herein in Section A and the Examples in Section B. The individuals will first obtain a baseline metric for Verbal Memory, followed by identifying a layer for a session and providing the session for the layer. Next, the individual using the CT-156 app will determine a next layer when the determination is to transition or to maintain the current layer. Finally, a session metric for Verbal Memory will be obtained that may or may not be an improvement over the baseline metric.

Following an improvement over the baseline metric, the individual will proceed to the next intervention, which is Social Perception. The process described above will be repeated for Social Perception. The individual will then move to the intervention of Problem Solving, and the process described above will be repeated for Problem Solving. Finally, the individual will move to the intervention of Emotional Processing, and the process described above will be repeated for Emotional Processing.

Upon completion of the four interventions in sequence, the users will experience an amelioration of functional impairments of schizophrenia, as measured by the MCAS value, the Lawton IADL value, the PSP scale value, the WHO-DAS 2.0 value, the C-SSRS value, or the time use survey value, among others, over the course of using the CT-156 mobile app.

### Example 10: Use of ELSCI in a Digital Therapeutics to Target Emotional Processing, Verbal Memory, Social Perception, and Problem Solving

In one example, the CT-156 mobile app (e.g., the application 125) will be given to individuals who have schizophrenia per ICD-11 or DSM-5, are experiencing mild-to-moderate functional impairment as evidenced by WHO-DAS 2.0, and are prescribed antipsychotic medication(s). The treatment will be 2-30 weeks long. It is expected that users will show an improvement in functional impairment, as measured by a MCAS value, a Lawton IADL value, a PSP scale value, a WHO-DAS 2.0 value, a C-SSRS value, or a time use survey value, among others, over the course of using the CT-156 mobile app.

Utilizing the CT-156 mobile app, individuals who have schizophrenia will carry out the method provided in FIG. 9 utilizing the interventions of Emotional Processing, Verbal Memory, Social Perception, and Problem Solving in order. The CT-156 mobile app may be used in conjunction with any of the functionalities or actions described herein in Section A and the Examples in Section B. The individuals will first obtain a baseline metric for Emotional Processing, followed by identifying a layer for a session and providing the session for the layer. Next, the individual using the CT-156 app will determine a next layer when the determination is to transition or to maintain the current layer. Finally, a session metric for Emotional Processing will be obtained that may or may not be an improvement over the baseline metric.

Following an improvement over the baseline metric, the individual will proceed to the next intervention, which is Verbal Memory. The process described above will be repeated for Verbal Memory. The individual will then move to the intervention of Social Perception, and the process described above will be repeated for Social Perception. Finally, the individual will move to the intervention of Problem Solving, and the process described above will be repeated for Problem Solving.

Upon completion of the four interventions in sequence, the users will experience an amelioration of functional impairments of schizophrenia, as measured by the MCAS value, the Lawton IADL value, the PSP scale value, the WHO-DAS 2.0 value, the C-SSRS value, or the time use survey value, among others, over the course of using the CT-156 mobile app.

### C. Network and Computing Environment

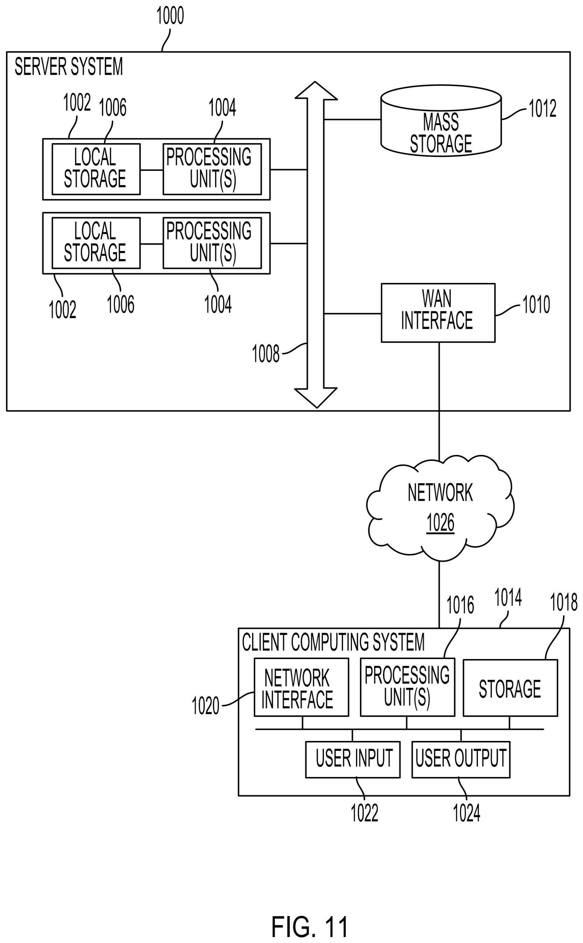

Various operations described herein can be implemented on computer systems. FIG. 11 shows a simplified block diagram of a representative server system 1000, client computer system 1014, and network 1026 usable to implement certain embodiments of the present disclosure. In various embodiments, server system 1000 or similar systems can implement services or servers described herein or portions thereof. Client computer system 1014 or similar systems can implement clients described herein. The system 100 described herein can be similar to the server system 1000. Server system 1000 can have a modular design that incorporates a number of modules 1002 (e.g., blades in a blade server embodiment); while two modules 1002 are shown, any number can be provided. Each module 1002 can include processing unit(s) 1004 and local storage 1006.

Processing unit(s) 1004 can include a single processor, which can have one or more cores, or multiple processors. In some embodiments, processing unit(s) 1004 can include a general-purpose primary processor as well as one or more special-purpose co-processors such as graphics processors, digital signal processors, or the like. In some embodiments, some or all processing units 1004 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 1004 can execute instructions stored in local storage 1006. Any type of processors in any combination can be included in processing unit(s) 1004.

Local storage 1006 can include volatile storage media (e.g., DRAM, SRAM, SDRAM, or the like) and/or non-volatile storage media (e.g., magnetic or optical disk, flash memory, or the like). Storage media incorporated in local storage 1006 can be fixed, removable, or upgradeable as desired. Local storage 1006 can be physically or logically divided into various subunits such as a system memory, a read-only memory (ROM), and a permanent storage device. The system memory can be a read-and-write memory device or a volatile read-and-write memory, such as dynamic random-access memory. The system memory can store some or all of the instructions and data that processing unit(s) 1004 need at runtime. The ROM can store static data and instructions that are needed by processing unit(s) 1004. The permanent storage device can be a non-volatile read-and-write memory device that can store instructions and data even when module 1002 is powered down. The term "storage medium" as used herein includes any medium in which data can be stored indefinitely (subject to overwriting, electrical disturbance, power loss, or the like) and does not include carrier waves and transitory electronic signals propagating wirelessly or over wired connections.

In some embodiments, local storage 1006 can store one or more software programs to be executed by processing unit(s) 1004, such as an operating system and/or programs implementing various server functions such as functions of the system 100 or any other system described herein, or any other server(s) associated with system 100 or any other system described herein.

"Software" refers generally to sequences of instructions that, when executed by processing unit(s) 1004, cause server system 1000 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 1004. Software can be implemented as a single program or a collection of separate programs or program modules that interact as desired. From local storage 1006 (or non-local storage described below), processing unit(s) 1004 can retrieve program instructions to execute and data to process in order to execute various operations described above.

In some server systems 1000, multiple modules 1002 can be interconnected via a bus or other interconnect 1008, forming a local area network that supports communication between modules 1002 and other components of server system 1000. Interconnect 1008 can be implemented using various technologies, including server racks, hubs, routers, etc.

A wide area network (WAN) interface 1010 can provide data communication capability between the local area network (e.g., through the interconnect 1008) and the network 1026, such as the Internet. Other technologies can be used to communicatively couple the server system with the network 1026, including wired (e.g., Ethernet, IEEE 802.3 standards) and/or wireless technologies (e.g., Wi-Fi, IEEE 802.11 standards).

In some embodiments, local storage 1006 is intended to provide working memory for processing unit(s) 1004, providing fast access to programs and/or data to be processed while reducing traffic on interconnect 1008. Storage for larger quantities of data can be provided on the local area network by one or more mass storage subsystems 1012 that can be connected to interconnect 1008. Mass storage subsystem 1012 can be based on magnetic, optical, semiconductor, or other data storage media. Direct attached storage, storage area networks, network-attached storage, and the like can be used. Any data stores or other collections of data described herein as being produced, consumed, or maintained by a service or server can be stored in mass storage subsystem 1012. In some embodiments, additional data storage resources may be accessible via WAN interface 1010 (potentially with increased latency).

Server system 1000 can operate in response to requests received via WAN interface 1010. For example, one of modules 1002 can implement a supervisory function and assign discrete tasks to other modules 1002 in response to received requests. Work allocation techniques can be used. As requests are processed, results can be returned to the requester via WAN interface 1010. Such operation can generally be automated. Further, in some embodiments, WAN interface 1010 can connect multiple server systems 1000 to each other, providing scalable systems capable of managing high volumes of activity. Other techniques for managing server systems and server farms (collections of server systems that cooperate) can be used, including dynamic resource allocation and reallocation.

Server system 1000 can interact with various user-owned or user-operated devices via a wide-area network such as the Internet. An example of a user-operated device is shown in FIG. 11 as client computing system 1014. Client computing system 1014 can be implemented, for example, as a consumer device such as a smartphone, other mobile phone, tablet computer, wearable computing device (e.g., smart watch, eyeglasses), desktop computer, laptop computer, and so on.

For example, client computing system 1014 can communicate via WAN interface 1010. Client computing system 1014 can include computer components such as processing unit(s) 1016, storage device 1018, network interface 1020, user input device 1022, and user output device 1024. Client computing system 1014 can be a computing device implemented in a variety of form factors, such as a desktop computer, laptop computer, tablet computer, smartphone, other mobile computing device, wearable computing device, or the like.

Processing unit 1016 and storage device 1018 can be similar to processing unit(s) 1004 and local storage 1006 described above. Suitable devices can be selected based on the demands to be placed on client computing system 1014. For example, client computing system 1014 can be implemented as a "thin" client with limited processing capability or as a high-powered computing device. Client computing system 1014 can be provisioned with program code executable by processing unit(s) 1016 to enable various interactions with server system 1000.

Network interface 1020 can provide a connection to the network 1026, such as a wide area network (e.g., the Internet) to which WAN interface 1010 of server system 1000 is also connected. In various embodiments, network interface 1020 can include a wired interface (e.g., Ethernet) and/or a wireless interface implementing various RF data communication standards such as Wi-Fi, Bluetooth, or cellular data network standards (e.g., 3G, 4G, LTE, etc.).

User input device 1022 can include any device (or devices) via which a user can provide signals to client computing system 1014; client computing system 1014 can interpret the signals as indicative of particular user requests or information. In various embodiments, user input device 1022 can include any or all of a keyboard, touch pad, touch screen, mouse or other pointing device, scroll wheel, click wheel, dial, button, switch, keypad, microphone, and so on.

User output device 1024 can include any device via which client computing system 1014 can provide information to a user. For example, user output device 1024 can include display-to-display images generated by or delivered to client computing system 1014. The display can incorporate various image generation technologies, e.g., a liquid crystal display (LCD), light-emitting diode (LED) display including organic light-emitting diodes (OLED), projection system, cathode ray tube (CRT), or the like, together with supporting electronics (e.g., digital-to-analog or analog-to-digital converters, signal processors, or the like). Some embodiments can include a device such as a touchscreen that function as both input and output device. In some embodiments, other user output devices 1024 can be provided in addition to or instead of a display. Examples include indicator lights, speakers, tactile "display" devices, printers, and so on.

Some embodiments include electronic components, such as microprocessors, storage, and memory that store computer program instructions in a computer readable storage medium. Many of the features described in this specification can be implemented as processes that are specified as a set of program instructions encoded on a computer readable storage medium. When these program instructions are executed by one or more processing units, they cause the processing unit(s) to perform various operations indicated in the program instructions. Examples of program instructions or computer code include machine code, such as is produced by a compiler, and files including higher-level code that are executed by a computer, an electronic component, or a microprocessor using an interpreter. Through suitable programming, processing unit(s) 1004 and 1016 can provide various functionality for server system 1000 and client computing system 1014, including any of the functionality described herein as being performed by a server or client, or other functionality.

It will be appreciated that server system 1000 and client computing system 1014 are illustrative and that variations and modifications are possible. Computer systems used in connection with embodiments of the present disclosure can have other capabilities not specifically described here. Further, while server system 1000 and client computing system 1014 are described with reference to particular blocks, it is to be understood that these blocks are defined for convenience of description and are not intended to imply a particular physical arrangement of component parts. For instance, different blocks can be but need not be located in the same facility, in the same server rack, or on the same motherboard. Further, the blocks need not correspond to physically distinct components. Blocks can be configured to perform various operations, e.g., by programming a processor or providing appropriate control circuitry, and various blocks might or might not be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including but not limited to specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, or any combination thereof. Further, while the embodiments described above may make reference to specific hardware and software components, those skilled in the art will appreciate that different combinations of hardware and/or software components may also be used and that particular operations described as being implemented in hardware might also be implemented in software or vice versa.

Computer programs incorporating various features of the present disclosure may be encoded and stored on various computer readable storage media; suitable media include magnetic disk or tape, optical storage media such as compact disk (CD) or digital versatile disk (DVD), flash memory, and other non-transitory media. Computer readable media encoded with the program code may be packaged with a compatible electronic device, or the program code may be provided separately from electronic devices (e.g., via Internet download or as a separately packaged computer-readable storage medium).

Various aspects of the present disclosure are set out in the following clauses.
1. A method of presenting interactive sessions to address functional impairment in users, comprising:
   identifying, by a computing system, a plurality of sessions to address an impairment associated with a condition of a user, each session of the plurality of sessions comprising a corresponding layer of a plurality of layers for the user;
   providing, by the computing system, a first session for a cognitive training layer by displaying one or more first images with which the user is to recognize one or more of a plurality of social cues associated with a social skill;
   providing, by the computing system, a second session for a virtual functional training layer for the user to apply the social skill in virtual social settings, comprising:
      (i) displaying a second image of a social setting with (a) a first prompt identifying a query associated with a character displaying one of the pluralities of social cues and (b) a set of interactive elements identifying a corresponding plurality of responses to the character,
      (ii) receiving a first response from the plurality of responses selected by the user via at least one of the set of interactive elements, and
      (iii) providing a feedback to the user based on the query for the social setting and the response; and
   providing, by the computing system, a third session for a functional training layer for the user to apply the social skill, comprising (i) displaying a second prompt to direct the user to perform an activity and (ii) receiving a second response associated with performance of the activity.
2. The method of clause 1, further comprising:
   generating, by the computing system, a performance metric for the user based on the first response received from the user during the second session at a first time instance;
   modifying, by the computing system, based on the performance metric, at least one of a plurality of parameters defining presentation of at least one of images, prompts, and interactive elements for the virtual functional training layer; and
   providing, by the computing system, the second session for the virtual functional training layer at a second time instance, comprising displaying a third image of a second social setting with (i) a third prompt identifying a second query of a second character in the second social setting and (ii) a second set of interactive elements identifying a corresponding plurality of responses to the second character in accordance with the plurality of parameters.
3. The method of clause 2, wherein the plurality of parameters comprises at least one of: (i) a type of modality for content, (ii) a context for social settings in images, (iii) a number of characters in social settings, (iv) a type of prompt, (v) a difficulty level for responses, (vi) a type of response, or (vii) a number of responses.
4. The method of any preceding clause, further comprising:
   generating, by the computing system, a performance metric for the user based on a rate of correct selections in one or more sessions for the cognitive training layer; and
   determining, by the computing system, responsive to the performance metric satisfying a threshold, to transition the user from the cognitive training layer to the virtual functional training layer.
5. The method of any preceding clause, further comprising:
   generating, by the computing system, a performance metric for the user based on a rate of correct responses in one or more sessions for the virtual functional training layer; and
   determining, by the computing system, responsive to the performance metric satisfying a threshold, to transition the user from the virtual functional training layer to the functional training layer.
6. The method of any preceding clause, wherein providing the first session further comprises (i) displaying a first image of a social cue with a set of interactive elements identifying the corresponding plurality of types of social cues associated with the first image and (ii) receiving a selection of one of the plurality of social cues by the user via at least one of the set of interactive elements,
   wherein the plurality of social cues for the first image in the first session for the cognitive training layer further comprises at least one of (a) a head movement, (b) a body language, (c) a gesticulation, or (d) an eye contact.
7. The method of any preceding clause, wherein the second session for the virtual functional training layer further comprises displaying a plurality of images of the social setting with the characters in accordance with a defined sequence.
8. The method of any preceding clause, wherein the third session for the functional training layer further comprises displaying a third prompt for the user to select a time at which to provide a message prompting the user to perform the activity.
9. The method of any preceding clause, further comprising identifying, by the computing system, a time at which to provide one of the plurality of sessions to the users in accordance with a session schedule.
10. The method of any preceding clause, wherein the condition of the user includes schizophrenia, wherein the user is receiving a treatment, at least in partial concurrence with at least one of the first session, the second session, or the third session, wherein the treatment comprises at least one of a psychosocial intervention or a medication to address schizophrenia, and/or wherein the medication comprises at least one of: haloperidol, chlorpromazine, fluphenazine, perphenazine, loxitane, thioridazine, trifluoperazine, aripiprazole, risperidone, clozapine, quetiapine, olanzapine, ziprasidone, lurasidone, paliperidone, or iclepertin.
11. A system for presenting interactive sessions to address functional impairment in users, comprising:
   a computing system having one or more processors coupled with memory, configured to:
      identify a plurality of sessions to address an impairment associated with a condition of a user, each session of the plurality of sessions comprising a corresponding layer of a plurality of layers for the user;
      provide a first session for a cognitive training layer by displaying one or more first images with which the user is to recognize one or more of a plurality of social cues associated with a social skill;
      provide a second session for a virtual functional training layer for the user to apply the social skill in virtual social settings, comprising:
         (i) displaying a second image of a social setting with (a) a first prompt identifying a query associated with a character displaying one of the plurality of social cues and (b) a set of interactive elements identifying a corresponding plurality of responses to the character,
         (ii) receiving a first response from the plurality of responses selected by the user via at least one of the set of interactive elements, and
         (iii) providing a feedback to the user based on the query for the setting and the response; and
   Provide a third session for a functional training layer for the user to apply the social skill, comprising (i) displaying a second prompt to direct the user to perform an activity and (ii) receiving a second response associated with performance of the activity.
12. The system of clause 11, wherein the computing system is further configured to:
   generate a performance metric for the user based on the first response received from the user during the second session at a first time instance;
   modify, based on the performance metric, at least one of a plurality of parameters defining presentation of at least one of images, prompts, and interactive elements for the virtual functional training layer; and
   provide the second session for the virtual functional training layer at a second time instance, comprising displaying a third image of a second social setting with (i) a third prompt identifying a second query of a second character in the second social setting and (ii) a second set of interactive elements identifying a corresponding plurality of responses to the second character in accordance with the plurality of parameters.
13. The system of clause 12, wherein the plurality of parameters comprises at least one of: (i) a type of modality for content, (ii) a context for social settings in images, (iii) a number of characters in social settings, (iv) a type of prompt, (v) a difficulty level for responses, (vi) a type of response, or (vii) a number of responses.
14. The system of any one of clauses 11 to 13, wherein the computing system is further configured to:
   generate a performance metric for the user based on a rate of correct selections in one or more sessions for the cognitive training layer; and
   determine, responsive to the performance metric satisfying a threshold, to transition the user from the cognitive training layer to the virtual functional training layer.
15. The system of any one of clauses 11 to 14, wherein the computing system is further configured to:
   generate a performance metric for the user based on a rate of correct responses in one or more sessions for the virtual functional training layer; and
   determine, responsive to the performance metric satisfying a threshold, to transition the user from the virtual functional training layer to the functional training layer.
16. The system of any one of clauses 11 to 15, wherein providing the first session further comprises (i) displaying a first image of a social cue with a set of interactive elements identifying the corresponding plurality of social cues associated with the first image and (ii) receiving a selection of one of the plurality of social cues by the user via at least one of the second set of interactive elements
   wherein the plurality of social cues for the first image in the first session for the cognitive training layer further comprises at least one of (a) a head movement, (b) a body language, (c) a gesticulation, or (d) an eye contact.
17. The system of any one of clauses 11 to 16, wherein the second session for the virtual functional training layer further comprises displaying a plurality of images of the social setting with the characters in accordance with a defined sequence.
18. The system of any one of clauses 11 to 17, wherein the third session for the functional training layer further comprises displaying a third prompt for the user to select a time at which to provide a message prompting the user to perform the activity.
19. The system of any one of clauses 11 to 18, wherein the computing system is further configured to identify a time at which to provide one of the plurality of sessions to the users in accordance with a session schedule.
20. The system of any one of clauses 11 to 19, wherein the condition of the user includes schizophrenia, wherein user is receiving a treatment, at least in partial concurrence with at least one of the first session, the second session, or the third session, wherein the treatment comprises at least one of a psychosocial intervention or a medication to address schizophrenia.
21. A method of ameliorating a functional impairment in a user suffering from schizophrenia in need thereof, comprising:
   obtaining, by a computing system, a first metric associated with the user prior to a plurality of sessions;
   repeating, by the computing system, provision of one or more of the plurality of sessions to the user, the plurality of sessions comprising:
      a first session for a cognitive training layer by displaying one or more first images with which the user is to recognize one or more of a plurality of social cues associated with a social skill;
      a second session for a virtual functional training layer for the user to apply the social skill in virtual social settings, comprising (i) displaying a second image of a social setting with (a) a first prompt identifying a query associated with a character displaying one of the plurality of social cues and (b) a second set of interactive elements identifying a corresponding plurality of responses to the character, (ii) receiving a response from the plurality of responses selected by the user via at least one of the second set of interactive elements, and (iii) providing a feedback to the user based on the query for the setting and the response; and
      a third session for a functional training layer, comprising (i) displaying a second prompt to direct the user to perform an activity and (ii) receiving a response associated with performance of the activity; and
   obtaining, by the computing system, a second metric associated with the user subsequent to at least one of the plurality of sessions,
   wherein amelioration in the functional impairment associated with schizophrenia occurs in the user, when the second metric is (i) decreased from the first metric by a first predetermined margin or (ii) increased from the first metric by a second predetermined margin.
22. The method of clause 21, wherein schizophrenia in the user further comprises at least one of: (i) schizophrenia with positive symptoms including hallucinations or delusions; or (ii) schizophrenia with negative symptoms including a decrease in motivation or emotional expressions.
23. The method of clause 21 or clause 22, wherein the functional impairment associated with the user further comprises at least one of (i) reduced attainment in education, (ii) reduced quality of life, (iii) difficulty in living independently, (iv) reduced social functioning, or (v) occupational dysfunction.
24. The method of any one of clauses 21 to 23, wherein the user is an adult aged at least 18 years or older and diagnosed with schizophrenia having the functional impairment.
25. The method of any one of clauses 21 to 24, wherein the amelioration in the functional impairment associated with schizophrenia occurs when the second metric is decreased from the first metric by the first predetermined margin, and wherein the first metric and the second metric are Multnomah Community Ability Scale (MCAS) values.
26. The method of any one of clauses 21 to 24, wherein the amelioration in the functional impairment associated with schizophrenia occurs when the second metric is increased from the first metric by the second predetermined margin, and wherein the first metric and the second metric are Lawton Instrumental Activities of Daily Living (IADL) scale values.
27. The method of any one of clauses 21 to 24, wherein the amelioration in the functional impairment associated with schizophrenia occurs when the second metric is increased from the first metric by the second predetermined margin, and wherein the first metric and the second metric are Personal and Social Performance (PSP) scale values.
28. The method of any one of clauses 21 to 24, wherein the amelioration in the functional impairment associated with schizophrenia occurs when the second metric is decreased from the first metric by the first predetermined margin, and wherein the first metric and the second metric are World Health Organization Disability Assessment Schedule 2.0 (WHO-DAS 2.0) scale values.
29. The method of any one of clauses 21 to 24, wherein the amelioration in the functional impairment associated with schizophrenia occurs when the second metric is decreased from the first metric by the first predetermined margin, and wherein the first metric and the second metric are Columbia-Suicide Severity Rating Scale (C-SSRS) values.
30. The method of any one of clauses 21 to 29, wherein the plurality of sessions further comprises the second session for the virtual functional training at a first time instance, comprising displaying a third image of a second setting with (i) a third prompt identifying a second query of a second character in the second setting and (ii) a third set of interactive elements identifying a corresponding plurality of responses to the second character in accordance with a plurality of parameters,
   wherein at least one of the plurality of parameters is modified based on a performance metric for the user using the response received from the user during the second session at a second time instance prior to the first time instance.
31. The method of clause 30, wherein the plurality of parameters comprises at least one of: (i) a type of modality for content, (ii) a context for settings in images, (iii) a number of characters in settings, (iv) a type of prompt, (v) a difficulty level for responses, (vi) a type of response, or (vii) a number of responses.
32. The method of any one of clauses 21 to 31, further comprising determining, by the computing system, for at least one of the plurality of sessions, to transition from one layer to another layer based on a performance metric for the user across one or more of the plurality of sessions.
33. The method of any one of clauses 21 to 32, wherein providing the first session further comprises (i) displaying a first image of a social cue with a set of interactive elements identifying the corresponding plurality of types of cues associated with the first image and (ii) receiving a selection of one of the plurality of types of social cues by the user via at least one of the second set of interactive elements,
   wherein the plurality of social cues for the first image in the first session for the cognitive training layer further comprises at least one of (a) a head movement, (b) a body language, (c) a gesticulation, or (d) an eye contact.
34. The method of any one of clauses 21 to 33, wherein the second session for the virtual functional training layer further comprises displaying a plurality of images of the setting with the characters in accordance with a defined sequence.
35. The method of any one of clauses 21 to 34, wherein the third session for the functional training layer further comprises displaying a third prompt for the user to select a time at which to provide a message prompting the user to perform the activity.
36. The method of any one of clauses 21 to 35, wherein the plurality of sessions are provided over a period of time ranging between 2 weeks to 10 weeks.
37. The method of any one of clauses 21 to 36, wherein the user is receiving a treatment, at least in partial concurrence with at least one of the plurality of sessions, wherein the treatment comprises at least one of a psychosocial intervention or a medication to address schizophrenia,
   wherein the medication comprises at least one of: haloperidol, chlorpromazine, fluphenazine, perphenazine, loxitane, thioridazine, trifluoperazine, aripiprazole, risperidone, clozapine, quetiapine, olanzapine, ziprasidone, lurasidone, paliperidone, or iclepertin.
38. A method of presenting interactive sessions to address impairments in verbal recall and cognitive association in users, comprising:
   identifying, by a computing system, a plurality of sessions to address an impairment associated with a condition of a user, each session of the plurality of sessions comprising a corresponding layer of a plurality of layers for the user;
   providing, by the computing system, a first session for a cognitive training layer associated with a verbal memory skill by presenting one or more first audio recordings with which the user is to recall one or more of a plurality of words;
   providing, by the computing system, a second session for a virtual functional training layer for the user to apply the verbal memory skill in virtual settings, comprising:
      (i) presenting a second audio recording of a speech sample with (a) a first prompt identifying a query associated with the speech sample and (b) a set of interactive elements identifying a corresponding plurality of responses,
      (ii) receiving a first response from the plurality of responses selected by the user via at least one of the set of interactive elements, and
      (iii) providing a feedback to the user based on the query for the speech sample and the response; and
   providing, by the computing system, a third session for a functional training layer for the user to apply the verbal memory skill, comprising (i) displaying a second prompt to direct the user to perform an activity and (ii) receiving a second response associated with performance of the activity.
39. The method of clause 38, further comprising:
   generating, by the computing system, a performance metric for the user based on the first response received from the user during the second session at a first time instance;
   modifying, by the computing system, based on the performance metric, at least one of a plurality of parameters defining presentation of at least one of audio recordings, prompts, and interactive elements for the virtual functional training layer; and
   providing, by the computing system, the second session for the virtual functional training layer at a second time instance, comprising presenting a third audio recording of a speech sample between characters in a social setting with (i) a third prompt identifying a second query associated with the speech sample of the characters the social setting and (ii) a second set of interactive elements identifying a corresponding plurality of responses in accordance with the plurality of parameters.
40. The method of clause 39, wherein the plurality of parameters comprises at least one of: (i) a type of modality for content, (ii) a context for social settings in audio recordings, (iii) a number of characters in social settings, (iv) a type of prompt, (v) a difficulty level for responses, (vi) a type of response, or (vii) a number of responses.
41. The method of any one of clauses 38 to 40, wherein providing the second session for the virtual functional training layer further comprises presenting the second audio recording in accordance with a plurality of parameters,
   wherein the plurality of parameters comprising at least one of: (a) an inclusion of a distraction, (b) an ability to repeat the second audio recording, (c) a modification in speed, (d) a time between each word, (e) a number of words in each sentence, (f) a length of the audio recording, or (g) an ability to control the distraction.
42. The method of any one of clauses 38 to 41, further comprising:
   generating, by the computing system, a performance metric for the user based on a rate of correct selections in one or more sessions for the cognitive training layer; and
   determining, by the computing system, responsive to the performance metric satisfying a threshold, to transition the user from the cognitive training layer to the virtual functional training layer.
43. The method of any one of clauses 38 to 42, further comprising:
   generating, by the computing system, a performance metric for the user based on a rate of correct responses in one or more sessions for the virtual functional training layer; and
   determining, by the computing system, responsive to the performance metric satisfying a threshold, to transition the user from the virtual functional training layer to the functional training layer.
44. The method of any one of clauses 38 to 43, wherein providing the first session further comprises:
   identifying, from a plurality of recordings, a set of recordings to present to the user, each of the plurality of recordings corresponding to one or more words;
   presenting, to the user, the set of recordings in accordance with a format to define a context in which the one or more words of each of the set of recordings are presented;
   displaying an interface to prompt the user to select at least one of a plurality of words as presented in the set of recordings; and
   receiving, via the interface, a selection of at least one word by the user.
45. The method of any one of clauses 38 to 44, wherein the third session for the functional training layer further comprises displaying a third prompt for the user to select a time at which to provide a message prompting the user to perform the activity.
46. The method of any one of clauses 38 to 45, further comprising identifying, by the computing system, a time at which to provide one of the plurality of sessions to the users in accordance with a session schedule.
47. The method of any one of clauses 38 to 46, wherein the condition of the user includes schizophrenia, wherein the user is receiving a treatment, at least in partial concurrence with at least one of the first session, the second session, or the third session, wherein the treatment comprises at least one of a psychosocial intervention or a medication to address schizophrenia, and
   wherein the medication comprises at least one of: haloperidol, chlorpromazine, fluphenazine, perphenazine, loxitane, thioridazine, trifluoperazine, aripiprazole, risperidone, clozapine, quetiapine, olanzapine, ziprasidone, lurasidone, paliperidone, or iclepertin.
48. A system for presenting interactive sessions to address impairments in verbal recall and cognitive association in users, comprising:
   a computing system having one or more processors coupled with memory, configured to:
   identify a plurality of sessions to address an impairment associated with a condition of a user, each session of the plurality of sessions comprising a corresponding layer of a plurality of layers for the user;
   provide a first session for a cognitive training layer associated with a verbal memory skill by presenting one or more first audio recordings with which the user is to recall one or more of a plurality of words;
   provide a second session for a virtual functional training layer for the user to apply the verbal memory skill in virtual social settings, comprising:
      (i) presenting a second audio recording of a speech sample with (a) a first prompt identifying a query associated with the speech sample and (b) a set of interactive elements identifying a corresponding plurality of responses,
      (ii) receiving a first response from the plurality of responses selected by the user via at least one of the set of interactive elements, and
      (iii) providing a feedback to the user based on the query for the speech sample and the response; and
   provide a third session for a functional training layer for the user to apply the verbal memory skill, comprising (i) displaying a second prompt to direct the user to perform an activity and (ii) receiving a second response associated with performance of the activity.
49. The system of clause 48, wherein the computing system is further configured to
   generate a performance metric for the user based on the first response received from the user during the second session at a first time instance;
   modify, based on the performance metric, at least one of a plurality of parameters defining presentation of at least one of audio recordings, prompts, and interactive elements for the virtual functional training layer; and
   provide the second session for the virtual functional training layer at a second time instance, comprising presenting a third audio recording of a speech sample between characters in a social setting with (i) a third prompt identifying a second query associated with the speech sample of the characters the social setting and (ii) a second set of interactive elements identifying a corresponding plurality of responses in accordance with the plurality of parameters.
50. The system of clause 48 or clause 49, wherein the plurality of parameters comprises at least one of: (i) a type of modality for content, (ii) a context for social settings in audio recordings, (iii) a number of characters in social settings, (iv) a type of prompt, (v) a difficulty level for responses, (vi) a type of response, or (vii) a number of responses.
51. The system of any one of clauses 48 to 50, wherein the computing system is further configured to present the second audio recording in accordance with a plurality of parameters,
   wherein the plurality of parameters comprising at least one of: (a) an inclusion of a distraction, (b) an ability to repeat the second audio recording, (c) a modification in speed, (d) a time between each word, (e) a number of words in each sentence, (f) a length of the audio recording, or (g) an ability to control the distraction.
52. The system of any one of clauses 48 to 51, wherein the computing system is further configured to:
   generate a performance metric for the user based on a rate of correct selections in one or more sessions for the cognitive training layer; and
   determine, responsive to the performance metric satisfying a threshold, to transition the user from the cognitive training layer to the virtual functional training layer.
53. The system of any one of clauses 48 to 52, wherein the computing system is further configured to:
   generate a performance metric for the user based on a rate of correct responses in one or more sessions for the virtual functional training layer; and
   determine, responsive to the performance metric satisfying a threshold, to transition the user from the virtual functional training layer to the functional training layer.
54. The system of any one of clauses 48 to 53, wherein the computing system is further configured to provide the first session by:
   identifying, from a plurality of recordings, a set of recordings to present to the user, each of the plurality of recordings corresponding to one or more words;
   presenting, to the user, the set of recordings in accordance with a format to define a context in which the one or more words of each of the set of recordings are presented;
   displaying an interface to prompt the user to select at least one of a plurality of words as presented in the set of recordings; and
   receiving, via the interface, a selection of at least one word by the user.
55. The system of any one of clauses 48 to 54, wherein the third session for the functional training layer further comprises displaying a third prompt for the user to select a time at which to provide a message prompting the user to perform the activity.
56. The system of any one of clauses 48 to 55, wherein the computing system is further configured to identify a time at which to provide one of the plurality of sessions to the users in accordance with a session schedule.
57. The system of clauses 48 to 56, wherein the condition of the user includes schizophrenia, wherein the user is receiving a treatment, at least in partial concurrence with at least one of the first session, the second session, or the third session, wherein the treatment comprises at least one of a psychosocial intervention or a medication to address schizophrenia.
58. A method of ameliorating a functional impairment associated with verbal memory in a user suffering from schizophrenia in need thereof, comprising:
   obtaining, by a computing system, a first metric associated with the user prior to a plurality of sessions;
   repeating, by the computing system, provision of one or more of the plurality of sessions to the user, the plurality of sessions comprising:
      a first session for a cognitive training layer associated with a verbal memory skill by presenting one or more first audio recordings with which the user is to recall one or more of a plurality of words;
      a second session for a virtual functional training layer for the user to apply the verbal memory skill in virtual social settings, comprising:
         (i) presenting a second audio recording with (a) a first prompt identifying a query associated with a speech sample and (b) a set of interactive elements identifying a corresponding plurality of responses,
         (ii) receiving a first response from the plurality of responses selected by the user via at least one of the set of interactive elements, and
         (iii) providing a feedback to the user based on the query for the speech sample and the response; and
      a third session for a functional training layer for the user to apply the verbal memory skill, comprising (i) displaying a second prompt to direct the user to perform an activity and (ii) receiving a second response associated with performance of the activity; and
   obtaining, by the computing system, a second metric associated with the user subsequent to at least one of the plurality of sessions,
   wherein amelioration in the functional impairment associated with schizophrenia occurs in the user, when the second metric is (i) decreased from the first metric by a first predetermined margin or (ii) increased from the first metric by a second predetermined margin.
59. The method of clause 58, wherein the schizophrenia further comprises schizophrenia with negative symptoms including a decrease in motivation or emotional expressions.
60. The method of clause 58 or clause 59, wherein the impairment associated with the user further comprises functional impairment including at least one of (i) reduced attainment in education, (ii) reduced quality of life, (iii) difficulty in living independently, (iv) reduced social functioning, or (v) occupational dysfunction.
61. The method of any one of clauses 58 to 60, wherein the user is an adult aged at least 18 years or older and diagnosed with the schizophrenia having the functional impairment.
62. The method of any one of clauses 58 to 61, wherein the plurality of sessions are provided over a period of time ranging between 2 weeks to 10 weeks.
63. The method of any one of clauses 58 to 62, wherein the amelioration in the functional impairment associated with schizophrenia occurs when the second metric is decreased from the first metric by the first predetermined margin, and wherein the first metric and the second metric are Multnomah Community Ability Scale (MCAS) values.
64. The method of any one of clauses 58 to 62, wherein the amelioration in the functional impairment associated with schizophrenia occurs when the second metric is increased from the first metric by the second predetermined margin, and wherein the first metric and the second metric are clinical rating scale (CRS) values.
65. The method of any one of clauses 58 to 62, wherein the amelioration in the functional impairment associated with schizophrenia occurs when the second metric is decreased from the first metric by the first predetermined margin, and wherein the first metric and the second metric are Patient Global Impression of Improvement (PGI-I) scale values.
66. The method of any one of clauses 58 to 62, wherein the amelioration in the functional impairment associated with schizophrenia occurs when the second metric is decreased from the first metric by the first predetermined margin, and wherein the first metric and the second metric are Clinical Global Impression of Improvement (CGI-I) scale values.
67. The method of any one of clauses 58 to 62, wherein the amelioration in the functional impairment associated with schizophrenia occurs when the second metric is increased from the first metric by the second predetermined margin, and wherein the first metric and the second metric are medication adherence rating scale (MARS-a) values.
68. The method of any one of clauses 58 to 62, wherein the amelioration in the functional impairment associated with schizophrenia occurs when the second metric is decreased from the first metric by the first predetermined margin, and wherein the first metric and the second metric are Columbia-Suicide Severity Rating Scale (C-SSRS) values.
69. The method of any one of clauses 58 to 68, further comprising determining, by the computing system, for at least one of the plurality of sessions, to transition from one layer to another layer based on a performance metric for the user across one or more of the plurality of sessions.
70. The method of any one of clauses 58 to 69, wherein the second session further comprises presenting the second audio recording in accordance with a plurality of parameters,
   wherein the plurality of parameters comprising at least one of: (a) an inclusion of a distraction, (b) an ability to repeat the second audio recording, (c) a modification in speed, (d) a time between each word, (e) a number of words in each sentence, (f) a length of the audio recording, or (g) an ability to control the distraction.
71. The method of any one of clauses 58 to 70, wherein the first session further comprises:
   identifying, from a plurality of recordings, a set of recordings to present to the user, each of the plurality of recordings corresponding to one or more words;
   presenting, to the user, the set of recordings in accordance with a format to define a context in which the one or more words of each of the set of recordings are presented;
   displaying an interface to prompt the user to select at least one of a plurality of words as presented in the set of recordings; and
   receiving, via the interface, a selection of at least one word by the user.
72. The method of any one of clauses 58 to 71, wherein the user is receiving a treatment, at least in partial concurrence with at least one of the plurality of sessions, wherein the treatment comprises at least one of a psychosocial intervention or a medication to address schizophrenia, and
   wherein the medication comprises at least one of: haloperidol, chlorpromazine, fluphenazine, perphenazine, loxitane, thioridazine, trifluoperazine, aripiprazole, risperidone, clozapine, quetiapine, olanzapine, ziprasidone, lurasidone, paliperidone, or iclepertin.
73. A computer readable storage medium comprising computer readable instructions that, when executed by one or more processors, cause the one or more processors to perform the method of any one of clauses 1 to 10, 21 to 47 or 58 to 72.

Thus, although the disclosure has been described with respect to specific embodiments, it will be appreciated that the disclosure is intended to cover all modifications and equivalents within the scope of the following claims.

## Claims

1. A method of presenting interactive sessions to address impairments in verbal learning, verbal memory, and/or cognitive association in users, comprising:
identifying, by a computing system, a plurality of sessions to address an impairment associated with a condition of a user, each session of the plurality of sessions comprising a corresponding layer of a plurality of layers for the user;
providing, by the computing system, a first session for a cognitive training layer associated with a verbal memory skill by presenting one or more first audio recordings with which the user is to recall one or more of a plurality of words;
providing, by the computing system, a second session for a virtual functional training layer for the user to apply the verbal memory skill in virtual settings, comprising:
(i) presenting a second audio recording of a speech sample with (a) a first prompt identifying a query associated with the speech sample and (b) a set of interactive elements identifying a corresponding plurality of responses,
(ii) receiving a first response from the plurality of responses selected by the user via at least one of the set of interactive elements, and
(iii) providing a feedback to the user based on the query for the speech sample and the response; and
providing, by the computing system, a third session for a functional training layer for the user to apply the verbal memory skill, comprising (i) displaying a second prompt to direct the user to perform an activity and (ii) receiving a second response associated with performance of the activity.

2. The method of claim 1, wherein providing the second session for the virtual functional training layer further comprises presenting the second audio recording in accordance with a plurality of parameters,
wherein the plurality of parameters comprising at least one of: (a) an inclusion of a distraction, (b) an ability to repeat the second audio recording, (c) a modification in speed, (d) a time between each word, (e) a number of words in each sentence, (f) a length of the audio recording, or (g) an ability to control the distraction.

3. The method of claim 1 or claim 2, further comprising:
generating, by the computing system, a performance metric for the user based on a rate of correct selections in one or more sessions for the cognitive training layer; and
determining, by the computing system, responsive to the performance metric satisfying a threshold, to transition the user from the cognitive training layer to the virtual functional training layer.

4. The method of any preceding claim, further comprising:
generating, by the computing system, a performance metric for the user based on a rate of correct responses in one or more sessions for the virtual functional training layer; and
determining, by the computing system, responsive to the performance metric satisfying a threshold, to transition the user from the virtual functional training layer to the functional training layer.

5. The method of any preceding claim, further comprising:
presenting other interactive sessions to address impairments in problem solving in users, to address impairments in social perception in users, to address impairments in emotional processing in users, or any combination thereof.

6. A system for presenting interactive sessions to address impairments in verbal learning, verbal memory, and/or cognitive association in users, comprising:
a computing system having one or more processors coupled with memory, configured to perform the method of any one preceding claim.

7. A use of the computing system of the system of claim 6 to ameliorate an impairment in verbal learning, verbal memory, and/or cognitive association in a user suffering from a neurological disorder or an affective disorder in need thereof.

8. A method of presenting interactive sessions to address impairments in problem solving in users, comprising:
identifying, by a computing system, a plurality of sessions to address an impairment associated with a condition of a user, each session of the plurality of sessions comprising a corresponding layer of a plurality of layers for the user;
providing, by the computing system, a first session for a cognitive training layer associated with problem solving by displaying one or more games for the user to play;
providing, by the computing system, a second session for a virtual functional training layer for the user to practice the problem solving in virtual settings, comprising:
(i) displaying an image or text with (a) a first prompt identifying a query and (b) a set of interactive elements identifying a corresponding plurality of responses based on the first prompt,
(ii) receiving a first response from the plurality of responses selected by the user via at least one of the set of interactive elements, and
(iii) providing a feedback to the user based on the query and the response; and
providing, by the computing system, a third session for a functional training layer for the user to apply the problem solving, comprising (i) displaying a second prompt to direct the user to perform an activity and (ii) receiving a second response associated with performance of the activity.

9. The method of claim 8, further comprising:
generating, by the computing system, a performance metric for the user based on a rate of correct selections in one or more sessions for the cognitive training layer; and
determining, by the computing system, responsive to the performance metric satisfying a threshold, to transition the user from the cognitive training layer to the virtual functional training layer.

10. The method of claim 8 or claim 9, further comprising:
generating, by the computing system, a performance metric for the user based on a rate of correct responses in one or more sessions for the virtual functional training layer; and
determining, by the computing system, responsive to the performance metric satisfying a threshold, to transition the user from the virtual functional training layer to the functional training layer.

11. The method of any one of claims 8-10, further comprising:
presenting other interactive sessions to address impairments in verbal memory in users, to address impairments in verbal learning in users, to address impairments in cognitive association in users, to address impairments in social perception in users, to address impairments in emotional processing in users, or any combination thereof.

12. The method of any one of claims 1-5 or claims 8-11, wherein the condition of the user includes a neurological disorder or an affective disorder, wherein the user is receiving a treatment, at least in partial concurrence with at least one of the first session, the second session, or the third session, wherein the treatment comprises at least one of a psychosocial intervention or a medication to address the condition.

13. A system for presenting interactive sessions to address impairments in problem solving in users, comprising:
a computing system having one or more processors coupled with memory, configured to perform the method of any one of claims 8-11 or claim 12 when dependent on one of claims 8-11.

14. A use of the computing system of the system of claim 13 to ameliorate an impairment in problem solving in a user suffering from a neurological disorder or an affective disorder in need thereof.

15. A computer readable storage medium comprising computer readable instructions that, when executed by one or more processors, cause the one or more processors to perform the method of any one of claims 1 to 5, 8 to 11 or claim 12 when dependent on one of claims 8-11.
